(19) Europäisches Patentamt
European Patent Office
Office européen des brevets

(11) EP 3 712 975 A1

(12) **EUROPEAN PATENT APPLICATION**
published in accordance with Art. 153(4) EPC

(43) Date of publication:
**23.09.2020 Bulletin 2020/39**

(21) Application number: **18878101.7**

(22) Date of filing: **14.11.2018**

(51) Int Cl.:
***H01L 51/50*** *(2006.01)* ***C07D 209/88*** *(2006.01)*
***C07D 307/91*** *(2006.01)* ***C09K 11/06*** *(2006.01)*
***C07C 211/54*** *(2006.01)* ***C07C 211/61*** *(2006.01)*
***C07F 15/00*** *(2006.01)*

(86) International application number:
**PCT/JP2018/042115**

(87) International publication number:
**WO 2019/098234 (23.05.2019 Gazette 2019/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.11.2017 JP 2017220535**

(71) Applicant: **Hodogaya Chemical Co., Ltd.**
**Chuo-ku, Tokyo 104-0028 (JP)**

(72) Inventors:
- **MOCHIZUKI, Shunji**
**Tokyo 104-0028 (JP)**
- **OHKUMA, Hiroshi**
**Tokyo 104-0028 (JP)**
- **YAMAMOTO, Takeshi**
**Tokyo 104-0028 (JP)**
- **SURUGA, Kazuyuki**
**Tokyo 104-0028 (JP)**

(74) Representative: **Müller-Boré & Partner Patentanwälte PartG mbB**
**Friedenheimer Brücke 21**
**80639 München (DE)**

(54) **ORGANIC ELECTROLUMINESCENT ELEMENT**

(57) [Object] It is an object of the present invention to provide an organic compound having excellent properties such as excellent hole injection/transport performance, electron blocking performance, high stability in a thin-film state, and high light emission efficiency as a material for a highly efficient organic EL device having a high durability, and a highly efficient organic EL device having a high durability by using this compound.

[Solving Means] An organic EL device, including, between an anode and a cathode, at least a first hole transport layer, a second hole transport layer, a green light-emitting layer, and an electron transport layer in the stated order from a side of the anode, the organic EL device being characterized in that, the second hole transport layer, or at least one of stacked films disposed between the first hole transport layer and the electron transport layer contains an arylamine compound represented by the following general formula (1) .

(Chem. 1)

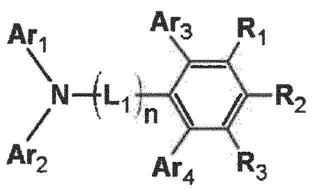

(1)

(In the formula, $Ar_1$, $Ar_2$, $Ar_3$, and $Ar_4$ may be the same as or different from each other, and each represent a substituted or unsubstituted aromatic hydrocarbon group, a substituted or unsubstituted aromatic heterocyclic group, or



Printed by Jouve, 75001 PARIS (FR)

(Cont. next page)

a substituted or unsubstituted fused polycyclic aromatic group. $L_1$ represents a divalent group of a substituted or unsubstituted aromatic hydrocarbon, a divalent group of a substituted or unsubstituted aromatic heterocyclic, or a divalent group of a substituted or unsubstituted fused polycyclic aromatic. $R_1$, $R_2$, and $R_3$ each represent a hydrogen atom, a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. n represents an integer of 1 to 3.)

## Description

Technical Field

The present invention relates to a compound suitable for an organic electroluminescence device (hereinafter, abbreviated as an organic EL device) that is a self-light-emitting device suitable for various display devices, and to the device, and specifically to an organic EL device that uses an arylamine compound.

Background Art

Since the organic EL device is a self-light-emitting device, it is brighter than the liquid crystal device and excellent in visibility, and capable of performing clear display, and thus, active research has been done thereon.

In 1987, C.W.Tang et al. (Eastman Kodak Company) have developed a stacked structural device in which various roles are assigned to the materials, and put an organic EL device using an organic material to practical use. They have stacked a fluorophore capable of transporting electrons and an aromatic amine compound capable of transporting tris(8-hydroxyquinoline) aluminum (hereinafter, abbreviated as $Alq_3$) and holes, and injected both charges into a fluorophore layer to emit light, thereby achieving high luminance of 1000 cd/$m^2$ or more with a voltage of 10 V or less (see, for example, Patent Literature 1 and Patent Literature 2).

Many improvements have been made for practical use of the organic EL device until now. In an electroluminescence device that subdivides the various roles and includes an anode, a hole injection layer, a hole transport layer, a light-emitting layer, an electron transport layer, an electron injection layer, and a cathode in the stated order on a substrate, high efficiency and durability have been achieved (see, for example, Non-Patent Literature 1).

Further, for the purpose of further improving the light emission efficiency, attempts have been made to use a triplet exciton and utilization of a phosphorescent emitter is being considered (see, for example, Non-Patent Literature 2).

The light-emitting layer can also be prepared by doping a charge transport compound generally called a host material with a fluorophore or a phosphorescent emitter. In recent years, a highly efficient organic EL device that uses an iridium complex as a phosphorescent material and a compound having a carbazole structure as a host material has been proposed (see, for example, Patent Literature 3).

Further, both a compound having a nitrogen-containing heteroaromatic ring structure with high electron transportability and a compound having a carbazole structure having hole transport ability are used as hosts to increase the transportability of electron and holes and improve the light emission efficiency has been remarkably as compared with the case of using one of them alone (see, for example, Patent Literature 5).

As a hole transport material and a host material having hole transportability that have been used for a phosphorescent organic EL device, a carbazole derivative (for example, HTM-1), which is disclosed in Patent Literature 6, has been known The carbazole derivative has a high triplet energy level (hereinafter, abbreviated as T1) and an excellent ability to confine triplet excitons, but has a low mobility of holes and a problem of electrical reduction durability. For this reason, in the case where a light-emitting layer having improved electron transportability is combined with them, there is a concern that the supply of holes to the light-emitting layer is rate-limiting, the number of electrons in the light-emitting layer is biased toward an excess, and the light emission efficiency is reduced and the lifetime is shortened in the organic EL devices using them.

(Chem. 1)

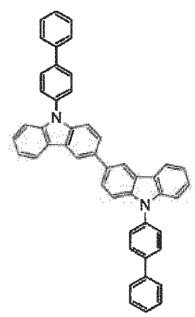

(HTM−1)

As an attempt to solve the above-mentioned problems, a monoamine compound (for example, HTM-2) represented by the following general formula, which has an excellent electric durability and high hole transport ability has been proposed in Patent Literature 7.

(Chem. 2)

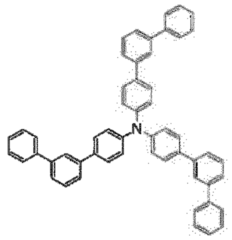

(HTM−2)

However, these monoamine compounds have a higher mobility of holes than carbazole derivatives, but have a problem of low T1. For this reason, triplet excitons are not sufficiently confined, and there is a concern that the light emission efficiency is reduced and the device lifetime is shortened due to exciton deactivation. For this reason, a hole transport material and a host material having a high mobility of holes and an excellent ability to confine triplet excitons s have been desired.

Citation List

Patent Literature

Patent Literature 1: Japanese Patent Application Laid-open No. 1996-048656
Patent Literature 2: Japanese Patent Application Laid-open No. 1995-126615
Patent Literature 3: Japanese Patent Application Laid-open No. 2006-151979
Patent Literature 4: WO 2015/034125
Patent Literature 5: WO 2016/013732
Patent Literature 6: Japanese Patent Application Laid-open No. 1996-003547
Patent Literature 7: Japanese Patent Application Laid-open No. 2006-352088
Patent Literature 8: WO 2016/199743
Patent Literature 9: Japanese Patent Application Laid-open No. 2002-105055
Patent Literature 10: WO 2014/007565
Patent Literature 11: WO 2014/188947
Patent Literature 12: WO 2015/190400
Patent Literature 13: Japanese Patent Application Laid-open No. 2010-83862
Patent Literature 14: WO 2015/038503
Patent Literature 15: Japanese Patent Application Laid-open No. 2005-108804
Patent Literature 16: WO 2008/62636
Patent Literature 17: WO 2014/009310

Non-Patent Literature

Non-Patent Literature 1: The Japan Society of Applied Physics, proceedings of the ninth workshop, pp. 55-61 (2001)
Non-Patent Literature 2: The Japan Society of Applied Physics, proceedings of the ninth workshop, pp. 23-31 (2001)
Non-Patent Literature 3: Synth.Commun.,11,513(1981)

Disclosure of Invention

Technical Problem

It is an object of the present invention to provide an organic compound having excellent properties such as excellent hole injection/transport performance, electron blocking performance, high stability in a thin-film state, and high light emission efficiency as a material for a highly efficient organic EL device having a high durability, and a highly efficient organic EL device having a high durability by using this compound.

Examples of the physical properties that an organic compound to be provided by the present invention should have include (1) having a high hole injection property, (2) having a high mobility of holes, (3) having excellent electron blocking performance, (4) having high stability in a thin-film state, and (5) having an excellent durability for electrons. Further, examples of the physical properties that an organic EL device to be provided by the present invention should have include (1) having high light emission efficiency and (2) having a long device lifetime.

Solution to Problem

In view of the above, in order to achieve the above-mentioned object, the present inventors have designed and chemically synthesized a novel monoamine compound having a triarylamine structure, expecting high hole injection/transport performance of an aromatic tertiary amine structure and the effect on the electric durability and stability in a thin film. Various organic EL devices have been prototyped using the compound, and the properties of the device were intensively evaluated. As a result, the present invention was completed.

[1] An organic EL device, including, between an anode and a cathode, at least a first hole transport layer, a second hole transport layer, a green light-emitting layer, and an electron transport layer in the stated order from a side of the anode, the organic EL device being characterized in that the second hole transport layer, or at least one of stacked films disposed between the first hole transport layer and the electron transport layer contains an arylamine compound represented by the following general formula (1).

(Chem. 3)

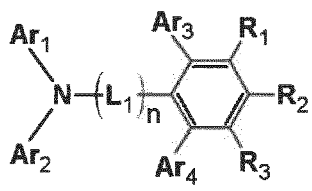

(1)

(In the formula, $Ar_1$, $Ar_2$, $Ar_3$, and $Ar_4$ may be the same as or different from each other, and each represent a substituted or unsubstituted aromatic hydrocarbon group, a substituted or unsubstituted aromatic heterocyclic group, or a substituted or unsubstituted fused polycyclic aromatic group. $L_1$ represents a divalent group of a substituted or unsubstituted aromatic hydrocarbon, a divalent group of a substituted or unsubstituted aromatic heterocyclic, or a divalent group of a substituted or unsubstituted fused polycyclic aromatic. $R_1$, $R_2$, and $R_3$ each represent a hydrogen atom, a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. n represents an integer of 1 to 3.)

[2] An organic EL device including, between an anode and a cathode, at least a first hole transport layer, a second hole transport layer, a green light-emitting layer, and an electron transport layer in the stated order from a side of the anode, the organic EL device being characterized in that the second hole transport layer, or at least one of stacked films disposed between the first hole transport layer and the electron transport layer contains an arylamine compound represented by the following general formula (2).

(Chem. 4)

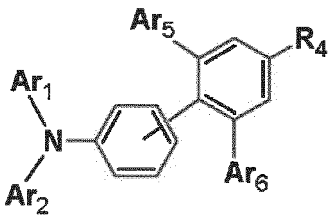

(2)

(In the formula, $Ar_1$ and $Ar_2$ may be the same as or different from each other, and each represent a substituted or unsubstituted aromatic hydrocarbon group, a substituted or unsubstituted aromatic heterocyclic group, or a substituted or unsubstituted fused polycyclic aromatic group. $Ar_5$ and $Ar_6$ may be the same as or different from each other, and each represent a phenyl group, a biphenylyl group, a naphthyl group, a phenanthrenyl group, or a fluorenyl group. $R_4$ represents a hydrogen atom, a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group.)

[3] The organic EL device according to [1] or [2] above, characterized in that the green light-emitting layer contains a host and a phosphorescent dopant, and the host contains at least one first host compound represented by the following chemical formula Host-A and at least one second host compound represented by the following chemical formula Host-B.

(Chem. 5)

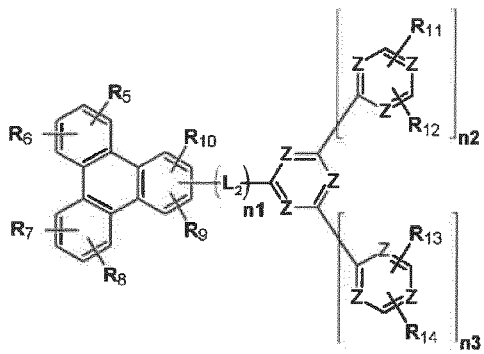

(Host-A)

(In the Host-A, Zs each independently represent N or CRa, and at least one of Zs represents N. $R_5$ to $R_{14}$ and Ra each independently represent a hydrogen atom, a deuterium atom, a fluorine atom, a chlorine atom, a cyano group, a nitro group, a substituted or unsubstituted alkyl group having 1 to 15 carbon atoms, or a substituted or unsubstituted aryl group having 6 to 12 ring carbon atoms. The total number of 6-membered rings substituted with triphenylene groups in the Host-A is six or less. $L_2$ represents a substituted or unsubstituted phenylene group, a substituted or unsubstituted biphenylene group, or a substituted or unsubstituted terphenylene group. n1 to n3 each independently represent 0 or 1, and n1+n2+n3≥1.)

(Chem. 6)

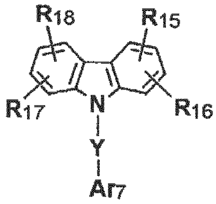

(Host-B)

(In the Host-B, Y represents a single bond, a substituted or unsubstituted arylene group having 6 to 30 ring carbon atoms, or a substituted or unsubstituted heteroarylene group having 5 to 30 ring carbon atoms. $Ar_7$ represents a substituted or unsubstituted aryl group having 6 to 30 ring carbon atoms or a substituted or unsubstituted heteroaryl group having 5 to 30 ring carbon atoms. $R_{15}$ to $R_{18}$ each independently represent a hydrogen atom, a deuterium atom, a fluorine atom, a chlorine atom, a cyano group, a nitro group, an alkyl group having 1 to 15 carbon atoms, a substituted or unsubstituted aryl group having 6 to 50 ring carbon atoms, or a substituted or unsubstituted heteroaryl group having 4 to 50 ring carbon atoms. At least one of $R_{15}$ to $R_{18}$ and $Ar_7$ includes a substituted or unsubstituted triphenylene group or a substituted or unsubstituted carbazole group.)

[4] The organic EL device according to any one of [1] to [3] above, characterized in that the green light-emitting layer contains a host and a phosphorescent dopant, and the phosphorescent dopant is a metal complex containing iridium.

[5] The organic EL device according to any one of [1] to [3] above, characterized in that the green light-emitting layer contains a host and a phosphorescent dopant, and the phosphorescent dopant is a metal complex represented by the following general formula (3).

(Chem. 3)

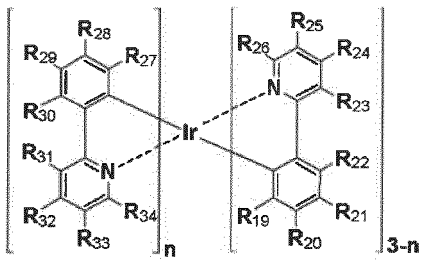

(3)

(In the formula, $R_{19}$ to $R_{34}$ may be the same as or different from each other, and each represent a hydrogen atom, a deuterium atom, a fluorine atom, a chlorine atom, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a trimethylsilyl group, a substituted or unsubstituted aromatic hydrocarbon group, a substituted or unsubstituted aromatic heterocyclic group, a substituted or unsubstituted fused polycyclic aromatic group, a substituted or unsubstituted aryloxy group, or a disubstituted amino group substituted with a group selected from an aromatic hydrocarbon group, an aromatic heterocyclic group, or a fused polycyclic aromatic group. n represents an integer of 1 to 3.)

[6] The organic EL device according to any one of [1] to [5] above, characterized in that the electron transport layer contains a compound having a pyrimidine structure, the compound being represented by the following general formula (4).

(Chem. 8)

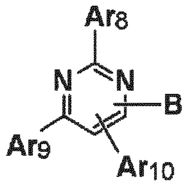

(4)

(In the formula, $Ar_8$ represents a substituted or unsubstituted aromatic hydrocarbon group or a substituted or unsubstituted fused polycyclic aromatic group. $Ar_9$ and $Ar_{10}$ may be the same as or different from each other, and each represent a hydrogen atom, a substituted or unsubstituted aromatic hydrocarbon group, or a substituted or unsubstituted fused polycyclic aromatic group. B represents a monovalent group represented by the following structural formula (5). Here, there is no case that both $Ar_9$ and $Ar_{10}$ are hydrogen atoms.)

(Chem. 9)

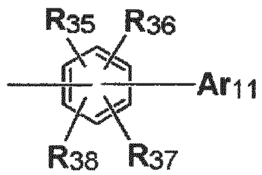

(5)

(In the formula, $Ar_{11}$ represents a substituted or unsubstituted aromatic heterocyclic group, and $R_{35}$ to $R_{38}$ may be the same as or different from each other, and each represent a hydrogen atom, a deuterium atom, a fluorine atom, a chlorine atom, a cyano group, a trifluoromethyl group, a linear or branched alkyl group having 1 to 6 carbon atoms, a substituted or unsubstituted aromatic hydrocarbon group, a substituted or unsubstituted aromatic heterocyclic group, or a substituted or unsubstituted fused polycyclic aromatic group.)

[7] The organic EL device according to any one of [1] to [5] above, characterized in that the electron transport layer contains a compound having a benzoazole structure, the compound being represented by the following general formula (6).

(Chem. 10)

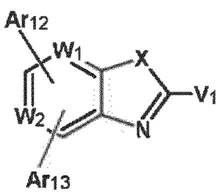

(6)

(In the formula, $Ar_{12}$ and $Ar_{13}$ may be the same as or different from each other, and each represent a hydrogen atom, a deuterium atom, a substituted or unsubstituted aromatic hydrocarbon group, a substituted or unsubstituted fused polycyclic aromatic group, or a substituted or unsubstituted aromatic heterocyclic group. $V_1$ represents a substituted or unsubstituted aromatic hydrocarbon group, a substituted or unsubstituted fused polycyclic aromatic group, a substituted or unsubstituted aromatic heterocyclic group, a linear or branched alkyl group having 1 to 6 carbon atoms which may have a substituted group, a cycloalkyl group having 5 to 10 carbon atoms which may have a substituted group, or a linear or branched alkenyl group having 2 to 6 carbon atoms which may have a substituted

group. X represents an oxygen atom or a sulfur atom. $W_1$ and $W_2$ may be the same as or different from each other, and each represent a carbon atom or a nitrogen atom.)

[8] The organic EL device according to any one of [1] to [7] above, characterized in that the first hole transport layer contains a triphenylamine derivative represented by the following general formula (7) or the following general formula (8).

(Chem. 11)

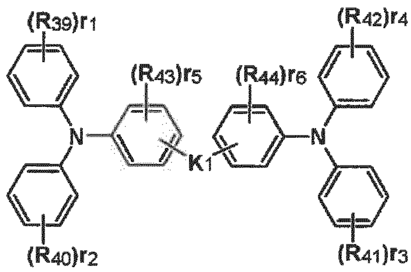

(7)

(In the formula, $R_{39}$ to $R_{44}$ each represent a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. $r_1$ to $r_6$ may be the same as or different from each other, $r_1$ to $r_4$ each represent an integer of 0 to 5, and $r_5$ and $r_6$ each represent an integer of 0 to 4. In a case where any of $r_1$ to $r_6$ is an integer of two or more, a plurality of $R_{39}$, a plurality of $R_{40}$, a plurality of $R_{41}$, a plurality of $R_{42}$, a plurality of $R_{43}$, or a plurality of $R_{44}$ bonded to the same benzene ring may be the same as or different from each other. Further, a benzene ring and a substituted group substituted with a benzene ring, a plurality of substituted groups substituted with the same benzene ring, or benzene rings adjacent to each other via a nitrogen atom may form a ring with a single bond, or may be bonded to each other via a substituted or unsubstituted methylene group, an oxygen atom, or a sulfur atom to form a ring. $K_1$ represents a divalent group represented by any of the following structural formulae (HTM-A) to (HTM-F) or a single bond.)

(Chem. 12)

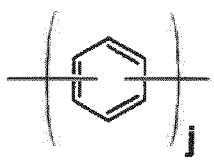

(HTM−A)

(In the formula, j represents an integer of 1 to 3.)

(Chem. 13)

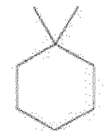

(HTM-B)

(Chem. 14)

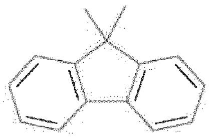

(HTM-C)

(Chem. 15)

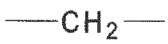

(HTM-D)

(Chem. 16)

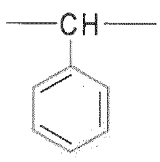

(HTM-E)

(Chem. 17)

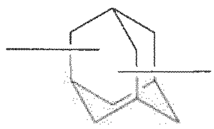

(HTM-F)

(Chem. 18)

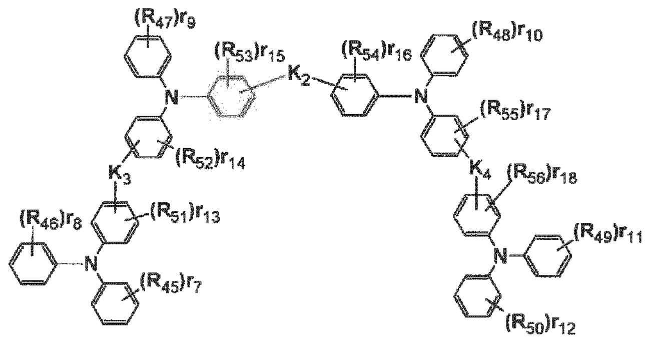

(8)

(In the formula, $R_{45}$ to $R_{56}$ each represent a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. $r_7$ to $r_{18}$ may be the same as or different from each other, $r_7$ to $r_{12}$ each represent an integer of 0 to 5, and $r_{13}$ to $r_{18}$ each represent an integer of 0 to 4. In a case where any of $r_7$ to $r_{18}$ is an integer of two or more, a plurality of $R_{45}$, a plurality of $R_{46}$, a plurality of $R_{47}$, a plurality of $R_{48}$, a plurality of $R_{49}$, a plurality of $R_{50}$, a plurality of $R_{51}$, a plurality of $R_{52}$, a plurality of $R_{53}$, a plurality of $R_{54}$, a plurality of $R_{55}$, or a plurality of $R_{56}$ bonded to the same benzene ring may be the same as or different from each other. Further, a benzene ring and a substituted group substituted with a benzene ring, a plurality of substituted groups substituted with the same benzene ring, or benzene rings adjacent to each other via a nitrogen atom may form a ring with a single bond, or may be bonded to each other via a substituted or unsubstituted methylene group, an oxygen atom, or a sulfur atom to form a ring. $K_2$ to $K_4$ may be the same as or different from each other, and each represent a divalent group represented by any of the structural formulae (HTM-A) to (HTM-F) in the general formula (7), or a single bond.)

Brief Description of Drawings

[Fig. 1] Fig. 1 is a diagram showing Compound (1-1) to Compound (1-12) as favorable specific examples of a compound including an arylamine compound, the compound being represented by the general formula (1) .

[Fig. 2] Fig. 2 is a diagram showing Compound (1-13) to Compound (1-24) as favorable specific examples of a compound including an arylamine compound, the compound being represented by the general formula (1).

[Fig. 3] Fig. 3 is a diagram showing Compound (1-25) to Compound (1-36) as favorable specific examples of a compound including an arylamine compound, the compound being represented by the general formula (1).

[Fig. 4] Fig. 4 is a diagram showing Compound (1-37) to Compound (1-48) as favorable specific examples of a compound including an arylamine compound, the compound being represented by the general formula (1).

[Fig. 5] Fig. 5 is a diagram showing Compound (1-49) to Compound (1-57) as favorable specific examples of a compound including an arylamine compound, the compound being represented by the general formula (1).

[Fig. 6] Fig. 6 is a diagram showing Compound (A-1) to Compound (A-12) as favorable specific examples of a compound represented by the chemical formula (Host-A).

[Fig. 7] Fig. 7 is a diagram showing Compound (A-13) to Compound (A-24) as favorable specific examples of a compound represented by the chemical formula (Host-A).

[Fig. 8] Fig. 8 is a diagram showing Compound (A-25) to Compound (A-36) as favorable specific examples of a compound represented by the chemical formula (Host-A).

[Fig. 9] Fig. 9 is a diagram showing Compound (A-37) to Compound (A-48) as favorable specific examples of a compound represented by the chemical formula (Host-A).

[Fig. 10] Fig. 10 is a diagram showing Compound (A-49) to Compound (A-57) as favorable specific examples of a compound represented by the chemical formula (Host-A).

[Fig. 11] Fig. 11 is a diagram showing Compound (B-1) to Compound (B-12) as favorable specific examples of a compound represented by the chemical formula (Host-B).

[Fig. 12] Fig. 12 is a diagram showing Compound (B-13) to Compound (B-24) as favorable specific examples of a compound represented by the chemical formula (Host-B).

[Fig. 13] Fig. 13 is a diagram showing Compound (B-25) to Compound (B-36) as favorable specific examples of a compound represented by the chemical formula (Host-B).

[Fig. 14] Fig. 14 is a diagram showing Compound (B-37) to Compound (B-48) as favorable specific examples of a compound represented by the chemical formula (Host-B).

[Fig. 15] Fig. 15 is a diagram showing Compound (B-49) to Compound (B-60) as favorable specific examples of a compound represented by the chemical formula (Host-B).

[Fig. 16] Fig. 16 is a diagram showing Compound (B-61) to Compound (B-72) as favorable specific examples of a compound represented by the chemical formula (Host-B).

[Fig. 17] Fig. 17 is a diagram showing Compound (B-73) to Compound (B-76) as favorable specific examples of a compound represented by the chemical formula (Host-B).

[Fig. 18] Fig. 18 is a diagram showing Compound (3-1) to Compound (3-12) as favorable specific examples of a compound (metal complex) represented by the chemical formula (3).

[Fig. 19] Fig. 19 is a diagram showing Compound (3-13) to Compound (3-24) as favorable specific examples of a compound (metal complex) represented by the chemical formula (3).

[Fig. 20] Fig. 20 is a diagram showing Compound (3-25) to Compound (3-29) and Compound (3-31) to Compound (3-33) as favorable specific examples of a compound (metal complex) represented by the chemical formula (3).

[Fig. 21] Fig. 21 is a diagram showing Compound (4-1) to Compound (4-12) as favorable specific examples of a compound having a pyrimidine structure, the compound being represented by the general formula (4).

[Fig. 22] Fig. 22 is a diagram showing Compound (4-13) to Compound (4-24) as favorable specific examples of a compound having a pyrimidine structure, the compound being represented by the general formula (4) .

[Fig. 23] Fig. 23 is a diagram showing Compound (4-25) to Compound (4-36) as favorable specific examples of a compound having a pyrimidine structure, the compound being represented by the general formula (4) .

[Fig. 24] Fig. 24 is a diagram showing Compound (4-37) to Compound (4-48) as favorable specific examples of a compound having a pyrimidine structure, the compound being represented by the general formula (4) .

[Fig. 25] Fig. 25 is a diagram showing Compound (4-49) to Compound (4-60) as favorable specific examples of a compound having a pyrimidine structure, the compound being represented by the general formula (4) .

[Fig. 26] Fig. 26 is a diagram showing Compound (4-61) to Compound (4-69) as favorable specific examples of a compound having a pyrimidine structure, the compound being represented by the general formula (4) .

[Fig. 27] Fig. 27 is a diagram showing Compound (4-70) to Compound (4-78) as favorable specific examples of a compound having a pyrimidine structure, the compound being represented by the general formula (4) .

[Fig. 28] Fig. 28 is a diagram showing Compound (6-1) to Compound (6-12) as favorable specific examples of a compound having a benzoazole structure, the compound being represented by the general formula (6).

[Fig. 29] Fig. 29 is a diagram showing Compound (6-13) to Compound (6-24) as favorable specific examples of a compound having a benzoazole structure, the compound being represented by the general formula (6) .

[Fig. 30] Fig. 30 is a diagram showing Compound (6-25) to Compound (6-36) as favorable specific examples of a compound having a benzoazole structure, the compound being represented by the general formula (6) .

[Fig. 31] Fig. 31 is a diagram showing Compound (6-37) to Compound (6-48) as favorable specific examples of a compound having a benzoazole structure, the compound being represented by the general formula (6) .

[Fig. 32] Fig. 32 is a diagram showing Compound (6-49) to Compound (6-60) as favorable specific examples of a compound having a benzoazole structure, the compound being represented by the general formula (6) .

[Fig. 33] Fig. 33 is a diagram showing Compound (6-61) to Compound (6-72) as favorable specific examples of a compound having a benzoazole structure, the compound being represented by the general formula (6) .

[Fig. 34] Fig. 34 is a diagram showing Compound (6-73) to Compound (6-77) as favorable specific examples of a compound having a benzoazole structure, the compound being represented by the general formula (6) .

[Fig. 35] Fig. 35 is a diagram showing Compound (7-1) to Compound (7-12) as favorable specific examples of a triphenylamine derivative represented by the general formula (7).

[Fig. 36] Fig. 36 is a diagram showing Compound (7-13) to Compound (7-24) as favorable specific examples of a triphenylamine derivative represented by the general formula (7).

[Fig. 37] Fig. 37 is a diagram showing Compound (7-25) to Compound (7-32) as favorable specific examples of a triphenylamine derivative represented by the general formula (7).

[Fig. 38] Fig. 38 is a diagram showing Compound (8-1) to Compound (8-8) as favorable specific examples of a triphenylamine derivative represented by the general formula (8).

[Fig. 39] Fig. 39 is a diagram showing Compound (8-9) to Compound (8-16) as favorable specific examples of a triphenylamine derivative represented by the general formula (8).

[Fig. 40] Fig. 40 is a diagram showing a configuration of EL devices according to Examples 11 to 18 and Comparative Examples 1 to 6.

Mode(s) for Carrying Out the Invention

Specific examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by $Ar_1$ to $Ar_4$ in the general formula (1) include a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, a triphenylenyl group, a pyridyl group, a pyrimidinyl group, a triazinyl group, a furil group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group.

Specific examples of the "substituted group" in the "substituted aromatic hydrocarbon group", "substituted aromatic heterocyclic group", or "substituted fused polycyclic aromatic group" represented by $Ar_1$ to $Ar_4$ in the general formula (1) include a deuterium atom, a cyano group, a nitro group; a halogen atom such as a fluorine atom, a chlorine atom, a bromine atom, and an iodine atom; a linear or branched alkyl group having 1 to 6 carbon atoms such as a methyl group, an ethyl group, an n-propyl group, an isopropyl group, an n-butyl group, an isobutyl group, a tert-butyl group, an n-pentyl group, an isopentyl group, a neopentyl group, and an n-hexyl group; a linear or branched alkyloxy group having 1 to 6 carbon atoms such as a methyloxy group, an ethyloxy group, and a propyloxy group; an alkenyl group such as a vinyl group and an allyl group; an aryloxy group such as a phenyloxy group and a tolyloxy group; an arylalkyloxy group such as a benzyloxy group and a phenethyloxy group; an aromatic hydrocarbon group or a fused polycyclic aromatic group such as a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, and a triphenylenyl group; an aromatic heterocyclic group such as a pyridyl group, a pyrimidinyl group, a triazinyl group, a thienyl group, a furil group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group; an arylvinyl group such as a styryl group and a naphthylvinyl group; and an acyl group such as an acetyl group and a benzoyl group. These substituted groups may be further substituted with the exemplified substituted groups. Further, these substituted groups may form a ring with a single bond, or may be bonded to each other via a substituted or unsubstituted methylene group, an oxygen atom, or a sulfur atom to form a ring.

Specific examples of the "divalent group of an aromatic hydrocarbon", "divalent group of an aromatic heterocyclic", or "divalent group of a fused polycyclic aromatic" in the "divalent group of a substituted or unsubstituted aromatic hydrocarbon", "divalent group of a substituted or unsubstituted aromatic heterocyclic", or "divalent group of a substituted or unsubstituted fused polycyclic aromatic" represented by $L_1$ in the general formula (1) include a phenylene group, a biphenylene group, a terphenylene group, a tetrakisphenylene group, a naphthylene group, an anthrylene group, a phenanthrylene group, a fluorenylene group, a phenanthrolylene group, an indenylene group, a pyrenylene group, a perylenylene group, a fluoranthenylene group, a triphenylenylene group, a pyridinylene group, a pyrimidinylene group, a quinolylene group, an isoquinolylene group, an indolylene group, a carbazolylene group, a quinoxalylene group, a benzimidazolylene group, a pyrazolylene group, a naphthyridinylene group, a phenanthrolinylene group, an acridinylene group, a thiophenylene group, a benzothiophenylene group, a benzothiazolylene group, and a dibenzothiophenylene group. Further, in the case where n is 2 or 3, a plurality of $L_1$ may be the same as or different from each other.

Examples of the "substituted group" in the "divalent group of a substituted aromatic hydrocarbon", "divalent group of a substituted aromatic heterocyclic", or "divalent group of a substituted fused polycyclic aromatic" represented by $L_1$ in the general formula (1) include the similar ones as described for the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1), and aspects similar to those of the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1) can be taken.

Specific examples of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have

a substituted group" represented by $R_1$ to $R_3$ in the general formula (1) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-buthenyl group. Further, these groups may form a ring with a single bond or may be bonded to each other via a substituted or unsubstituted methylene group, an oxygen atom, or a sulfur atom.

Specific examples of the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $R_1$ to $R_3$ in the general formula (1) include a deuterium atom, a cyano group, a nitro group; a halogen atom such as a fluorine atom, a chlorine atom, a bromine atom, and an iodine atom; a linear or branched alkyloxy group having 1 to 6 carbon atoms such as a methyloxy group, an ethyloxy group, and a propyloxy group; an alkenyl group such as a vinyl group and an allyl group; an aryloxy group such as a phenyloxy group and a tolyloxy group; an arylalkyloxy group such as a benzyloxy group and a phenethyloxy group; an aromatic hydrocarbon group or a fused polycyclic aromatic group such as a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, and a triphenylenyl group; and an aromatic heterocyclic group such as a pyridyl group, a pyrimidinyl group, a triazinyl group, a thienyl group, a furil group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group. These substituted groups may be further substituted with the exemplified substituted groups. Further, these substituted groups may form a ring with a single bond, or may be bonded to each other via a substituted or unsubstituted methylene group, an oxygen atom, or a sulfur atom to form a ring.

Specific examples of the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituted group" or "cycloalkyloxy group having 5 to 10 carbon atoms which may have a substituted group" represented by $R_1$ to $R_3$ in the general formula (1) include a methyloxy group, an ethyloxy group, an n-propyloxy group, an isopropyloxy group, an n-butyloxy group, a tert-butyloxy group, an n-pentyloxy group, an n-hexyloxy group, a cyclopentyloxy group, a cyclohexyloxy group, a cycloheptyloxy group, a cyclooctyloxy group, a 1-adamantyloxy group, and a 2-adamantyloxy group. Further, these groups may form a ring with a single bond, or may be bonded to each other via a substituted or unsubstituted methylene group, an oxygen atom, or a sulfur atom to form a ring.

Examples of the "substituted group" in the "linear or branched alkyloxy group having 1 to 6 carbon atoms which has a substituted group" or "cycloalkyloxy group having 5 to 10 carbon atoms which has a substituted group" represented by $R_1$ to $R_3$ in the general formula (1) include the similar ones as described for the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $Ar_1$ to $Ar_4$ in the above-mentioned general formula (1), and aspects similar to those of the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $Ar_1$ to $Ar_4$ in the above-mentioned general formula (1) can be taken.

Examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by $R_1$ to $R_3$ in the general formula (1) include the similar ones as described for the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by $Ar_1$ to $Ar_4$ in $Ar_1$ to $Ar_4$ in the above-mentioned general formula (1). Further, these groups may form a ring with a single bond or may be bonded to each other via a substituted or unsubstituted methylene group, an oxygen atom, or a sulfur atom.

Further, these groups may each have a substituted group. Examples of such a substituted group include the similar ones as described for the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1), and aspects similar to those of the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1) can be taken.

Specific examples of the "aryloxy group" in the "substituted or unsubstituted aryloxy group" represented by $R_1$ to $R_3$ in the general formula (1) include a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy

group, and a perylenyloxy group. Further, these groups may form a ring with a single bond, or may be bonded to each other via a substituted or unsubstituted methylene group, an oxygen atom, or a sulfur atom to form a ring. Further, these groups may each have a substituted group. Examples of such a substituted group include the similar ones as described for the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1), and aspects similar to those of the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1) can be taken.

As $Ar_1$ and $Ar_2$ in the general formula (1), a "substituted or unsubstituted aromatic hydrocarbon group" or a "substituted or unsubstituted fused polycyclic aromatic group" is favorable, a phenyl group, a naphthyl group, a phenanthrenyl group, or a fluorenyl group is more favorable, and a phenyl group having a substituted group or a fluorenyl group having a substituted group is particularly favorable. Here, a phenyl group, a biphenylyl group, a terphenylyl group, a naphthyl group, a phenanthrenyl group, or a fluorenyl group is favorable as the substituted group of a phenyl group, and a methyl group or a phenyl group is favorable as the substituted group of a fluorenyl group.

As $Ar_3$ or $Ar_4$ in the general formula (1), a "substituted or unsubstituted aromatic hydrocarbon group" or a "substituted or unsubstituted fused polycyclic aromatic group" is favorable, a phenyl group, a biphenylyl group, a naphthyl group, a phenanthrenyl group, or a fluorenyl group is more favorable, and an unsubstituted phenyl group, an unsubstituted biphenylyl group, an unsubstituted naphthyl group, or a fluorenyl group having a substituted group is more favorable. Here, as the substituted group of a fluorenyl group, a methyl group or a phenyl group is favorable.

As $L_1$ in the general formula (1), a "divalent group of a substituted or unsubstituted aromatic hydrocarbon" or a "divalent group of a substituted or unsubstituted fused polycyclic aromatic" is favorable, a divalent group formed by removing two hydrogen atoms from benzene, biphenyl, naphthalene, or phenanthrene is more favorable, and a divalent group formed by removing two hydrogen atoms from benzene, i.e., a phenylene group is particularly favorable. In this case, an unsubstituted phenylene group is favorable, and the bonding mode of a phenylene group is favorably bonding at the para-meta position or bonding between para positions, i.e., a 1,3-phenylene group or a 1,4-phenylene group is favorable.

n representing the number of $L_1$ represents an integer of 1 to 3, and is favorably one.

As $R_1$ and $R_3$ in the general formula (1), a hydrogen atom or a deuterium atom is favorable, and a hydrogen atom is more favorable from the viewpoint of synthesis.

As $Ar_1$ and $Ar_2$ in the general formula (1), a "substituted or unsubstituted aromatic hydrocarbon group" or a "substituted or unsubstituted fused polycyclic aromatic group" is favorable, a phenyl group, a biphenylyl group, a naphthyl group, a phenanthrenyl group, or a fluorenyl group is more favorable, and an unsubstituted phenyl group, an unsubstituted biphenylyl group, an unsubstituted naphthyl group, or a fluorenyl group having a substituted group is more favorable. Here, as the substituted group of a fluorenyl group, a methyl group or a phenyl group is favorable.

Specific examples of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by $R_4$ in the general formula (2) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-buthenyl group.

Specific examples of the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $R_4$ in the general formula (2) include a deuterium atom, a cyano group, a nitro group; a halogen atom such as a fluorine atom, a chlorine atom, a bromine atom, and an iodine atom; a linear or branched alkyloxy group having 1 to 6 carbon atoms such as a methyloxy group, an ethyloxy group, and a propyloxy group; an alkenyl group such as a vinyl group and an allyl group; an aryloxy group such as a phenyloxy group and a tolyloxy group; an arylalkyloxy group such as a benzyloxy group and a phenethyloxy group; an aromatic hydrocarbon group or a fused polycyclic aromatic group such as a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, and a triphenylenyl group; and an aromatic heterocyclic group such as a pyridyl group, a pyrimidinyl group, a triazinyl group, a thienyl group, a furil group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group.

Specific examples of the "linear or branched alkyloxy group having 1 to 6 carbon atoms" or "cycloalkyloxy group having 5 to 10 carbon atoms" in the "linear or branched alkyloxy group having 1 to 6 carbon atoms which may have a

substituted group" or "cycloalkyloxy group having 5 to 10 carbon atoms which may have a substituted group" represented by $R_4$ in the general formula (2) include a methyloxy group, an ethyloxy group, an n-propyloxy group, an isopropyloxy group, an n-butyloxy group, a tert-butyloxy group, an n-pentyloxy group, an n-hexyloxy group, a cyclopentyloxy group, a cyclohexyloxy group, a cycloheptyloxy group, a cyclooctyloxy group, a 1-adamantyloxy group, and a 2-adamantyloxy group, and similar aspects can be taken.

Examples of the "substituted group" in the "linear or branched alkyloxy group having 1 to 6 carbon atoms which has a substituted group" or "cycloalkyloxy group having 5 to 10 carbon atoms which has a substituted group" represented by $R_4$ in the general formula (2) include the similar ones as described for the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $Ar_1$ to $Ar_4$ in the above-mentioned general formula (1), and aspects similar to those of the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $Ar_1$ to $Ar_4$ in the above-mentioned general formula (1) can be taken.

Examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by $R_4$ in the general formula (2) include the similar ones as described for the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by $Ar_2$ to $Ar_4$ in the above-mentioned general formula (1). Further, these groups may each have a substituted group. Examples of such a substituted group include the similar ones as described for the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1), and aspects similar to those of the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1) can be taken.

Specific examples of the "aryloxy group" in the "substituted or unsubstituted aryloxy group" represented by $R_4$ in the general formula (2) include a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy group, and a perylenyloxy group. Further, these groups may each have a substituted group. Examples of such a substituted group include the similar ones as described for the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1), and aspects similar to those of the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1) can be taken.

As $R_4$ in the general formula (2), a hydrogen atom or a "substituted or unsubstituted aromatic hydrocarbon group" is favorable, a phenyl group or a biphenylyl group is more favorable, and an unsubstituted phenyl group is more favorable.

As $Ar_5$ and $Ar_6$ in the general formula (2), a phenyl group, a biphenylyl group, a naphthyl group, a phenanthrenyl group, or a fluorenyl group is more favorable, and an unsubstituted phenyl group, an unsubstituted biphenylyl group, an unsubstituted naphthyl group, or a fluorenyl group including a substituted group is more favorable. Here, as the substituted group of the fluorenyl group, a methyl group or a phenyl group is favorable.

Specific examples of the "substituted or unsubstituted alkyl group having 1 to 15 carbon atoms" represented by $R_5$ to $R_{14}$ and Ra in the general formula (HOST-A) include a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, an iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl

group, a 2,3-dinitro-t-butyl group, a 1,2,3-trinitropropyl group, a cyclopropyl group, a cyclobutyl group, a cyclopentyl group, a cyclohexyl group, a 4-methylcyclohexyl group, a 1-adamantyl group, a 2-adamantyl group, a 1-norbornyl group, and a 2-norbornyl group.

Examples of the "substituted group" in the "substituted or unsubstituted alkyl group having 1 to 15 carbon atoms" represented by $R_5$ to $R_{14}$ and Ra in the general formula (HOST-A) include the similar ones as described for the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $R_1$ to $R_3$ in the above-mentioned general formula (1), and aspects similar to those of the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $R_1$ to $R_3$ in the above-mentioned general formula (1) can be taken.

Specific examples of the "substituted or unsubstituted aryl group having 6 to 12 ring carbon atoms" represented by $R_5$ to $R_{14}$ and Ra in the general formula (HOST-A) include a phenyl group, a biphenylyl group, a 1-naphthyl group, a 2-naphthyl group, a fluorophenyl group, a difluorophenyl group, a trifluorophenyl group, a tetrafluorophenyl group, a pentafluorophenyl group, a tolyl group, a nitrophenyl group, a cyanophenyl group, a fluorobiphenylyl group, a nitrobiphenylyl group, a cyanobiphenyl group, a cyanonaphthyl group, a nitronaphthyl group, and a fluoronaphthyl group. Of the above, a phenyl group or a biphenylyl group is particularly favorable.

Examples of the "substituted group" in the "substituted or unsubstituted aryl group having 6 to 12 ring carbon atoms" represented by $R_5$ to $R_{14}$ and Ra in the general formula (HOST-A) include the similar ones as described for the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1), and aspects similar to those of the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1) can be taken.

Specific examples of the "alkyl group having 1 to 15 carbon atoms" represented by $R_{15}$ to $R_{18}$ in the general formula (HOST-B) include a methyl group, an ethyl group, a propyl group, an isopropyl group, an n-butyl group, an s-butyl group, an isobutyl group, a t-butyl group, an n-pentyl group, an n-hexyl group, an n-heptyl group, an n-octyl group, a hydroxymethyl group, a 1-hydroxyethyl group, a 2-hydroxyethyl group, a 2-hydroxyisobutyl group, a 1,2-dihydroxyethyl group, a 1,3-dihydroxyisopropyl group, a 2,3-dihydroxy-t-butyl group, a 1,2,3-trihydroxypropyl group, a chloromethyl group, a 1-chloroethyl group, a 2-chloroethyl group, a 2-chloroisobutyl group, a 1,2-dichloroethyl group, a 1,3-dichloroisopropyl group, a 2,3-dichloro-t-butyl group, a 1,2,3-trichloropropyl group, a bromomethyl group, a 1-bromoethyl group, a 2-bromoethyl group, a 2-bromoisobutyl group, a 1,2-dibromoethyl group, a 1,3-dibromoisopropyl group, a 2,3-dibromo-t-butyl group, a 1,2,3-tribromopropyl group, an iodomethyl group, a 1-iodoethyl group, a 2-iodoethyl group, a 2-iodoisobutyl group, a 1,2-diiodoethyl group, a 1,3-diiodoisopropyl group, a 2,3-diiodo-t-butyl group, a 1,2,3-triiodopropyl group, an aminomethyl group, a 1-aminoethyl group, a 2-aminoethyl group, a 2-aminoisobutyl group, a 1,2-diaminoethyl group, a 1,3-diaminoisopropyl group, a 2,3-diamino-t-butyl group, a 1,2,3-triaminopropyl group, a cyanomethyl group, a 1-cyanoethyl group, a 2-cyanoethyl group, a 2-cyanoisobutyl group, a 1,2-dicyanoethyl group, a 1,3-dicyanoisopropyl group, a 2,3-dicyano-t-butyl group, a 1,2,3-tricyanopropyl group, a nitromethyl group, a 1-nitroethyl group, a 2-nitroethyl group, a 2-nitroisobutyl group, a 1,2-dinitroethyl group, a 1,3-dinitroisopropyl group, a 2,3-dinitro-t-butyl group, a 1,2,3-trinitropropyl group, a cyclopropyl group, a cyclobutyl group, a cyclopentyl group, a cyclohexyl group, a 4-methylcyclohexyl group, a 1-adamantyl group, a 2-adamantyl group, a 1-norbornyl group, and a 2-norbornyl group.

Examples of the "substituted group" in the "alkyl group having 1 to 15 carbon atoms" represented by $R_{15}$ to $R_{18}$ in the general formula (HOST-B) include the similar ones as described for the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $R_1$ to $R_3$ in the above-mentioned general formula (1), and aspects similar to those of the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $R_1$ to $R_3$ in the above-mentioned general formula (1) can be taken.

Specific examples of the "substituted or unsubstituted aryl group having 6 to 50 ring carbon atoms" or "substituted or unsubstituted heteroaryl group having 4 to 50 ring carbon atoms " represented by $R_{15}$ to $R_{18}$ in the general formula (HOST-B) include a phenyl group, a biphenyl group, a terphenyl group, a naphthyl group, an anthryl group, a phenanthryl group, a fluorenyl group, an indenyl group, a pyrenyl group, an acetonaphthenyl group, a fluoranthenyl group, a triphenylenyl group, a pyridyl group, a pyranyl group, a quinolyl group, an isoquinolyl group, a benzofuranyl group, a benzothienyl group, an indolyl group, a carbazolyl group, a benzoxazolyl group, a benzothiazolyl group, a quinoxalyl group, a benzimidazolyl group, a dibenzofuranyl group, and a dibenzothienyl group.

Examples of the "substituted group" in the "substituted or unsubstituted aryl group having 6 to 50 ring carbon atoms" or "substituted or unsubstituted heteroaryl group having 4 to 50 ring carbon atoms" represented by $R_{15}$ to $R_{18}$ in the general formula (HOST-B) include the similar ones as described for the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1), and aspects similar to those of the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1) can be taken.

Specific examples of the "substituted or unsubstituted arylene group having 6 to 30 ring carbon atoms" or "substituted or unsubstituted heteroarylene group having 5 to 30 ring carbon atoms" represented by Y in the general formula (HOST-B) include a phenylene group, a biphenylene group, a terphenylene group, a naphthylene group, an anthrylene group, a phenanthrylene group, a fluorenylene group, an indenylene group, a pyrenylene group, an acetonaphthenylene group, a fluoranthenylene group, a triphenylenylene group, a pyridylene group, a pyranylene group, a quinolylene group, an isoquinolylene group, a benzofuranylene group, a benzothienylene group, an indolylene group, a carbazolylene group, a benzoxazolylene group, a benzothiazolylene group, a quinoxalylene group, a benzimidazolylene group, a pyrazolylene group, a dibenzofuranylene group, and a dibenzothienylene group.

Examples of the "substituted group" in the "substituted or unsubstituted arylene group having 6 to 30 ring carbon atoms" or "substituted or unsubstituted heteroarylene group having 5 to 30 ring carbon atoms" represented by Y in the general formula (HOST-B) include the similar ones as described for the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1), and aspects similar to those of the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1) can be taken.

Specific examples of the "substituted or unsubstituted aryl group having 6 to 30 ring carbon atoms" or "substituted or unsubstituted heteroaryl group having 5 to 30 ring carbon atoms" represented by $Ar_7$ in the general formula (HOST-B) include a phenyl group, a naphthyl group, an anthracenyl group, a phenanthryl group, a biphenyl group, a p-terphenyl group, an m-terphenyl group, a quarterphenyl group, a fluorenyl group, a triphenylene group, a biphenylene group, a pyrenyl group, a benzofluoranthenyl group, a chrysenyl group, a phenylnaphthyl group, a naphthylphenyl group, a pyridyl group, a quinolyl group, an isoquinolyl group, a benzofuryl group, a benzothienyl group, an indolyl group, a benzoxazolyl group, a benzothiazolyl group, a quinoxalyl group, a benzimidazolyl group, a dibenzofuryl group, a dibenzothienyl group, and a carbazolyl group.

Examples of the "substituted group" in the "substituted or unsubstituted aryl group having 6 to 30 ring carbon atoms" or "substituted or unsubstituted heteroaryl group having 5 to 30 ring carbon atoms" represented by $Ar_7$ in the general formula (HOST-B) include the similar ones as described for the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1), and aspects similar to those of the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1) can be taken.

Specific examples of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by $R_{19}$ to $R_{34}$ in the general formula (3) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-buthenyl group. Further, these groups may form a ring with a single bond, or may be bonded to each other via a substituted or unsubstituted methylene group, an oxygen atom, or a sulfur atom to form a ring.

Examples of the "substituted group" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by $R_{19}$ to $R_{34}$ in the general formula (3) include the similar ones as described for the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $R_1$ to $R_3$ in the above-mentioned general formula (1), and aspects similar to those of the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $R_1$ to $R_3$ in the above-mentioned general formula (1) can be taken.

Specific examples of the "aryloxy group" in the "substituted or unsubstituted aryloxy group" represented by $R_{19}$ to $R_{34}$ in the general formula (3) include a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy group, and a perylenyloxy group. Further, these groups may form a ring with a single bond, or may be bonded to each other via a substituted or unsubstituted methylene group, an oxygen atom, or a sulfur atom to form a ring.

Examples of the "substituted group" in the "substituted or unsubstituted aryloxy group" represented by $R_{19}$ to $R_{34}$ in the general formula (3) include the similar ones as described for the "substituted group" in the "substituted or unsubstituted aryloxy group" represented by $R_1$ to $R_3$ in the above-mentioned general formula (1), and aspects similar to those of the "substituted group" in the "substituted or unsubstituted aryloxy group" represented by $R_1$ to $R_3$ in the above-mentioned general formula (1) can be taken.

The "substituted or unsubstituted aromatic hydrocarbon group", "substituted or unsubstituted aromatic heterocyclic group", "substituted or unsubstituted fused polycyclic aromatic group", or "substituted or unsubstituted aryloxy group" represented by $R_{19}$ to $R_{34}$ in the general formula (3) can specifically be, but not limited to, a phenyl group, a naphthyl group, an anthracenyl group, a phenanthryl group, a naphthacenyl group, a pyrenyl group, a biphenylyl group, a p-terphenyl group, an m-terphenyl group, a chrysenyl group, a triphenylenyl group, a perylenyl group, an indenyl group, a furanyl group, a thiophenyl group, a pyrrolyl group, a pyrazolyl group, an imidazolyl group, a triazolyl group, an oxazolyl group, a thiazolyl group, an oxadiazolyl group, a thiadiazolyl group, a pyridyl group, a pyrimidinyl group, a pyrazinyl group, a triazinyl group, a benzofuranyl group, a benzothiophenyl group, a benzimidazolyl group, an indolyl group, a quinolinyl group, an isoquinolinyl group, a quinazolinyl group, a quinoxalinyl group, a naphthyridinyl group, a benzoxazinyl group, a benzthiazinyl group, an acridinyl group, a phenazinyl group, a phenothiazinyl group, a phenoxazinyl group, or a combination thereof.

Examples of the "substituted group" in the "substituted or unsubstituted aromatic hydrocarbon group", "substituted or unsubstituted aromatic heterocyclic group", "substituted or unsubstituted fused polycyclic aromatic group", or "substituted or unsubstituted aryloxy group" represented by $R_{19}$ to $R_{34}$ in the general formula (3) include the similar ones as described for the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1), and aspects similar to those of the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1) can be taken.

Specific examples of the "aromatic hydrocarbon group" or "fused polycyclic aromatic group" in the "substituted or unsubstituted aromatic hydrocarbon group" or "substituted or unsubstituted fused polycyclic aromatic group" represented by $Ar_8$ to $Ar_{10}$ in the general formula (4) include a phenyl group, a biphenylyl group, a terphenylyl group, a tetrakisphenyl group, a styryl group, a naphthyl group, an anthracenyl group, an acenaphthenyl group, a phenanthrenyl group, a fluorenyl group, an indenyl group, a pyrenyl group, a perylenyl group, a fluoranthenyl group, and a triphenylenyl group.

Examples of the "substituted group" in the "substituted aromatic hydrocarbon group" or "substituted fused polycyclic aromatic group" represented by $Ar_8$ to $Ar_{10}$ in the general formula (4) include the similar ones as described for the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1), and aspects similar to those of the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1) can be taken.

Specific examples of the "aromatic heterocyclic group" in the "substituted or unsubstituted aromatic heterocyclic group" represented by $Ar_{11}$ in the structural formula (5) include a triazinyl group, a pyridyl group, a pyrimidinyl group, a furil group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group.

Examples of the "substituted group" in the "substituted aromatic heterocyclic group" represented by $Ar_{11}$ in the structural formula (5) include the similar ones as described for the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1), and aspects similar to those of the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1) can be taken.

Specific examples of the "linear or branched alkyl group having 1 to 6 carbon atoms" represented by $R_{35}$ to $R_{38}$ in the structural formula (5) include a methyl group, an ethyl group, an n-propyl group, an i-propyl group, an n-butyl group, a 2-methylpropyl group, a t-butyl group, an n-pentyl group, a 3-methylbutyl group, a tert-pentyl group, an n-hexyl group, an iso-hexyl group, and a tert-hexyl group.

Specific examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by $R_{35}$ to $R_{38}$ in the structural formula (5) include a phenyl group, a biphenylyl group, a terphenylyl group, a tetrakisphenyl group, a styryl group, a naphthyl group, an anthracenyl group, an acenaphthenyl group, a phenanthrenyl group, a fluorenyl group, an indenyl group, a pyrenyl group, a perylenyl group, a fluoranthenyl group, a triphenylenyl group, a triazinyl group, a pyridyl group, a pyrimidinyl group, a furil group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group.

Examples of the "substituted group" in the "substituted aromatic hydrocarbon group", "substituted aromatic heterocyclic group", or "substituted fused polycyclic aromatic group" represented by $R_{35}$ to $R_{38}$ in the structural formula (5) include the similar ones as described for the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1), and aspects similar to those of the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1) can be taken.

As $Ar_8$ in the general formula (4), a phenyl group, a biphenylyl group, a naphthyl group, an anthracenyl group, an acenaphthenyl group, a phenanthrenyl group, a fluorenyl group, an indenyl group, a pyrenyl group, a perylenyl group, a fluoranthenyl group, or a triphenylenyl group is favorable, and a phenyl group, a biphenylyl group, a naphthyl group, an anthracenyl group, a phenanthrenyl group, a pyrenyl group, a fluoranthenyl group, or a triphenylenyl group is more favorable. Here, the phenyl group favorably has a substituted or unsubstituted fused polycyclic aromatic group as a substituted group, and more favorably has a substituted group selected from the group consisting of a naphthyl group, an anthracenyl group, a phenanthrenyl group, a pyrenyl group, a fluoranthenyl group, and a triphenylenyl group.

As $Ar_9$ in the general formula (4), a phenyl group having a substituted group is favorable. As the substituted group in this case, an aromatic hydrocarbon group such as a phenyl group, a biphenylyl group, and a terphenyl group, or a fused polycyclic aromatic group such as a naphthyl group, an anthracenyl group, an acenaphthenyl group, a phenanthrenyl group, a fluorenyl group, an indenyl group, a pyrenyl group, a perylenyl group, a fluoranthenyl group, and a triphenylenyl group is favorable, and a phenyl group, a naphthyl group, an anthracenyl group, a phenanthrenyl group, a pyrenyl group, a fluoranthenyl group, or a triphenylenyl group is more favorable.

As $Ar_{10}$ in the general formula (4), a phenyl group having a substituted group is favorable. As the substituted group in this case, an aromatic hydrocarbon group such as a phenyl group, a biphenylyl group, and a terphenyl group, or a fused polycyclic aromatic group such as a naphthyl group, an anthracenyl group, an acenaphthenyl group, a phenanthrenyl group, a fluorenyl group, an indenyl group, a pyrenyl group, a perylenyl group, a fluoranthenyl group, and a triphenylenyl group is favorable, and a phenyl group, a naphthyl group, an anthracenyl group, a phenanthrenyl group, a pyrenyl group, a fluoranthenyl group, or a triphenylenyl group is more favorable.

As $Ar_{11}$ in the structural formula (5), a nitrogen-containing heterocyclic group such as a triazinyl group, a pyridyl group, a pyrimidinyl group, a pyrrolyl group, a quinolyl group, an isoquinolyl group, an indolyl group, a carbazolyl group, a benzoxazolyl group, a benzothiazolyl group, a quinoxalinyl group, a benzimidazolyl group, a pyrazolyl group, a naphthyridinyl group, a phenanthrolinyl group, an acridinyl group, and a carbolinyl group is favorable, a triazinyl group, a pyridyl group, a pyrimidinyl group, a quinolyl group, an isoquinolyl group, an indolyl group, a quinoxalinyl group, a benzimidazolyl group, a naphthyridinyl group, a phenanthrolinyl group, or an acridinyl group is more favorable, or a pyridyl group, a pyrimidinyl group, a quinolyl group, an isoquinolyl group, an indolyl group, a quinoxalinyl group, a benzimidazolyl group, a phenanthrolinyl group, or an acridinyl group is particularly favorable.

Specific examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by $Ar_{12}$, $Ar_{13}$, and $V_1$ in the general formula (6) include a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furil group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group.

Examples of the "substituted group" in the "substituted or unsubstituted aromatic hydrocarbon group", "substituted or unsubstituted aromatic heterocyclic group", or "substituted or unsubstituted fused polycyclic aromatic group" represented by $Ar_{12}$, $Ar_{13}$, and $V_1$ in the structural formula (6) include the similar ones as described for the "substituted group" in the "substituted aromatic hydrocarbon group", "substituted aromatic heterocyclic group", or "substituted fused

polycyclic aromatic group" represented by $Ar_1$ to $Ar_4$ in the above-mentioned general formula (1), and aspects similar to those of the "substituted group" in the "substituted aromatic hydrocarbon group", "substituted aromatic heterocyclic group", or "substituted fused polycyclic aromatic group" represented by $Ar_1$ to $Ar_4$ in the above-mentioned general formula (1) can be taken.

Specific examples of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by $V_1$ in the general formula (6) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-buthenyl group.

Examples of the "substituted group" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by $V_1$ in the general formula (6) include the similar ones as described for the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $R_1$ to $R_3$ in the above-mentioned general formula (1), and aspects similar to those of the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $R_1$ to $R_3$ in the above-mentioned general formula (1) can be taken.

Specific examples of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by $R_{39}$ to $R_{44}$ in the general formula (7) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-buthenyl group. Further, these groups may form a ring with a single bond or may be bonded to each other via a substituted or unsubstituted methylene group, an oxygen atom, or a sulfur atom to form a ring.

Specific examples of the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $R_{39}$ to $R_{44}$ in the general formula (7) include a deuterium atom, a cyano group, a nitro group; a halogen atom such as a fluorine atom, a chlorine atom, a bromine atom, and an iodine atom; a linear or branched alkyloxy group having 1 to 6 carbon atoms such as a methyloxy group, an ethyloxy group, and a propyloxy group; an alkenyl group such as a vinyl group and an allyl group; an aryloxy group such as a phenyloxy group and a tolyloxy group; an arylalkyloxy group such as a benzyloxy group and a phenethyloxy group; an aromatic hydrocarbon group or a fused polycyclic aromatic group such as a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, and a triphenylenyl group; and an aromatic heterocyclic group such as a pyridyl group, a pyrimidinyl group, a triazinyl group, a thienyl group, a furil group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group. These substituted groups may be further substituted with the exemplified substituted groups. Further, these substituted groups may form a ring with a single bond, or may be bonded to each other via a substituted or unsubstituted methylene group, an oxygen atom, or a sulfur atom to form a ring.

Specific examples of the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituted group" or "cycloalkyloxy group having 5 to 10 carbon atoms which may have a substituted group" represented by $R_{39}$ to $R_{44}$ in the general formula (7) include a methyloxy group, an ethyloxy group, an n-propyloxy group, an isopropyloxy group, an n-butyloxy group, a tert-butyloxy group, an n-pentyloxy group, an n-hexyloxy group, a cyclopentyloxy group, a cyclohexyloxy group, a cycloheptyloxy group, a cyclooctyloxy group, a 1-adamantyloxy group, and a 2-adamantyloxy group. Further, these groups may form a ring with a single bond, or may be bonded to each other via a substituted or unsubstituted methylene group, an oxygen atom, or a sulfur atom to form a ring.

Further, these groups may each have a substituted group. Examples of such a substituted group include the similar ones as described for the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $R_{39}$ to $R_{44}$ in the above-mentioned general formula (7), and aspects similar to those of the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $R_{39}$ to $R_{44}$ in the above-mentioned general formula (7)can be taken.

Specific examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by $R_{39}$ to $R_{44}$ in the general formula (7) include a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, a triphenylenyl group, a pyridyl group, a pyrimidinyl group, a triazinyl group, a furil group, a pyrrolyl group, a thienyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group. Further, these groups may form a ring with a single bond or may be bonded to each other via a substituted or unsubstituted methylene group, an oxygen atom, or a sulfur atom to form a ring.

Specific examples of the "substituted group" in the "substituted or unsubstituted aromatic hydrocarbon group", "substituted or unsubstituted aromatic heterocyclic group", or "substituted or unsubstituted fused polycyclic aromatic group" represented by $R_{39}$ to $R_{44}$ in the general formula (7) include a deuterium atom, a cyano group, a nitro group; a halogen atom such as a fluorine atom, a chlorine atom, a bromine atom, and an iodine atom; a linear or branched alkyl group having 1 to 6 carbon atoms such as a methyl group, an ethyl group, an n-propyl group, an isopropyl group, an n-butyl group, an isobutyl group, a tert-butyl group, an n-pentyl group, an isopentyl group, a neopentyl group, and an n-hexyl group; a linear or branched alkyloxy group having 1 to 6 carbon atoms such as a methyloxy group, an ethyloxy group, and a propyloxy group; an alkenyl group such as a vinyl group and an allyl group; an aryloxy group such as a phenyloxy group and a tolyloxy group; an arylalkyloxy group such as a benzyloxy group and a phenethyloxy group; an aromatic hydrocarbon group or a fused polycyclic aromatic group such as a phenyl group, a biphenylyl group, a terphenylyl group, a naphthyl group, an anthracenyl group, a phenanthrenyl group, a fluorenyl group, an indenyl group, a pyrenyl group, a perylenyl group, a fluoranthenyl group, and a triphenylenyl group; an aromatic heterocyclic group such as a pyridyl group, a pyrimidinyl group, a triazinyl group, a thienyl group, a furil group, a pyrrolyl group, a quinolyl group, an isoquinolyl group, a benzofuranyl group, a benzothienyl group, an indolyl group, a carbazolyl group, a benzoxazolyl group, a benzothiazolyl group, a quinoxalinyl group, a benzimidazolyl group, a pyrazolyl group, a dibenzofuranyl group, a dibenzothienyl group, and a carbolinyl group; an arylvinyl group such as a styryl group and a naphthylvinyl group; an acyl group such as an acetyl group and a benzoyl group; and a silyl group such as a trimethylsilyl group and a triphenylsilyl group. These substituted groups may be further substituted with the exemplified substituted groups. Further, these substituted groups may form a ring with a single bond, or may be bonded to each other via a substituted or unsubstituted methylene group, an oxygen atom, or a sulfur atom to form a ring.

Specific examples of the "aryloxy group" in the "substituted or unsubstituted aryloxy group" represented by $R_{39}$ to $R_{44}$ in the general formula (7) include a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy group, and a perylenyloxy group. Further, these groups may form a ring with a single bond, or may be bonded to each other via a substituted or unsubstituted methylene group, an oxygen atom, or a sulfur atom to form a ring.

Examples of the "substituted group" in the "substituted or unsubstituted aryloxy group" represented by $R_{39}$ to $R_{44}$ in the general formula (7) include the similar ones as described for the "substituted group" in the "substituted or unsubstituted aryloxy group" represented by $R_1$ to $R_3$ in the above-mentioned general formula (1), and aspects similar to those of the "substituted group" in the "substituted or unsubstituted aryloxy group" represented by $R_1$ to $R_3$ in the above-mentioned general formula (1) can be taken.

In the general formula (7), $r_1$ to $r_6$ may be the same as or different from each other, $r_1$ to $r_4$ each represent an integer of 0 to 5, and $r_5$ and $r_6$ each represent an integer of 0 to 4. In the case where any of $r_1$ to $r_4$ is an integer of 2 to 5 or in the case where $r_5$ or $r_6$ is an integer of 2 to 4, a plurality of $R_{39}$, a plurality of $R_{40}$, a plurality of $R_{41}$, a plurality of $R_{42}$, a plurality of $R_{43}$, or a plurality of $R_{44}$ bonded to the same benzene ring may be the same as or different from each other, may form a ring with a single bond, or may be bonded to each other via a substituted or unsubstituted methylene group, an oxygen atom, or a sulfur atom to form a ring.

Examples of the "divalent linking group" represented by $K_1$ in the general formula (7) include a divalent group such as a "linear or branched alkylene group having 1 to 6 carbon atoms" such as a methylene group, an ethylene group, an n-propylene group, an isopropylene group, an n-butylylene group, an isobutylene group, a tert-butylylene group, an

n-pentylylene group, an isopentylylene group, an neopentylylene group, and an n-hexylylene group; a "cycloalkylene group having 5 to 10 carbon atoms" such as a cyclopentylylene group, a cyclohexylylene group, and an adamantylylene group; a "linear or branched alkenylene group having 2 to 6 carbon atoms" such as a vinylene group, an arylene group, an isopropenylene group, and a butenylene group; a "divalent group of an aromatic hydrocarbon" formed by removing two hydrogen atoms from an aromatic hydrocarbon such as benzene, biphenyl, terphenyl, and tetrakisphenyl; and a "divalent group of a fused polycyclic aromatic" formed by removing two hydrogen atoms from a fused polycyclic aromatic such as naphthalene, anthracene, acenaphthalene, fluorene, phenanthrene, indan, pyrene, and triphenylene.

Further, these divalent groups may each have a substituted group. Examples of the substituted group of the "linear or branched alkylene group having 1 to 6 carbon atoms", "cycloalkylene group having 5 to 10 carbon atoms", or "linear or branched alkenylene group having 2 to 6 carbon atoms" include the similar ones as described for the "substituted group" in the "linear or branched alkyl group having 1 to 6 carbon atoms which has a substituted group", "cycloalkyl group having 5 to 10 carbon atoms which has a substituted group", or "linear or branched alkenyl group having 2 to 6 carbon atoms which has a substituted group" represented by $R_{39}$ to $R_{44}$ in the above-mentioned general formula (7), examples of the substituted group of the "divalent group of an aromatic hydrocarbon" or "divalent group of a fused polycyclic aromatic" include the similar ones as described for the "substituted group" in the "substituted or unsubstituted aromatic hydrocarbon group", "substituted or unsubstituted aromatic heterocyclic group", or "substituted or unsubstituted fused polycyclic aromatic group" represented by $R_{39}$ to $R_{44}$ in the above-mentioned general formula (7), and aspects similar to those of these substituted groups can be taken.

Examples of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by $R_{45}$ to $R_{56}$ in the general formula (8) include the similar ones as described for the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by $R_{39}$ to $R_{44}$ in the above-mentioned general formula (7), and aspects similar to those of the "linear or branched alkyl group having 1 to 6 carbon atoms", "cycloalkyl group having 5 to 10 carbon atoms", or "linear or branched alkenyl group having 2 to 6 carbon atoms" in the "linear or branched alkyl group having 1 to 6 carbon atoms which may have a substituted group", "cycloalkyl group having 5 to 10 carbon atoms which may have a substituted group", or "linear or branched alkenyl group having 2 to 6 carbon atoms which may have a substituted group" represented by $R_{39}$ to $R_{44}$ in the above-mentioned general formula (7) can be taken.

Examples of the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituted group" or "cycloalkyloxy group having 5 to 10 carbon atoms which may have a substituted group" represented by $R_{45}$ to $R_{56}$ in the general formula (8) include the similar ones as described for the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituted group" or "cycloalkyloxy group having 5 to 10 carbon atoms which may have a substituted group" represented by $R_{39}$ to $R_{44}$ in the above-mentioned general formula (7), and aspects similar to those of the "linear or branched alkyloxy group having 1 to 6 carbon atoms" or "cycloalkyloxy group having 5 to 10 carbon atoms" in the "linear or branched alkyloxy group having 1 to 6 carbon atoms which may have a substituted group" or "cycloalkyloxy group having 5 to 10 carbon atoms which may have a substituted group" represented by $R_{39}$ to $R_{44}$ in the above-mentioned general formula (7) can be taken.

Examples of the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by $R_{45}$ to $R_{56}$ in the general formula (8) include the similar ones as described for the "aromatic hydrocarbon group", "aromatic heterocyclic group", or "fused polycyclic aromatic group" in the "substituted or unsubstituted aromatic hydrocarbon group", "substituted or unsubstituted aromatic heterocyclic group", or "substituted or unsubstituted fused polycyclic aromatic group" represented by $R_{39}$ to $R_{44}$ in the above-mentioned general formula (7) These groups may form a ring with a single bond, or may be bonded to each other via a substituted or unsubstituted methylene group, an oxygen atom, or a sulfur atom to form a ring.

Further, these groups may each have a substituted group. Examples of such a substituted group include the similar ones as described for the "substituted group" in the "substituted aromatic hydrocarbon group", "substituted aromatic heterocyclic group", or "substituted fused polycyclic aromatic group" represented by $R_{39}$ to $R_{44}$ in the above-mentioned general formula (7), and aspects similar to those of the "substituted group" in the "substituted aromatic hydrocarbon group", "substituted aromatic heterocyclic group", or "substituted fused polycyclic aromatic group" represented by $R_{39}$ to $R_{44}$ in the above-mentioned general formula (7) can be taken.

Examples of the "aryloxy group" in the "substituted or unsubstituted aryloxy group" represented by $R_{45}$ to $R_{56}$ in the general formula (8) include the similar ones as described for the "aryloxy group" in the "substituted or unsubstituted aryloxy group" represented by $R_{39}$ to $R_{44}$ in the above-mentioned general formula (7), and aspects similar to those of the "aryloxy group" in the "substituted or unsubstituted aryloxy group" represented by $R_{39}$ to $R_{44}$ in the above-mentioned general formula (7) can be taken.

In the general formula (8), $r_7$ to $r_{18}$ may be the same as or different from each other, $r_7$ to $r_{12}$ each represent an integer of 0 to 5, and $r_{13}$ to $r_{18}$ each represent an integer of 0 to 4. In the case where any of $r_7$ to $r_{12}$ is an integer of 2 to 5 or in the case where $r_{13}$ to $r_{18}$ is an integer of 2 to 4, a plurality of $R_{45}$, a plurality of $R_{46}$, a plurality of $R_{47}$, a plurality of $R_{48}$, a plurality of $R_{49}$, a plurality of $R_{50}$, a plurality of $R_{51}$, a plurality of $R_{52}$, a plurality of $R_{53}$, a plurality of $R_{54}$, a plurality of $R_{55}$, or a plurality of $R_{56}$ bonded to the same benzene ring may be the same as or different from each other, may form a ring with a single bond, or may be bonded to each other via a substituted or unsubstituted methylene group, an oxygen atom, or a sulfur atom to form a ring.

Examples of the "divalent linking group" represented by $K_2$, $K_3$, and $K_4$ in the general formula (8) include the similar ones as described for the "divalent linking group" represented by $K_1$ in the above-mentioned general formula (7), and aspects similar to those of the "divalent linking group" represented by $K_1$ in the above-mentioned general formula (7) can be taken.

The arylamine compound according to the present invention, which is represented by the general formula (1), is a novel compound, and has an excellent ability to confine triplet exciton, excellent hole transportability, an excellent amorphous property, and high stability in a thin-film state as compared with the existing hole transport material.

The arylamine compound according to the present invention, which is represented by the general formula (1), can be used as a host material of a second hole transport layer adjacent to a light-emitting layer of an organic EL device and/or the light-emitting layer. Since the material having a high hole injection property, a high mobility of holes, a high electron blocking property, and high stability to electrons as compared with the existing material is used, there are provided effects of being capable of confining excitons generated in the light-emitting layer, improving the probability of recombination of holes and electrons, achieving high light emission efficiency, and improving the durability of the organic EL device because the drive voltage is reduced.

The arylamine compound according to the present invention, which is represented by the general formula (1), can be used also as a constituent material of a light-emitting layer of an organic EL device. The compound has an excellent hole transport property as compared with the existing material, and provides an effect of more suitably improving the light emission efficiency of the organic EL device particularly in the case where it contains a green phosphorescent light-emitting material.

The organic EL device according to the present invention is capable of achieving high efficiency and a high durability because it uses an arylamine compound, which has a high mobility of holes, excellent electron blocking performance, an excellent amorphous property, and a high electrical reduction durability as compared with the existing hole transport material.

The arylamine compound according to the present invention is useful as a light-emitting layer of an organic EL device or as a second hole transport layer adjacent to the light-emitting layer, has excellent electron blocking performance, an excellent durability for electrons, and a favorable amorphous property, and is stable in a thin-film state and excellent in heat resistance. The organic EL device according to the present invention has high light emission efficiency and high power efficiency, and has an excellent durability for electrons, which makes it possible to prolong the device lifetime.

Compound (1-1) to Compound (1-57) are shown in Fig. 1 to Fig. 5 as specific examples of favorable compounds among the arylamine compounds represented by the general formula (1). However, the present invention is not limited to these Compounds.

Note that the above-mentioned arylamine compound can be synthesized in accordance with a method known per se (see, for example, Patent Literature 8).

Compound (A-1) to Compound (A-57) are shown in fig. 6 to fig.10 as specific examples of favorable compounds among the compounds represented by the chemical formula (Host-A), which are suitably used for the organic EL device according to the present invention. However, the present invention is not limited to these Compounds.

Note that the above-mentioned compound having a nitrogen-containing heteroaromatic ring structure can be synthesized in accordance with a method known per se (see, for example, Patent Literatures 4 and 5).

Compound (B-1) to Compound (B-76) are shown in Fig. 11 to Fig. 17 as specific examples of favorable compounds among the compounds represented by the chemical formula (Host-B), which are suitably used for the organic EL device according to the present invention. However, the present invention is not limited to these Compounds.

Note that the above-mentioned compound having a carbazole structure can be synthesized in accordance with a method known per se (see, for example, Patent Literatures 4 and 5).

Compound (3-1) to Compound (3-33) are shown in Fig. 18 to Fig.20 as specific examples of favorable compounds among the compounds (metal complexes) represented by the chemical formula (3), which are suitably used for the organic EL device according to the present invention. However, the present invention is not limited to these Compounds.

Note that the above-mentioned iridium complex can be synthesized in accordance with a method known per se (see, for example, Patent Literatures 9 and 10).

Compound (4-1) to Compound (4-78) are shown in Fig. 21 to Fig. 27 as specific examples of favorable compounds among the compounds having a pyrimidine structure, which are represented by the above-mentioned general formula (4) and suitably used for the organic EL device according to the present invention. However, the present invention is not limited to these Compounds.

Note that the above-mentioned compound having a pyrimidine structure can be synthesized by a method known per se (see, for example, Patent Literatures 10 and 11).

Compound (6-1) to Compound (6-77) are shown in Fig. 28 to Fig. 34 as specific examples of favorable compounds among the compounds having a benzoazole structure, which are represented by the above-mentioned general formula (6) and suitably used for the organic EL device according to the present invention. However, the present invention is not limited to these Compounds.

Note that the above-mentioned compound having a benzoazole structure can be synthesized in accordance with a method known per se (see, for example, Patent Literatures 13 and 14).

Compound (7-1) to Compound (7-32) are shown in fig. 35 to Fig. 37 as specific examples of favorable compounds among the triphenylamine derivatives, which are represented by the above-mentioned general formula (7) and suitably used for the organic EL device according to the present invention. However, the present invention is not limited to these Compounds.

Compound (8-1) to Compound (8-16) are shown in Fig. 38 and Fig. 39 as specific examples of favorable compounds among the triphenylamine derivatives, which are represented by the above-mentioned general formula (8) and suitably used for the organic EL device according to the present invention. However, the present invention is not limited to these Compounds.

Note that the above-mentioned compound having a triarylamine structure can be synthesized in accordance with a method known per se (see, for example, Patent Literatures 1 and 2 and Patent Literature 15).

Purification for the general formulae (1) and (8), (HOST-A), and (HOST-B) was carried out by purification by column chromatography, adsorption purification with silica gel, activated carbon, activated clay, or the like, recrystallization with a solvent, a crystallization method, a sublimation purification method, or the like. Identification of the compounds was performed by NMR analysis. As physical property values, a melting point, a glass transition point (Tg), and a work function were measured. The melting point is an index of a vapor deposition property. The glass transition point (Tg) is an index of stability in a thin film state. The work function is an index of a hole transport property and a hole blocking property.

In addition, regarding the compound used for the organic EL device according to the present invention, those purified by purification by column chromatography, adsorption purification with silica gel, activated carbon, activated clay, or the like, recrystallization with a solvent, a crystallization method, or the like, and finally purified by a sublimation purification method were used.

The melting point and the glass transition point (Tg) were measured with a powder using a high sensitivity differential scanning calorimeter (DSC3100SA manufactured by Bruker AXS GmbH).

The work function was obtained by preparing a thin film of 100 nm on an ITO substrate and using an ionization potential measuring apparatus (PYS-202 manufactured by Sumitomo Heavy Industries, Ltd.).

The T1 of these compounds can be calculated by the measured phosphorescent spectrum. The phosphorescent spectrum can be measured using a commercially available spectrophotometer. As a general method of measuring the phosphorescent spectrum, there are a method of dissolving in a solvent and irradiating with excitation light at a low temperature for measurement (see, for example, Non-Patent Literature 3) or a method of forming a thin film by vapor deposition on a silicon substrate and irradiating with excitation light at a low temperature to measure the phosphorescent spectrum (see, for example, Patent Literature 16). The T1 can be calculated by reading the wavelength of the first peak on the short wavelength side of the phosphorescent spectrum or reading the wavelength at the rising position on the short wavelength side and converting it into the energy value of light in accordance with the following formula. The T1 is an index of confining triplet excitons of the phosphorescent emitter.

(Math. 1)

$$E(eV) = hc/\lambda$$

Here, E represents the value of light energy, h represents Planck's constant ($6.63\times10^{-34}$ Js), c represents the speed of light ($3.00\times10^{8}$ m/s), and $\lambda$ represents the wavelength (nm) at the rising position on the short wavelength side

of the phosphorescent spectrum. Thus, 1 eV is $1.60\times10^{-19}$ J.

Examples of the structure of the organic EL device according to the present invention include those including an anode, a hole injection layer, a first hole transport layer, a second hole transport layer, a light-emitting layer, an electron transport layer, an electron injection layer, and a cathode in the stated order on a substrate, and those including a hole blocking layer between the light-emitting layer and the electron transport layer. In the multilayer structures, several organic layers can be omitted or combined. For example, the electron injection layer and the electron transport layer may be combined. Further, two or more organic layers having the same function can be stacked. For example, two light-emitting layers may be stacked, or two electron transport layers may be stacked.

For the anode of the organic EL device according to the present invention, an electrode material having a large work function such as ITO and gold is used. As the hole injection layer of the organic EL device according to the present invention, a porphyrin compound typified by copper phthalocyanine, a starburst type triphenylamine derivative, an acceptor heterocyclic compound such as hexacyanoazatriphenylene, a coating type polymer material, or the like in addition to the arylamine compounds represented by the above-mentioned general formulae (7) and (8) can be used. These materials can be formed into a thin film by a known method such as a spin coat method and an ink jet method in addition to a vapor deposition method.

For the first hole transport layer of the organic EL device according to the present invention, the arylamine compounds represented by the above-mentioned general formulae (7) and (8) are more favorable. However, in addition thereto, a benzidine derivative such as N,N'-diphenyl-N,N'-di(m-tolyl)benzidine (hereinafter, abbreviated as TPD), N,N'-diphenyl-N,N'-di($\alpha$-naphthyl)benzidine (hereinafter, abbreviated as NPD), and N,N,N',N'-tetrabiphenylylbenzidine, 1,1-bis[4-(di-4-tolylamino)phenyl]cyclohexane (hereinafter, abbreviated as TAPC), or the like can be also used. These materials may be deposited alone. However, any of the materials may be mixed with another material and used as a single deposited layer. Further, a stacked structure of layers deposited alone, which are formed of any of the plurality of materials, layers mixed and deposited, which are formed of the plurality of materials, or at least one layer deposited alone, which is formed of any of the plurality of materials, and at least one layer mixed and deposited, which is formed of the plurality of materials, may be achieved. Further, for the hole injection/transport layer, a coating polymer material such as poly(3,4-ethylenedioxythiophene) (hereinafter, abbreviated as PEDOT)/poly(styrene sulfonate) (hereinafter, abbreviated as PSS) can be used. These materials can be formed into a thin film by a known method such as a spin coat method and an ink jet method in addition to a vapor deposition method.

Further, in the hole injection layer or the hole transport layer, those obtained by P-doping the material typically used for the respective layers with trisbromophenylaminehexachloroantimony or a radialene derivative (see, for example, Patent Literature 17), a polymer compound having, as a partial structure, the structure of a benzidine derivative such as TPD, or the like can be used.

For the second hole transport layer of the organic EL device according to the present invention, a compound having an electron blocking effect, such as a carbazole derivative such as 4,4',4"-tri(N-carbazolyl) triphenylamine (hereinafter, abbreviated as TCTA), 9,9-bis[4-(carbazol-9-yl)phenyl]fluorene, 1,3-bis(carbazol-9-yl)benzene (hereinafter, abbreviated as mCP), and 2,2-bis(4-carbazol-9-ylphenyl)adamantane (hereinafter, abbreviated as Ad-Cz), and a compound having a triphenylsilyl group and a triarylamine structure typified by 9-[4-(carbazol-9-yl)phenyl]-9-[4-(triphenylsilyl)phenyl]-9H-fluorene in addition to the arylamine compound according to the present invention, which is represented by the general formula (1) can be used. These materials may be deposited alone. However, any of the materials may be mixed with another material and used as a single deposited layer. Further, a stacked structure of layers deposited alone, layers mixed and deposited, or at least one layer deposited alone and at least one layer mixed and deposited may be achieved. These materials can be formed into a thin film by a known method such as a spin coat method and an ink jet method in addition to a vapor deposition method.

For the host of the light-emitting layer of the organic EL device according to the present invention, a host material having a hole transport property or a host material having an electron transport property can be used. As the host material having a hole transport property, a carbazole derivative such as 4,4'-di (N-carbazolyl) biphenyl (CBP), TCTA, and mCP in addition to the compound having a carbazole ring structure, which is represented by the above-mentioned general formula (HOST-B) and the arylamine compound according to the present invention, which is represented by the general formula (1), can be used. As the host material having an electron transport property, p-bis(triphenylsilyl)benzene (UGH2), 2,2',2"-(1,3,5-phenylene)-tris(1-phenyl-1H-benzimidazole) (TPBi), or the like in addition to the compound having a nitrogen-containing heteroaromatic ring structure, which is represented by the above-mentioned general formula (HOST-A), can be used. These materials may be deposited alone. However, a plurality of materials may be mixed with each other and used as a single deposited layer. Further, a stacked structure of layers deposited alone, layers mixed and deposited, or at least one layer deposited alone and at least one layer mixed and deposited may be achieved. These materials can be formed into a thin film by a known method such as a spin coat method and an ink jet method in addition to a vapor deposition method.

In the present invention, it is favorable to use two or more compounds of a first host compound having electron transportability and a second host compound having hole transportability. One or two or more types of the above-mentioned second host compound may be used. The above-mentioned first host compound and the above-mentioned second host compound may be contained in, for example, a weight ratio of 1:10 to 10:1.

As the above-mentioned first host compound of the light-emitting layer of the organic EL device according to the present invention, a compound having a nitrogen-containing heteroaromatic ring structure, which is represented by the above-mentioned general formula (HOST-A), is favorable. As the above-mentioned second host compound, a compound having a carbazole ring structure, which is represented by the above-mentioned general formula (HOST-B), or the arylamine compound according to the present invention, which is represented by the general formula (1), is favorable.

In addition to the first host compound and the second host compound, one or more types of host compounds may be further contained.

As the phosphorescent light-emitting material of the organic EL device according to the present invention, the iridium complex represented by the general formula (3) of the present invention is favorable. However, in addition thereto, an organometallic compound containing Pt, Os, Ti, Zr, Hf, Eu, Tb, Tm, Fe, Co, Ni, Ru, Rh, Pd, or a combination thereof can be used. The dopant may be a red, green, or blue dopant, and an organic EL device having high performance can be prepared.

In order to avoid concentration quenching, it is favorable to dope the phosphorescent light-emitting material with the host material by co-deposition in the range of 1 to 30 weight percent with respect to the entire light-emitting layer.

These materials can be formed into a thin film by a known method such as a spin coat method and an ink jet method in addition to a vapor deposition method.

For the hole blocking layer of the organic EL device according to the present invention, the pyrimidine compound and the benzoazole compound represented by the above-mentioned general formulae (4) and (6) are more favorable. However, in addition thereto, a compound having a hole blocking effect, such as various rare earth complexes, an oxazole derivative, a triazole derivative, and a triazine derivative, in addition to a phenanthroline derivative such as bathocuproin (hereinafter, abbreviated as BCP) and a metal complex of a quinolinol derivative such as BAlq, can be used. These materials may double the material of the electron transport layer. These materials may be deposited alone. However, any of the materials may be mixed with another material and used as a single deposited layer. Further, a stacked structure of layers deposited alone, which are formed of any of the plurality of materials, layers mixed and deposited, which are formed of the plurality of materials, or at least one layer deposited alone, which is formed of any of the plurality of materials, and at least one layer mixed and deposited, which is formed of any of the plurality of materials, may be achieved. These materials can be formed into a thin film by a known method such as a spin coat method and an ink jet method in addition to a vapor deposition method.

For the electron transport layer of the organic EL device according to the present invention, the pyrimidine compound and the benzoazole compound represented by the above-mentioned general formulae (4) and (6) are more favorable. However, in addition thereto, various metal complexes, a triazole derivative, a triazine derivative, an oxadiazole derivative, a pyridine derivative, a benzimidazole derivative, a thiadiazole derivative, an anthracene derivative, a carbodiimide derivative, a quinoxaline derivative, a pyridoindole derivative, a phenanthroline derivative, a silole derivative, or the like in addition to a metal complex of a quinolinol derivative including $Alq_3$ and BAlq can be used. These materials may be deposited alone. However, any of the materials may be mixed with another material and used as a single deposited layer. Further, a stacked structure of layers deposited alone, which are formed of any of the plurality of materials, layers mixed and deposited, which are formed of the plurality of materials, or at least one layer deposited alone, which is formed of any of the plurality of materials, and at least one layer mixed and deposited, which is formed of any of the plurality of materials, may be achieved. These materials can be formed into a thin film by a known method such as a spin coat method and an ink jet method in addition to a vapor deposition method.

For the electron injection layer of the organic EL device according to the present invention, an alkali metal salt such as lithium fluoride and cesium fluoride, an alkaline earth metal salt such as magnesium fluoride, a metal complex of a quinolinol derivative such as lithium quinolinol, a metal oxide such as aluminum oxide, or the like can be used. However, this can be omitted in the favorable selection of the electron transport layer and the cathode.

Further, in the electron injection layer or the electron transport layer, those obtained by N-doping the organic compound typically used for the respective layers with a metal such as cesium, lithium fluoride, and ytterbium can be used.

In the cathode of the organic EL device according to the present invention, an electrode material having a low work function, such as aluminum and ytterbium, an alloy having a lower work function, such as a magnesium silver alloy, a magnesium indium alloy, and an aluminum magnesium alloy, or the like is used as the electrode material.

Hereinafter, the embodiment of the present invention will be specifically described by way of Examples. However, the present invention is not limited to the following Examples unless it exceeds the gist of thereof.

(Example 1)

<Synthesis of N,N-bis(biphenyl-4-yl)-N-[4-{(2,4,6-triphenyl)phenyl}phenyl]amine (Compound 1-1)>

After adding 1,3,5-triphenylbenzene: 50.7 g and chloroform to a reaction vessel purged with nitrogen, bromine: 29.1 g was added thereto and the mixture was stirred for 16 hours at room temperature. After adding a saturated sodium sulfite aqueous solution thereto and stirring the solution, a liquid separation operation was performed thereon to collect an organic layer. The organic layer was dehydrated over magnesium sulfate and then concentrated under reduced pressure to obtain a crude product. Hexane was added to the crude product, and the mixture was dispersed and washed to obtain a white powder of 2-bromo-1,3,5-triphenylbenzene: 55.0 g (yield of 86%).

The obtained 2-bromo-1,3,5-triphenylbenzene: 5.0 g, 4-{N,N-bis(biphenyl-4-yl)amino}phenyl boronic acid: 6.9 g, tripotassium phosphate: 8.3 g, 1,4-dioxane: 90 ml, and water: 10 ml were added to a reaction vessel purged with nitrogen, and a nitrogen gas was bubbled for 30 minutes. Palladium (II) acetate: 0.087 g and tricyclohexylphosphine: 0.25 g were added thereto, and the mixture was heated and stirred at 85°C for 6 hours. Water: 50 ml was added thereto, and the precipitated solid was collected by filtration. Toluene was added to the obtained crude product, and the mixture was dissolved by heating. Silica gel was added thereto, the mixture was stirred, and then hot filtration was performed thereon. The filtrate was cooled to room temperature, and the precipitated solid was collected by filtration. Recrystallization using toluene was performed thereon to obtain a white powder of N,N-bis(biphenyl-4-yl)-N-[4-{(2,4,6-triphenyl)phenyl}phenyl]amine (Compound 1-1): 7.7 g (yield of 84%).

(Chem. 19)

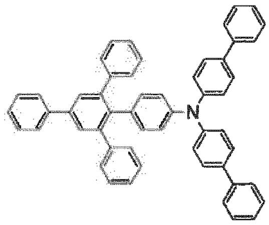

(Compound 1−1)

The structure of the obtained white powder was identified using NMR. The $^1$H-NMR measurement results are shown below.

The following 39 hydrogen signals were detected by $^1$H-NMR(THF-$d_8$).

δ (ppm) =7.92 (2H), 7.87(2H), 7.75(4H), 7.67(4H), 7.60(2H), 7.54(4H), 7.49(1H), 7.40(12H), 7.21(4H), 6.96(4H).

(Example 2)

<Synthesis of N-(biphenyl-4-yl)-N-(1,1':4',1"-terphenyl-4-yl)-N-[4-{(2,4,6-triphenyl)phenyl}phenyl]amine (Compound 1-2)>

N-(4-bromophenyl)-4-biphenylamine: 38.0 g, 4-biphenylboronic acid: 25.5 g, potassium carbonate: 32.4 g, toluene: 3000 ml, ethanol: 76 ml, and water: 113 ml were added to a reaction vessel purged with nitrogen, and a nitrogen gas was bubbled for 30 minutes. Tetrakistriphenylphosphine palladium: 2.7 g was added thereto, and the mixture was heated and stirred at 73°C for 5 hours. Water 100 ml was added thereto, and the precipitated solid was collected by filtration. o-dichlorobenzene was added to the obtained solid, and the mixture was dissolved by heating. After that, silica gel was added thereto, the mixture was stirred and then, hot filtration was performed thereon. The filtrate was concentrated under reduced pressure, and the precipitated solid was collected by filtration to obtain a yellow powder of N-(biphenyl-4-yl)-N-(1,1':4',1"-terphenyl-4-yl) amine: 20.1 g (yield of 43%) .

The obtained N-(biphenyl-4-yl)-N-(1,1':4',1"-terphenyl-4-yl)amine: 20.0 g, iodobenzene: 15.4 g, a copper powder: 0.3 g, potassium carbonate: 13.9 g, 3,5-di-tert-butylsalicylic acid: 1.2 g, sodium bisulfite: 1.5 g, and dodecylbenzene: 20 ml were added to a reaction vessel purged with nitrogen, and the mixture was heated and stirred at 180°C for 16 hours. After the mixture was cooled to 100°C, toluene was added thereto, and the precipitated solid was collected by

filtration. After subsequently performing washing with water and washing with methanol, the mixture was dissolved in o-dichlorobenzene, and adsorption purification using silica gel was performed thereon to obtain a white powder of N-(biphenyl-4-yl)-N-phenyl-N-(1,1':4',1"-terphenyl-4-yl)amine: 17.1 g (yield of 72%) .

After adding the obtained N-(biphenyl-4-yl)-N-phenyl-N-(1,1':4',1"-terphenyl-4-yl)amine: 17.0 g and dimethylformamide: 340 ml were added to a reaction vessel purged with nitrogen, N-bromosuccinimide: 7.0 g was added thereto, and the mixture was stirred at room temperature for 13 hours. Methanol was added thereto, and the precipitated solid was collected by filtration to obtain a white powder of N-(biphenyl-4-yl)-N-(4-bromophenyl)-N-(1,1':4',1"-terphenyl-4-yl)amine: 17.2 g (yield of 87%).

The obtained N-(biphenyl-4-yl)-N-(4-bromophenyl)-N-(1,1':4',1"-terphenyl-4-yl)amine: 5.0 g, a bis(pinacolato) diboron: 2.8 g, potassium acetate: 2.2 g, and 1,4-dioxane: 100 ml were added to a reaction vessel purged with nitrogen, and a nitrogen gas was bubbled for 30 minutes. A dichloromethane adduct : 0.2 g of {1,1'-bis (diphenylphosphino)ferrocene}palladium(II)dichlorid e was added thereto, and the mixture was heated and stirred at 97°C for 5 hours. After cooling the mixture to room temperature, water and toluene were added thereto and a liquid separation operation was performed thereon to collect an organic layer. The organic layer was dehydrated with anhydrous magnesium sulfate and then concentrated under reduced pressure to obtain a crude product. The crude product was dissolved in toluene and adsorption purification using silica gel was performed thereon. After filtration, the filtrate was concentrated under reduced pressure, and the precipitated solid was collected by filtration to obtain a gray powder of N-(biphenyl-4-yl)-N-{4-(4,4,5,5-tetramethyl-[1,3,2]dioxaboran-2-yl-phenyl}-N-(1,1':4',1''-terphenyl-4-yl)amine: 4.4 g (yield of 81%).

A reaction was caused to occur in conditions similar to those of Example 1 except that N-(biphenyl-4-yl)-N-{4-(4,4,5,5-tetramethyl-[1,3,2]dioxaboran-2-yl-phenyl}-N-(1,1':4',1"-terphenyl-4-yl)amine was used instead of 4-{N,N-bis(biphenyl-4-yl)amino}phenylboronic acid, thereby obtaining a white powder of N-(biphenyl-4-yl)-N-(1,1':4',1"-terphenyl-4-yl)-N-[4-{(2,4,6-triphenyl-phenyl}phenyl]amine (Compound 1-2): 3.8 g (yield of 75%).

(Chem. 20)

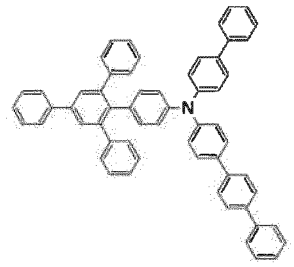

(Compound 1−2)

The structure of the obtained white powder was identified using NMR. The $^1$H-NMR measurement results are shown below.

The following 43 hydrogen signals were detected by $^1$H-NMR(THF-$d_8$).

δ (ppm) =7.92(2H), 7.87(2H), 7.85(4H), 7.83(2H), 7.74(4H), 7.68(2H), 7.57(6H), 7.49(2H), 7.40(11H), 7.22(4H), 6.97(4H).

(Example 3)

<Synthesis of N-(biphenyl-4-yl)-N-(9,9-dimethyl-9H-fluorene-2-yl)-N-[4-{(2,4,6-triphenyl)phenyl}phenyl]amine (Compound 1-3)>

N-(biphenyl-4-yl)-N-(4-bromophenyl)-N-(9,9-dimethyl-9H-fluorene-2-yl)amine: 71.9 g and tetrahydrofuran: 360 ml were added to a reaction vessel purged with nitrogen, and the mixture was cooled to - 78°C. A hexane solution: 100 ml of n-butyllithium (1.6M) was slowly added dropwise, and the mixture was stirred at the same temperature for 1 hour. Subsequently, trimethyl borate: 19 ml was slowly added dropwise and the mixture was stirred at the same temperature for 1 hour. After the temperature was raised to room temperature, the mixture was further stirred for 1 hour. Subsequently, a 1N aqueous hydrochloric acid solution was added thereto, and the mixture was stirred for 1 hour. After performing a liquid separation operation thereon to collect an organic layer, the organic layer was dehydrated with anhydrous magnesium sulfate and then concentrated under reduced pressure to obtain a crude product. The obtained crude product was purified by crystallization using a mixed solution of ethyl acetate/n-hexane to obtain a gray powder of 4-{N-(biphenyl-4-yl)-N-(9,9-dimethyl-9H-fluorene-2-yl)amino}phenylboronic acid: 44.6 g (yield of 67%).

A reaction was caused to occur in conditions similar to those in example 1 except that 4-{N-(biphenyl-4-yl)-N-(9,9-dimethyl-9H-fluorene-2-yl)amino}phenylboronic acid was used instead of 4-{N,N-bis(biphenyl-4-yl)amino}phenylboronic acid, thereby obtaining a white powder of N-(biphenyl-4-yl)-N-(9,9-dimethyl-9H-fluorene-2-yl)-N-[4-{(2,4,6-triphenyl)phenyl}phenyl]amine (Compound 1-3): 4.9 g (yield of 85%).

(Chem. 21)

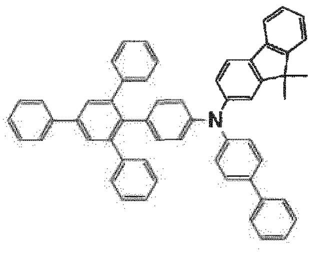

(Compound 1−3)

The structure of the obtained white powder was identified using NMR. The $^1$H-NMR measurement results are shown below.

The following 43 hydrogen signals were detected by $^1$H-NMR(THF-$d_8$).

$\delta$ (ppm)= 7.92(2H), 7.87(2H), 7.81(1H), 7.76(3H), 7.65(2H), 7.60(2H), 7.57-7.52(3H), 7.50(1H), 7.40(13H), 7.29(1H), 7.19(2H), 7.13(1H), 6.95(4H), 1.55(6H).

(Example 4)

<Synthesis of N,N-bis(9,9-dimethyl-9H-fluorene-2-yl)-N-[4-{(2,4,6-triphenyl)phenyl}phenyl]amine (Compound 1-4)>

A reaction was caused to occur in conditions similar to those in example 1 except that N,N-bis(9,9-dimethyl-9H-fluorene-2-yl)-N-{4-(4,4,5,5-tetramethyl-[1,3,2]dioxaboran-2-yl-phenyl}amine was used instead of 4-{N,N-bis(biphenyl-4-yl)amino}phenylboronic acid, thereby obtaining a white powder of N,N-bis(9,9-dimethyl-9H-fluorene-2-yl)-N-[4-{(2,4,6-triphenyl)phenyl}phenyl]amine (Compound 1-4): 11.0 g (yield of 91%).

(Chem. 22)

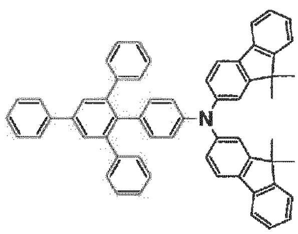

(Compound 1−4)

The structure of the obtained white powder was identified using NMR. The $^1$H-NMR measurement results are shown below.

The following 47 hydrogen signals were detected by $^1$H-NMR(THF-$d_8$).

$\delta$(ppm)=7.92(2H), 7.87(2H), 7.80(2H), 7.74(2H), 7.60(2H), 7.56(2H), 7.50(1H), 7.40(14H), 7.29(2H), 7.10(2H), 6.97(2H), 6.93(2H), 1.55(12H).

(Example 5)

<Synthesis of N-(biphenyl-4-yl)-N-(9,9-diphenyl-9H-fluorene-2-yl)-N-[4-{(2,4,6-triphenyl)phenyl}phenyl]amine (Compound 1-5)>

2-amino-9,9-biphenyl-9H-fluorene: 10.0 g, 4-bromobiphenyl: 7.3 g, sodium-tert-butoxide: 4.3 g, and toluene: 100 ml were added to a reaction vessel purged with nitrogen, and a nitrogen gas was bubbled for 40 minutes. Acetate palladium: 0.1 g, a toluene solution: 0.7g of tri-tert-butylphosphine (50wt%) was added thereto, and the mixture was heated and stirred at 80°C for 5 hours. The mixture was cooled to room temperature and then concentrated under reduced pressure. Toluene was added thereto, the mixture was dissolved by heating, and silica gel was added thereto. The obtained mixture was stirred and hot filtration was performed thereon. The filtrate was concentrated under reduced pressure, and the precipitated solid was collected by filtration to obtain a white solid of N-(biphenyl-4-yl)-N-(9,9-diphenyl-9H-fluorene-2-yl)amine: 12.0 g (yield of 82%).

The obtained N-(biphenyl-4-yl)-N-(9,9-diphenyl-9H-fluorene-2-yl)amine: 11.9 g, 1-bromo-4-iodobenzene: 8.3g , sodium-tert-butoxide: 3.5 g, and xylene: 240 ml were added to a reaction vessel purged with nitrogen, and a nitrogen gas was bubbled for 1 hour. Acetate palladium: 0.1 g and xantphos: 0.6 g were added thereto, and the mixture was heated and stirred at 120°C for 4 hours. After cooling the mixture to room temperature, water was added thereto and the mixture was filtered to collect an organic layer. The organic layer was dried with magnesium sulfate and then concentrated under reduced pressure. Then, adsorption purification using silica gel was performed to obtain a white solid of N-(biphenyl-4-yl)-N-(4-bromophenyl)-N-(9,9-diphenyl-9H-fluorene-2-yl)amine: 13.2 g (yield of 84%).

The obtained N-(biphenyl-4-yl)-N-(4-bromophenyl)-N-(9,9-diphenyl-9H-fluorene-2-yl)amine: 12.9 g and tetrahydrofuran: 100 ml were added to a reaction vessel purged with nitrogen, and cooled to - 68°C. A hexane solution: 15 ml of n-butyllithium (1.6 M) was added dropwise over 20 minutes, and then, the mixture was stirred for 40 minutes. Trimethyl borate: 3 ml was added dropwise over 15 minutes, and then, the mixture was stirred at -68°C for 1 hour. Further, the mixture was stirred at room temperature for 2 hours, 1N hydrochloric acid was added thereto, and the mixture was stirred for 2 hours. The organic layer was dried with magnesium sulfate and then concentrated under reduced pressure. The obtained product was purified by crystallization using hexane and the precipitated solid was collected by filtration to obtain a green-white solid of 4-{N-(biphenyl-4-yl)-N-(9,9-diphenyl-9H-fluorene-2-yl)amino}phenylboronic acid: 7.3 g (yield of 60%) .

A reaction was caused to occur in conditions similar to those in example 1 except that 4-{N-(biphenyl-4-yl)-N-(9,9-diphenyl-9H-fluorene-2-yl)amino}phenylboronic acid was used instead of 4-{N,N-bis(biphenyl-4-yl)amino}phenylboronic acid, thereby obtaining a yellow-white solid of N-(biphenyl-4-yl)-N-(9,9-diphenyl-9H-fluorene-2-yl)-N-[4-{(2,4,6-triphenyl)phenyl}phenyl]amine (Compound 1-5) 7.5 g (yield of 81%).

(Chem. 23)

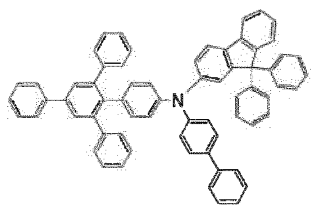

(Compound 1−5)

The structure of the obtained yellow-white solid was identified using NMR. The $^1$H-NMR measurement results are shown below.

The following 47 hydrogen signals were detected by $^1$H-NMR(THF-$d_8$).
δ(ppm)=7.93(2H), 7.90(1H), 7.88(2H), 7.83(1H), 7.75(2H), 7.64-7.61(4H), 7.55(3H), 7.53-7.48(2H), 7.43(1H), 7.36(11H), 7.31(11H), 7.17(2H), 7.14(1H), 6.94(4H).

(Example 6)

<Synthesis of N-(biphenyl-4-yl)-N-(9-phenyl-9H-carbazol-3-yl)-N-[4-{(2,4,6-triphenyl)phenyl}phenyl]amine (Compound1-6)>

N-(4-bromobiphenyl)-4-biphenylamine: 50.0g, a bis(pinacolato) diboron: 47.0 g, potassium acetate: 37.8 g, and 1,4-dioxane: 500 ml were added to a reaction vessel purged with nitrogen, and a nitrogen gas was bubbled for 50 minutes. {1,1-bis(diphenylphosphino)ferrocene}palladium(II)dichloride : 2.5 g was added thereto, and the mixture was heated and stirred at 180°C for 5 hours. After cooling the mixture to 90°C, toluene and saturated saline were added thereto and a liquid separation operation was performed thereon to collect an organic layer. The organic layer was dehydrated with magnesium sulfate and concentrated under reduced pressure, methanol was added thereto. Then, the mixture was purified by crystallization, and the precipitated solid was collected by filtration to obtain a yellow powder of N-(biphenyl-4-yl)-N-{4-(4,4,5,5-tetramethyl-1,3,2-dioxaboran-2-yl-phenyl}amine: 38.9 g (yield of 68%).

The obtained N-(biphenyl-4-yl)-N-{4-(4,4,5,5-tetramethyl-1,3,2-dioxaboran-2-yl-phenyl}amine: 21.2 g, 2-bromo-1,3,5-triphenylbenzene: 20.0 g, tripotassium phosphate: 22.1 g, 1,4-dioxane: 200 ml, and water: 60 ml were added to a reaction vessel purged with nitrogen, and a nitrogen gas was bubbled for 35 minutes. Tetrakistriphenylphosphine palladium: 0.6 g was added thereto, and the mixture was heated and stirred at 86°C for 8 hours. After cooling the mixture to room temperature, water was added thereto and the precipitated solid was collected by filtration. Toluene was added to the obtained solid, the mixture was dissolved by heating, and then, silica gel was added thereto. Then, the mixture was stirred, and hot filtration was performed thereon. The filtrate was concentrated under reduced pressure, methanol was added thereto, and the mixture was purified by crystallization. Then, the precipitated solid was collected by filtration to obtain a white solid of N-(biphenyl-4-yl)-N-{4-(2,4,6-triphenylphenyl)phenyl}amine: 28.3 g (yield of 99%).

The obtained N-(biphenyl-4-yl)-N-{4-(2,4,6-triphenylphenyl)phenyl}amine: 6.0 g, 3-bromo-9-phenyl-9H-carbazole: 3.9 g, sodium-tert-butoxide: 1.6 g, and toluene: 60 ml were added to a reaction vessel purged with nitrogen, and a nitrogen gas was bubbled for 45 minutes. Acetate palladium: 0.1 g and tri-tert-butylphosphine: 0.2 g were added thereto, and the mixture was heated and stirred at 104°C for 3 hours. After cooling the mixture to room temperature, methanol was added thereto and the precipitated solid was collected by filtration. Toluene was added to the obtained solid, and the mixture was dissolved by heating. After that, silica gel was added thereto, and the mixture was stirred and filtered. The filtrate was concentrated under reduced pressure, methanol was added to the precipitated solid, and the mixture was dispersed and washed to obtain a white solid of N-(biphenyl-4-yl)-N-(9-phenyl-9H-carbazol-3-yl)-N-[4-{(2,4,6-triphenyl)phenyl}phenyl]amine (Compound 1-6): 6.7 g (yield of 88%).

(Chem. 24)

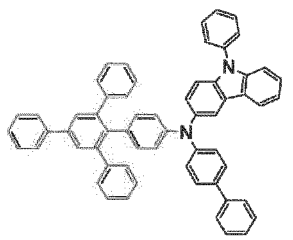

(Compound 1−6)

The structure of the obtained white solid was identified using NMR. The $^1$H-NMR measurement results are shown below.

The following 42 hydrogen signals were detected by $^1$H-NMR(THF-$d_8$).
δ(ppm)=8.23(1H), 8.04(1H), 7.93(2H), 7.88(2H), 7.84-7.79(4H), 7.75(2H), 7.64(5H), 7.55-7.50(6H), 7.41(12H), 7.33(1H), 7.20(2H), 6.98(2H), 6.92(2H).

(Example 7)

<Synthesis of N-(biphenyl-4-yl)-N-{4-(dibenzofurandibenzofuran-4-yl-phenyl}-N- [4-{(2,4,6-triphenyl-phenyl}phenyl]amine(Compound 1-7)>

1-bromo-4-iodobenzene: 10.0 g, 4-dibenzofuranylboronic acid: 7.9 g, potassium carbonate: 9.8 g, toluene: 80

ml, ethanol: 20 ml, and water: 40 ml were added to a reaction vessel purged with nitrogen, and a nitrogen gas was bubbled for 40 minutes. Tetrakistriphenylphosphine palladium: 0.8 g was added thereto, and the mixture was heated and stirred at 74°C for 6 hours. After cooling the mixture to room temperature, water was added thereto and a liquid separation operation was performed to obtain an organic layer. The organic layer was dehydrated with magnesium sulfate and then concentrated under reduced pressure, and the precipitated solid was collected by filtration to obtain a yellow solid of 4-(4-bromophenyl)dibenzofuran: 5.6 g (yield of 49%).

The obtained 4-(4-bromophenyl)dibenzofuran: 3.9 g, the N-(biphenyl-4-yl)-N-{4-(2,4,6-triphenylphenyl)phenyl} amine obtained in Example 9: 6.0 g, sodium-tert-butoxide: 1.6 g, and toluene: 60 ml were added to a reaction vessel purged with nitrogen, and a nitrogen gas was bubbled for 1 hour. Acetate palladium: 0.1 g and tri-tert-butylphosphine: 0.2 g were added thereto, and the mixture was heated and stirred at 103°C for 3 hours. After cooling the mixture to room temperature, the precipitated solid was collected by filtration. Monochlorobenzene was added thereto, the mixture was dissolved by heating, and then, silica gel was added thereto. The obtained mixture was stirred and hot filtration was performed thereon. The filtrate was concentrated under reduced pressure, methanol was added to the precipitated solid, and the mixture was dispersed and washed to obtain a white solid of N-(biphenyl-4-yl)-N-{4-(dibenzofuran-4-yl-phenyl} -N-[4-{(2,4,6-triphenyl-phenyl}phenyl]amine (Compound 1-7): 7.5 g (yield of 87%).

(Chem. 25)

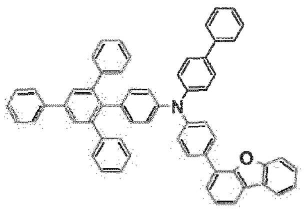

(Compound 1−7)

The structure of the obtained white solid was identified using NMR. The $^{1}H$-NMR measurement results are shown below.

The following 41 hydrogen signals were detected by $^{1}H$-NMR(THF-$d_8$).

δ(ppm)=8.24(1H), 8.15(1H), 8.03(2H), 7.95(2H), 7.90 (2H), 7.83(2H), 7.79(2H), 7.73(2H), 7.67-7.51(8H), 7.43(11H), 7.30(4H), 7.02(4H).

(Example 8)

The glass transition point of the arylamine compound represented by the general formula (1) was obtained using a high sensitivity differential scanning calorimeter (DSC3100SA manufactured by Bruker AXS GmbH).
Glass transition point

Compound of Example 1 according to present invention 110°C
Compound of Example 2 according to present invention 121°C
Compound of Example 3 according to present invention 121°C
Compound of Example 4 according to present invention 135°C
Compound of Example 5 according to present invention 145°C
Compound of Example 6 according to present invention 134°C
Compound of Example 7 according to present invention 123°C

The arylamine compound represented by the general formula (1) has a glass transition point of 100°C or higher, which indicates that the thin film state is stable.

(Example 9)

A deposition film of 100 nm was prepared on an ITO substrate by using the compound according to the present invention, and the work function thereof was measured by an ionization potential measuring apparatus (PYS-202 manufactured by Sumitomo Heavy Industries, Ltd.).

Work function

Compound of Example 1 according to present invention 5.71eV
Compound of Example 2 according to present invention 5.67eV
Compound of Example 3 according to present invention 5.63eV
Compound of Example 4 according to present invention 5.55eV
Compound of Example 5 according to present invention 5.67eV
Compound of Example 6 according to present invention 5.50eV
Compound of Example 7 according to present invention 5.70eV

It can be seen that the compound according to the present invention has favorable hole transport performance because it has a more favorable energy level than the work function that a general hole transport material such as NPD and TPD has, which is 5.4 eV.

(Example 10)

For the compound used in the present invention, a 2-methyltetrahydrofuran solution of $1.0\times10^{-5}$ mol/L was prepared. The prepared solution was put into a dedicated quartz tube, oxygen was removed by aeration with pure nitrogen, and the tube was plugged with a septa rubber so that no oxygen was mixed. After cooling to 77K, the phosphorescent spectrum was measured by irradiating with exciton light using a fluorescent phosphorescence spectrophotometer (FP-8500 manufactured by JASCO Corporation). The wavelength of the first peak of the phosphorescent spectrum on the short wavelength side was read, and the obtained wavelength value was converted to light energy to calculate T1.

| | T1 |
|---|---|
| Compound (1-1) of Example 1 according to present invention 2.54 eV | |
| Compound (1-3) of Example 3 according to present invention 2.55 eV | |
| Compound (1-5) of Example 5 according to present invention 2.55 eV | |
| HTM-2 | 2.40 eV |
| Compound 3-3 | 2.43 eV |

(Chem. 26)

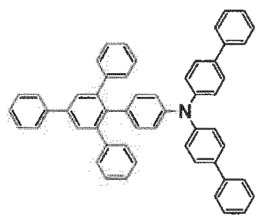

(1−1)

(Chem. 27)

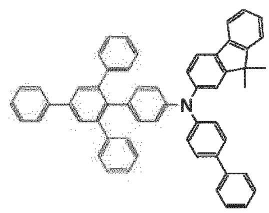

(1-3)

(Chem. 28)

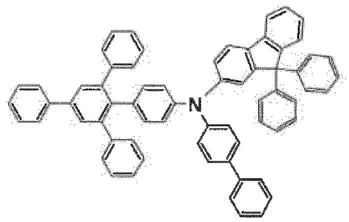

(1-5)

(Chem. 29)

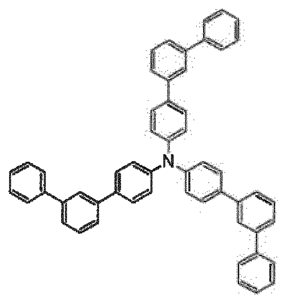

(HTM-2)

(Chem. 30)

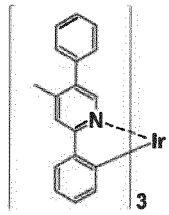

(3-3)

As described above, the compound used in the present invention has a value larger than the T1 of tri(m-terphenyl-4-yl)amine (HTM-2) that is a commonly used hole transport material. Since the two phenyl groups ortho to the phenyl group of phenylamine act as large steric hindrance groups, the compound used in the present invention achieves a higher T1 than HTM-2. Further, the compound used in the present invention has a value larger than the T1 of tris(4-methyl-2,5-diphenylpyridine)iridium(III) (Compound 3-3) that is a green phosphorescent light-emitting material and an ability to sufficiently confine triplet excitons excited in the light-emitting layer.

(Example 11)

The organic EL device was prepared by depositing a hole injection layer 3, a first hole transport layer 4, a second hole transport layer 5, a light-emitting layer 6, an electron transport layer 7, an electron injection layer 8, and a cathode (aluminum electrode) 9 in the stated order on a transparent anode 2, which has been formed on a glass substrate 1 as an ITO electrode in advance, as shown in Fig. 40.

Specifically, after performing, in isopropyl alcohol for 20 minutes, ultrasonic cleaning on the glass substrate 1 on which ITO having a film thickness of 150 nm was formed, the glass substrate 1 was dried for 10 minutes on a hot plate heated to 200°C. After that, UV ozone treatment was performed for 15 minutes, and then, the ITO-attached glass substrate was mounted in a vacuum deposition machine. The pressure in the vacuum deposition machine was reduced to 0.001 Pa or less. Subsequently, a film of a compound (Acceptor-1) having the following structural formula and the Compound (7-1) was formed, as the hole injection layer 3, to have a film thickness of 10 nm and cover the transparent anode 2 by binary deposition at a deposition rate in which the ratio of the deposition rates of (Acceptor-1) and the Compound (7-1) was 3:97. As the first hole transport layer 4, a film of the Compound (7-1) was formed on the hole injection layer 3 to have a film thickness of 70 nm. As the second hole transport layer 5, a film of the Compound (1-1) according to Example 1 was formed on the first hole transport layer 4 to have a film thickness of 10 nm. The light-emitting layer 6 was formed on the second hole transport layer 5 by simultaneously using the above-mentioned first host compound(A-19) and the above-mentioned second host compound (B-22) as hosts and doping the iridium compound (3-3) to 5 wt% as a dopant to have a film thickness of 40 nm by vacuum deposition. Here, the above-mentioned first host compound (A-19) and the above-mentioned second host compound (B-22) were used in the ratio of 1:1.

Next, a film of the Compound (4-78) having the following structural formula and the Compound ETM-2 having the following structural formula was formed on the light-emitting layer 6, as the electron transport layer 6 to have a film thickness of 30 nm by binary deposition at a deposition rate in which the ratio of the deposition rates of the Compound (4-78) and the Compound (ETM-1) was 50:50. A film of lithium fluoride was formed, as the electron injection layer 7, on the electron transport layer 6 to have a film thickness of 1 nm. Finally, aluminum was deposited to have a thickness of 100 nm to form the cathode 8. The characteristics of the prepared organic EL device were measured at room temperature in the atmosphere. The measurement results of the light-emitting characteristics when a direct current voltage was applied to the prepared organic EL device were collectively shown in Table 1.

(Chem. 31)

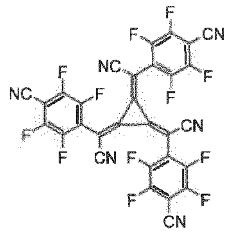

(Acceptor-1)

(Chem. 32)

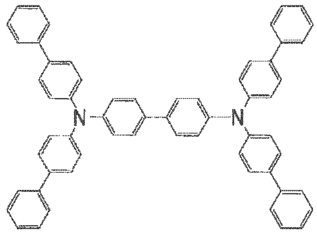

(7-3)

(Chem. 33)

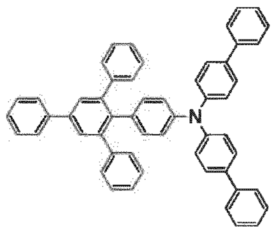

(1-1)

(Chem. 34)

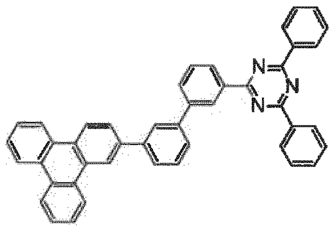

(A-19)

(Chem. 35)

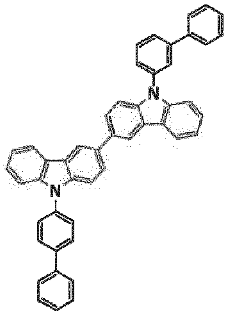

(B−22)

(Chem. 36)

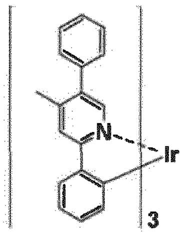

(3−3)

(Chem. 37)

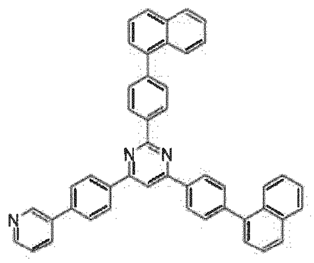

(4−78)

(Chem. 38)

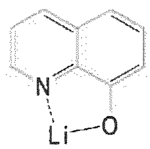

(ETM−1)

(Example 12)

An organic EL device was prepared similarly to Example 11 except that the Compound (6-1) was used as the material of the electron transport layer 6 instead of the Compound (4-78). The characteristics of the prepared organic EL device were measured at room temperature in the atmosphere. The measurement results of the light-emitting characteristics when a direct current voltage was applied to the prepared organic EL device were collectively shown in Table 1.

(Chem. 39)

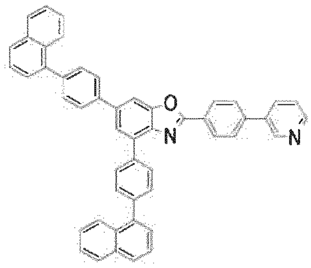

(6−1)

(Example 13)

An organic EL device was prepared similarly to Example 11 except that the Compound (1-5) according to Example 5 was used as the material of the second hole transport layer 5 instead of the Compound (1-1) according to Example 1. The characteristics of the prepared organic EL device were measured at room temperature in the atmosphere. The measurement results of the light-emitting characteristics when a direct current voltage was applied to the prepared organic EL device were collectively shown in Table 1.

(Chem. 40)

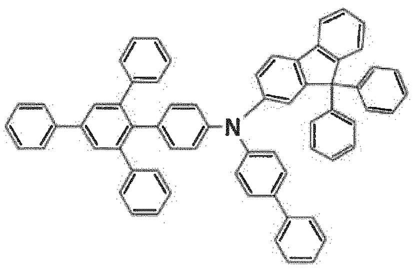

(1−5)

(Example 14)

An organic EL device was prepared similarly to Example 13 except that the Compound (6-1) was used as the material of the electron transport layer 6 instead of the Compound (4-78). The characteristics of the prepared organic EL device were measured at room temperature in the atmosphere. The measurement results of the light-emitting characteristics when a direct current voltage was applied to the prepared organic EL device were collectively shown in Table 1.

(Example 15)

An organic EL device was prepared similarly to Example 11 except that the Compound (1-1) according to Example 1 was used as the second host material instead of the Compound (B-22). Here, the first host compound (A-19) and the second host compound (1-1) were used in the ratio of 1:1. The characteristics of the prepared organic EL device were measured at room temperature in the atmosphere. The measurement results of the light-emitting characteristics when a direct current voltage was applied to the prepared organic EL device were collectively shown in Table 1.

(Example 16)

An organic EL device was prepared similarly to Example 12 except that the Compound (1-1) according to Example 1 was used as the second host material instead of the Compound (B-22). Here, the first host compound (A-19) and the second host compound (1-1) were used in the ratio of 1:1. The characteristics of the prepared organic EL device were measured at room temperature in the atmosphere. The measurement results of the light-emitting characteristics when a direct current voltage was applied to the prepared organic EL device were collectively shown in Table 1.

(Example 17)

An organic EL device was prepared similarly to Example 13 except that the Compound (1-5) according to Example 5 was used as the second host material instead of the Compound (B-22). Here, the first host compound (A-19) and the second host compound (1-5) were used in the ratio of 1:1. The characteristics of the prepared organic EL device were measured at room temperature in the atmosphere. The measurement results of the light-emitting characteristics when a direct current voltage was applied to the prepared organic EL device were collectively shown in Table 1.

(Example 18)

An organic EL device was prepared similarly to Example 14 except that the Compound (1-5) according to Example 5 was used as the second host material instead of the Compound (B-22). Here, the first host compound (A-19) and the second host compound (1-5) were used in the ratio of 1:1. The characteristics of the prepared organic EL device were measured at room temperature in the atmosphere. The measurement results of the light-emitting characteristics when a direct current voltage was applied to the prepared organic EL device were collectively shown in Table 1.

[Comparative Example 1]

For comparison, an organic EL device was prepared similarly to Example 11 except that the Compound (HTM-2) was used as the material of the second hole transport layer 5 instead of the Compound (1-1) according to Example 1. The characteristics of the prepared organic EL device were measured at room temperature in the atmosphere. The measurement results of the light-emitting characteristics when a direct current voltage was applied to the prepared organic EL device were collectively shown in Table 1.

(Chem. 41)

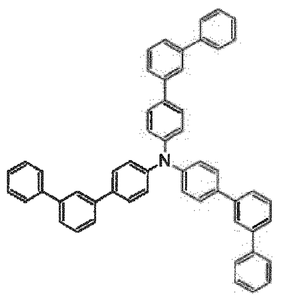

(HTM-2)

[Comparative Example 2]

For comparison, an organic EL device was prepared similarly to Example 12 except that the Compound (HTM-2) was used as the material of the second hole transport layer 5 instead of the Compound (1-1) according to Example 1. The characteristics of the prepared organic EL device were measured at room temperature in the atmosphere. The

measurement results of the light-emitting characteristics when a direct current voltage was applied to the prepared organic EL device were collectively shown in Table 1.

[Comparative Example 3]

For comparison, an organic EL device was prepared similarly to Example 11 except that the Compound (B-22) having the following structural formula was used as the material of the second hole transport layer 5 instead of the Compound (1-1) according to Example 1. The characteristics of the prepared organic EL device were measured at room temperature in the atmosphere. The measurement results of the light-emitting characteristics when a direct current voltage was applied to the prepared organic EL device were collectively shown in Table 1.

(Chem. 42)

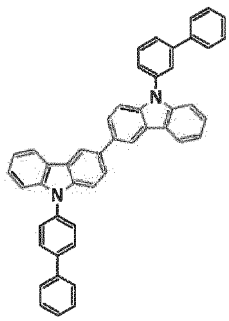

(B−22)

[Comparative Example 4]

For comparison, an organic EL device was prepared similarly to Example 12 except that the Compound (B-22) was used as the material of the second hole transport layer 5 instead of the Compound (1-1) according to Example 1. The characteristics of the prepared organic EL device were measured at room temperature in the atmosphere. The measurement results of the light-emitting characteristics when a direct current voltage was applied to the prepared organic EL device were collectively shown in Table 1.

[Comparative Example 5]

For comparison, an organic EL device was prepared similarly to Example 15 except that the Compound (HTM-2) was used as the material of the second hole transport layer 5 instead of the Compound (1-1) according to Example 1 and the Compound (HTM-2) was used as the second host material instead of the Compound (1-1) according to Example 1. Here, the first host compound (A-19) and the second host compound (HTM-2) were used in the ratio of 1:1. The characteristics of the prepared organic EL device were measured at room temperature in the atmosphere. The measurement results of the light-emitting characteristics when a direct current voltage was applied to the prepared organic EL device were collectively shown in Table 1.

For comparison, an organic EL device was prepared similarly to Example 16 except that the Compound (HTM-2) was used as the material of the second hole transport layer 5 instead of the Compound (1-1) according to Example 1 and the Compound (HTM-2) was used as the second host material instead of the Compound (1-1) according to Example 1. Here, the first host compound (A-19) and the second host compound (HTM-2) were used in the ratio of 1:1. The characteristics of the prepared organic EL device were measured at room temperature in the atmosphere. The measurement results of the light-emitting characteristics when a direct current voltage was applied to the prepared organic EL device were collectively shown in Table 1.

The device lifetime was measured using each of the organic EL devices prepared in Examples 11 to 18 and Comparative Examples 1 to 6, and the results were collectively shown in Table 1. The device lifetime was measured as the time until the light emission luminance attenuated to 9500 cd/$m^2$ (corresponding to 95% in the case where the initial luminance was 100%: 95% attenuation) when constant current driving was performed with the light emission luminance (initial luminance) at the start of light emission set to 10000 cd/$m^2$.

(Table 1)

| | Second hole transport layer | First host/ Second host | First host: Second host (wt: wt) | Electron transport layer | Voltage [V] (@10mA/cm2) | Luminance [cd/m2] (@10mA/cm2) | Light emission efficiency [cd/A] (@10mA/cm2) | Power efficiency [lm/W] (@10mA/cm2) | Element lifetime (hour) 95% attenuated |
|---|---|---|---|---|---|---|---|---|---|
| Example 11 | Compound 1-1 | A-19/B-22 | 1:1 | Compound 4-78/ ETM-1 | 4.33 | 7722 | 77.24 | 56.05 | 475 |
| Example 12 | Compound 1-1 | A-19/B-22 | 1:1 | Compound 6-1/ ETM-1 | 4.30 | 7708 | 77.10 | 56.34 | 480 |
| Example 13 | Compound 1-5 | A-19/B-22 | 1:1 | Compound 4-78/ ETM-1 | 4.25 | 7623 | 76.26 | 56.38 | 570 |
| Example 14 | Compound 1-5 | A-19/B-22 | 1:1 | Compound 6-1 / ETM-1 | 4.22 | 7565 | 75.70 | 56.36 | 522 |
| Example 15 | Compound 1-1 | A-19/ Compound 1-1 | 1:1 | Compound 4-78/ ETM-1 | 4.38 | 7500 | 75.06 | 53.84 | 433 |
| Example 16 | Compound 1-1 | A-19/ Compound 1-1 | 1:1 | Compound 6-1/ ETM-1 | 4.35 | 7435 | 74.38 | 53.72 | 420 |
| Example 17 | Compound 1-5 | A-19/ Compound 1-5 | 1 :1 | Compound 4-78/ ETM-1 | 4.30 | 7376 | 73.77 | 53.90 | 474 |
| Example 18 | Compound 1-5 | A-19/ Compound 1-5 | 1:1 | Compound 6-1/ ETM-1 | 4.29 | 7343 | 73.44 | 53.78 | 465 |
| Comparative Example 1 | HTM-2 | A-19/B-22 | 1:1 | Compound 4-78/ ETM-1 | 4.35 | 7282 | 72,91 | 52.66 | 400 |
| Comparative Example 2 | HTM-2 | A-19/B-22 | 1:1 | Compound 6-1/ ETM-1 | 4.30 | 7252 | 72.52 | 52.99 | 374 |

(continued)

| | Second hole transport layer | First host/ Second host | First host: Second host (wt: wt) | Electron transport layer | Voltage [V] (@10mA/cm2) | Luminance [cd/m2] (@10mA/cm2) | Light emission efficiency [cd/A] (@10mA/cm2) | Power efficiency [lm/W] (@10mA/cm2) | Element lifetime (hour) 95% attenuated |
|---|---|---|---|---|---|---|---|---|---|
| Comparative Example 3 | B-22 | A-19/B-22 | 1:1 | Compound 4-78/ ETM-1 | 4.35 | 7201 | 72.06 | 52.00 | 384 |
| Comparative Example 4 | B-22 | A-19/B-22 | 1:1 | Compound 6-1/ ETM-1 | 4.31 | 7298 | 73.03 | 53.27 | 341 |
| Comparative Example 5 | HTM-2 | A-19/HTM-2 | 1:1 | Compound 4-78/ ETM-1 | 4.38 | 6631 | 66.33 | 47.58 | 333 |
| Comparative Example 6 | HTM-2 | A-19/HTM-2 | 1:1 | Compound 6-1/ ETM-1 | 4.36 | 6363 | 63.74 | 45.93 | 309 |

As shown in Table 1, in comparison between Examples 11 to 14 in which the arylamine compound according to the present invention was used as the second hole transport material and Comparative Examples 1 to 4 in which the above-mentioned Compound (HTM-2) and the above-mentioned Compound (B-22) were used as the second hole transport materials, the light emission efficiency when a current having a current density of 10 $mA/cm^2$ was caused to flow was high, i.e., 75.70 to 77.24 cd/A in the organic EL devices according to Examples 11 to 14 as compared with 72.06 to 73.03 cd/A of the organic EL devices according to Comparative Examples 1 to 4. Further, also the power efficiency of the organic EL devices according to Examples 11 to 14 was high, i.e., 56.05 to 56.38 lm/W as compared with 52.00 to 53.27 lm/W of the organic EL devices according to Comparative Examples 1 to 4. Meanwhile, it can be seen that the device lifetime (95% attenuation) was largely extended to 475 to 570 hours in the organic EL devices according to Examples 11 to 14 as compared with 341 to 400 hours of the organic EL devices according to Comparative Examples 1 to 4.

As shown in Table 1, in comparison between Examples 15 to 18 in which the arylamine compound according to the present invention was used as the second hole transport material and the second host material and Comparative Examples 3 to 6 in which the above-mentioned Compound (HTM-2) and the above-mentioned Compound (B-22) were used as the second hole transport material and the second host material, the light emission efficiency when a current having a current density of 10 $mA/cm^2$ was caused to flow was high, i.e., 73.44 to 75.06 cd/A in the organic EL devices according to Examples 15 to 18, as compared with 63.74 to 73.03 cd/A of the organic EL devices according to Comparative Examples 3 to 6. Further, also the power efficiency of the organic EL devices according to Examples 15 to 18 was high, i.e., 53.72 to 53.90 lm/W, as compared with 45.93 to 53.27 of the organic EL devices according to Comparative Examples 3 to 6. Meanwhile, it can be seen that the device lifetime (95% attenuation) was largely extended to 420 to 474 hours in the organic EL devices according to Examples 15 to 18 as compared with 309 to 384 hours of the organic EL devices according to Comparative Examples 3 to 6.

As is clear from the results in Table 1, it has been found that an organic EL device that includes a light-emitting layer using both a first host material having high electron transportability and a second host material having hole transportability and uses an arylamine compound according to the present invention as the material of a second hole transport layer is capable of improving power efficiency and prolonging the lifetime even as compared with the organic EL device using the above-mentioned Compound (HTM-2) that is an arylamine compound similarly. This is because the arylamine compound according to the present invention has a value larger than the T1 of the Compound (HTM-2). The organic EL device using the arylamine compound according to the present invention is capable of sufficiently confining triplet excitons excited in a light-emitting layer as compared with the organic EL device using the Compound (HTM-2), and realizes an organic EL device having improved efficiency characteristics and remarkably improved lifetime characteristics. Further, it has been found that the power efficiency can be improved and the lifetime can be prolonged even as compared with the organic EL device using, as the material of the second hole transport layer, the above-mentioned Compound (B-22) that is a carbazole derivative. By combining an arylamine compound having a specific structure, holes are efficiently supplied to a light-emitting layer and the excess in electrons in the light-emitting layer has been improved. As a result, the carrier balance in the light-emitting layer is further refined, and an organic EL device having improved efficiency characteristics and remarkably improved lifetime characteristics is realized.

Further, it has been found that the organic EL device using the arylamine compound according to the present invention as the second host material is capable of improving power efficiency and prolonging the lifetime even as compared with the organic EL device using the Compound (HTM-2) that is an arylamine compound similarly. The arylamine compound according to the present invention has a value larger than the T1 of the above-mentioned compound (Compound 3-3) that is a green phosphorescent light-emitting material, and excited triplet excitons are sufficiently confined even in the case where the arylamine compound according to the present invention is used as the second host material. Meanwhile, the Compound (HTM-2) has a lower T1, the triplet excitons are not sufficiently confined, and the light emission efficiency and the device lifetime were significantly reduced due to deactivation of the excited triplet excitons. Further, it has been found that the power efficiency can be improved and the lifetime can be prolonged even as compared with the Compound (B-22) that is a carbazole derivative. By combining an arylamine compound having an excellent electric durability and excellent hole transportability, holes are efficiently supplied to a light-emitting layer and the excess in electrons in the light-emitting layer has been improved. As a result, the carrier balance in the light-emitting layer is further refined, and an organic EL device having improved efficiency characteristics and remarkably improved lifetime characteristics is realized.

Industrial Applicability

The organic EL device according to the present invention has improved light emission efficiency and significantly improved durability, and, for example, it has become possible to expand to home appliances and lighting applications.

1 glass substrate
2 transparent anode
3 hole injection layer
4 first hole transport layer
5 second hole transport layer
6 light-emitting layer
7 electron transport layer
8 electron injection layer
9 cathode

## Claims

1. An organic electroluminescence device, comprising, between an anode and a cathode, at least a first hole transport layer, a second hole transport layer, a green light-emitting layer, and an electron transport layer in the stated order from a side of the anode, the organic EL device being **characterized in that**, the second hole transport layer, or at least one of stacked films disposed between the first hole transport layer and the electron transport layer contains an arylamine compound represented by the following general formula (1) .

(Chem. 1)

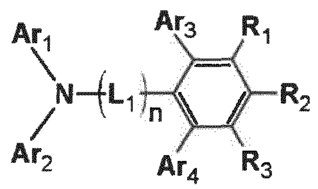

(1)

(In the formula, $Ar_1$, $Ar_2$, $Ar_3$, and $Ar_4$ may be the same as or different from each other, and each represent a substituted or unsubstituted aromatic hydrocarbon group, a substituted or unsubstituted aromatic heterocyclic group, or a substituted or unsubstituted fused polycyclic aromatic group. $L_1$ represents a divalent group of a substituted or unsubstituted aromatic hydrocarbon, a divalent group of a substituted or unsubstituted aromatic heterocyclic, or a divalent group of a substituted or unsubstituted fused polycyclic aromatic. $R_1$, $R_2$, and $R_3$ each represent a hydrogen atom, a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. n represents an integer of 1 to 3.)

2. An organic electroluminescence device, comprising, between an anode and a cathode, at least a first hole transport layer, a second hole transport layer, a green light-emitting layer, and an electron transport layer in the stated order from a side of the anode, the organic EL device being **characterized in that** the second hole transport layer, or at least one of stacked films disposed between the first hole transport layer and the electron transport layer contains an arylamine compound represented by the following general formula (2) .

(Chem. 2)

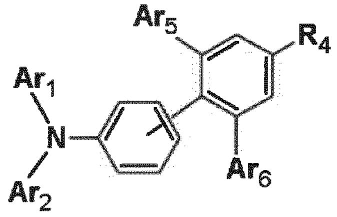

(2)

(In the formula, $Ar_1$ and $Ar_2$ may be the same as or different from each other, and each represent a substituted or unsubstituted aromatic hydrocarbon group, a substituted or unsubstituted aromatic heterocyclic group, or a substituted or unsubstituted fused polycyclic aromatic group. $Ar_5$ and $Ar_6$ may be the same as or different from each other, and each represent a phenyl group, a biphenylyl group, a naphthyl group, a phenanthrenyl group, or a fluorenyl group. $R_4$ represents a hydrogen atom, a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group.)

**3.** The organic electroluminescence device according to claim 1 or claim 2, **characterized in that** the green light-emitting layer contains a host and a phosphorescent dopant, and the host contains at least one first host compound represented by the following chemical formula Host-A and at least one second host compound represented by the following chemical formula Host-B.

(Chem. 3)

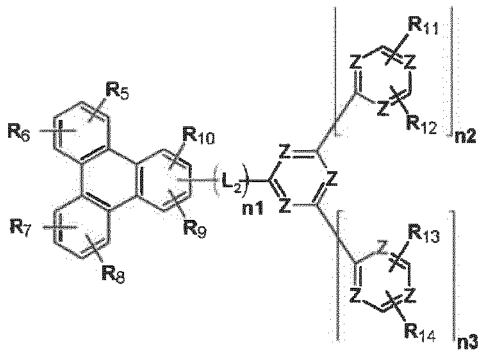

(Host−A)

(In the Host-A, Zs each independently represent N or CRa, and at least one of Zs represents N. $R_5$ to $R_{14}$ and Ra each independently represent a hydrogen atom, a deuterium atom, a fluorine atom, a chlorine atom, a cyano group, a nitro group, a substituted or unsubstituted alkyl group having 1 to 15 carbon atoms, or a substituted or unsubstituted aryl group having 6 to 12 ring carbon atoms. The total number of 6-membered rings substituted with triphenylene groups in the Host-A is six or less. $L_2$ represents a substituted or unsubstituted phenylene group, a substituted or unsubstituted biphenylene group, or a substituted or unsubstituted terphenylene group. n1 to n3 each independently represent 0 or 1, and n1+n2+n3≥1.)

(Chem. 4)

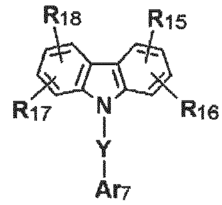

(Host−B)

(In the Host-B, Y represents a single bond, a substituted or unsubstituted arylene group having 6 to 30 ring carbon atoms, or a substituted or unsubstituted heteroarylene group having 5 to 30 ring carbon atoms. $Ar_7$ represents a substituted or unsubstituted aryl group having 6 to 30 ring carbon atoms or a substituted or unsubstituted heteroaryl group having 5 to 30 ring carbon atoms. $R_{15}$ to $R_{18}$ each independently represent a hydrogen atom, a deuterium atom, a fluorine atom, a chlorine atom, a cyano group, a nitro group, an alkyl group having 1 to 15 carbon atoms, a substituted or unsubstituted aryl group having 6 to 50 ring carbon atoms, or a substituted or unsubstituted heteroaryl group having 4 to 50 ring carbon atoms. At least one of $R_{15}$ to $R_{18}$ and $Ar_7$ includes a substituted or unsubstituted triphenylene group or a substituted or unsubstituted carbazole group.)

4. The organic electroluminescence device according to any one of claims 1 to 3, **characterized in that** the green light-emitting layer contains a host and a phosphorescent dopant, and the phosphorescent dopant is a metal complex containing iridium.

5. The organic electroluminescence device according to any one of claims 1 to 3, **characterized in that** the green light-emitting layer contains a host and a phosphorescent dopant, and the phosphorescent dopant is a metal complex represented by the following general formula (3).

(Chem. 5)

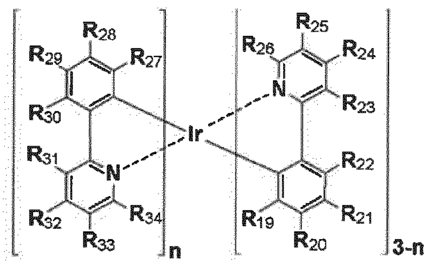

(3)

(In the formula, $R_{19}$ to $R_{34}$ may be the same as or different from each other, and each represent a hydrogen atom, a deuterium atom, a fluorine atom, a chlorine atom, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a trimethylsilyl group, a substituted or unsubstituted aromatic hydrocarbon group, a substituted or unsubstituted aromatic heterocyclic group, a substituted or unsubstituted fused polycyclic aromatic group, a substituted or unsubstituted aryloxy group, or a disubstituted amino group substituted with a group selected from an aromatic hydrocarbon group, an aromatic heterocyclic group, or a fused polycyclic aromatic group. n represents an integer of 1 to 3.)

6. The organic electroluminescence device according to any one of claims 1 to 5, **characterized in that** the electron transport layer contains a compound having a pyrimidine structure, the compound being represented by the following general formula (4).

(Chem. 6)

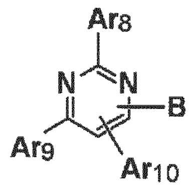

(4)

(In the formula, $Ar_8$ represents a substituted or unsubstituted aromatic hydrocarbon group or a substituted or unsubstituted fused polycyclic aromatic group. $Ar_9$ and $Ar_{10}$ may be the same as or different from each other, and each represent a hydrogen atom, a substituted or unsubstituted aromatic hydrocarbon group, or a substituted or unsubstituted fused polycyclic aromatic group. B represents a monovalent group represented by the following structural formula (5). Here, there is no case that both $Ar_9$ and $Ar_{10}$ are hydrogen atoms.)

(Chem. 7)

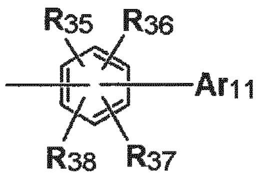

(5)

(In the formula, $Ar_{11}$ represents a substituted or unsubstituted aromatic heterocyclic group, and $R_{35}$ to $R_{38}$ may be the same as or different from each other, and each represent a hydrogen atom, a deuterium atom, a fluorine atom, a chlorine atom, a cyano group, a trifluoromethyl group, a linear or branched alkyl group having 1 to 6 carbon atoms, a substituted or unsubstituted aromatic hydrocarbon group, a substituted or unsubstituted aromatic heterocyclic group, or a substituted or unsubstituted fused polycyclic aromatic group.)

7. The organic electroluminescence device according to any one of claims 1 to 5, **characterized in that** the electron transport layer contains a compound having a benzoazole structure, the compound being represented by the following general formula (6).

(Chem. 8)

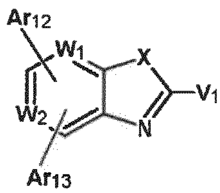

(6)

(In the formula, $Ar_{12}$ and $Ar_{13}$ may be the same as or different from each other, and each represent a hydrogen atom, a deuterium atom, a substituted or unsubstituted aromatic hydrocarbon group, a substituted or unsubstituted

fused polycyclic aromatic group, or a substituted or unsubstituted aromatic heterocyclic group. $V_1$ represents a substituted or unsubstituted aromatic hydrocarbon group, a substituted or unsubstituted fused polycyclic aromatic group, a substituted or unsubstituted aromatic heterocyclic group, a linear or branched alkyl group having 1 to 6 carbon atoms which may have a substituted group, a cycloalkyl group having 5 to 10 carbon atoms which may have a substituted group, or a linear or branched alkenyl group having 2 to 6 carbon atoms which may have a substituted group. X represents an oxygen atom or a sulfur atom. $W_1$ and $W_2$ may be the same as or different from each other, and each represent a carbon atom or a nitrogen atom.)

**8.** The organic electroluminescence device according to any one of claims 1 to 7, **characterized in that** the first hole transport layer contains a triphenylamine derivative represented by the following general formula (7) or the following general formula (8) .

(Chem. 9)

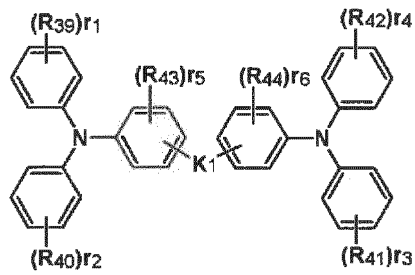

(7)

(In the formula, $R_{39}$ to $R_{44}$ each represent a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. $r_1$ to $r_6$ may be the same as or different from each other, $r_1$ to $r_4$ each represent an integer of 0 to 5, and $r_5$ and $r_6$ each represent an integer of 0 to 4. In a case where any of $r_1$ to $r_6$ is an integer of two or more, a plurality of $R_{39}$, a plurality of $R_{40}$, a plurality of $R_{41}$, a plurality of $R_{42}$, a plurality of $R_{43}$, a plurality of $R_{44}$ bonded to the same benzene ring may be the same as or different from each other. Further, a benzene ring and a substituted group substituted with a benzene ring, a plurality of substituted groups substituted with the same benzene ring, or benzene rings adjacent to each other via a nitrogen atom may form a ring with a single bond, or may be bonded to each other via a substituted or unsubstituted methylene group, an oxygen atom, or a sulfur atom to form a ring. $K_1$ represents a divalent group represented by any of the following structural formulae (HTM-A) to (HTM-F) or a single bond.)

(Chem. 10)

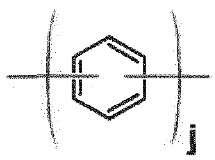

(HTM−A)

(In the formula, j represents an integer of 1 to 3.)

(Chem. 11)

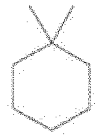

(HTM−B)

(Chem. 12)

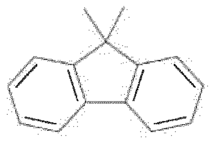

(HTM−C)

(Chem. 13)

$$-CH_2-$$

(HTM−D)

(Chem. 14)

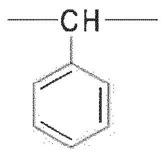

(HTM−E)

(Chem. 15)

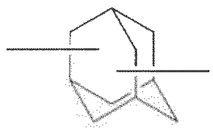

(HTM−F)

(Chem. 16)

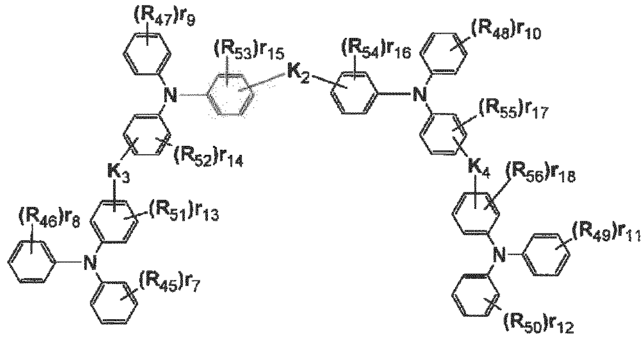

(8)

(In the formula, $R_{45}$ to $R_{56}$ each represent a deuterium atom, a fluorine atom, a chlorine atom, a cyano group, a nitro group, a linear or branched alkyl group having 1 to 6 carbon atoms which may have a substituted group, a cycloalkyl group having 5 to 10 carbon atoms which may have a substituted group, a linear or branched alkenyl group having 2 to 6 carbon atoms which may have a substituted group, a linear or branched alkyloxy group having 1 to 6 carbon atoms which may have a substituted group, a cycloalkyloxy group having 5 to 10 carbon atoms which may have a substituted group, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group. $r_7$ to $r_{18}$ may be the same as or different from each other, $r_7$ to $r_{12}$ each represent an integer of 0 to 5, and $r_{13}$ to $r_{18}$ each represent an integer of 0 to 4. In a case where any of $r_7$ to $r_{18}$ is an integer of two or more, a plurality of $R_{45}$, a plurality of $R_{46}$, a plurality of $R_{47}$, a plurality of $R_{48}$, a plurality of $R_{49}$, a plurality of $R_{50}$, a plurality of $R_{51}$, a plurality of $R_{52}$, a plurality of $R_{53}$, a plurality of $R_{54}$, a plurality of $R_{55}$, or a plurality of $R_{56}$ bonded to the same benzene ring may be the same as or different from each other. Further, a benzene ring and a substituted group substituted with a benzene ring, a plurality of substituted groups substituted with the same benzene ring, or benzene rings adjacent to each other via a nitrogen atom may form a ring with a single bond, or may be bonded to each other via a substituted or unsubstituted methylene group, an oxygen atom, or a sulfur atom to form a ring. $K_2$ to $K_4$ may be the same as or different from each other, and each represent a divalent group represented by any of the structural formulae (HTM-A) to (HTM-F) in the general formula (7), or a single bond.)

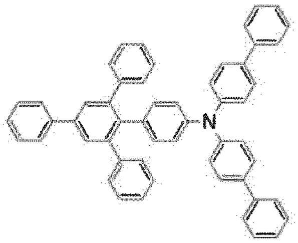

(1—1)

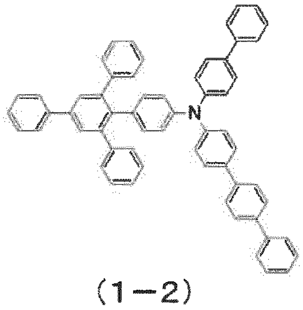

(1—2)

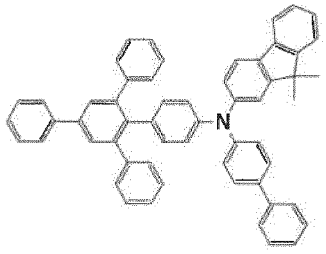

(1—3)

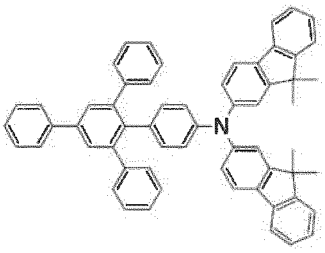

(1—4)

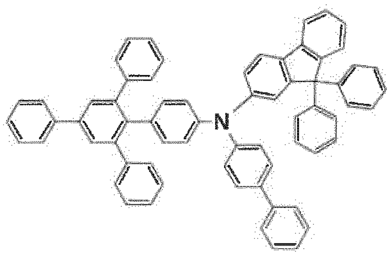

(1—5)

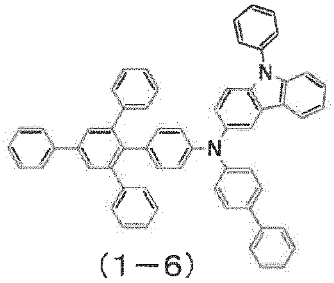

(1—6)

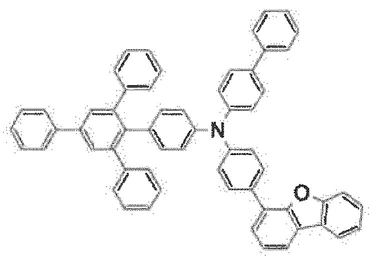

(1—7)

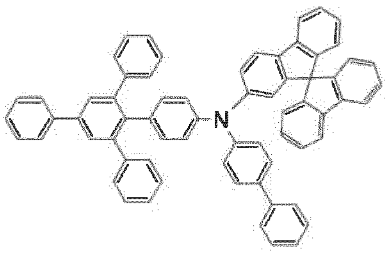

(1—8)

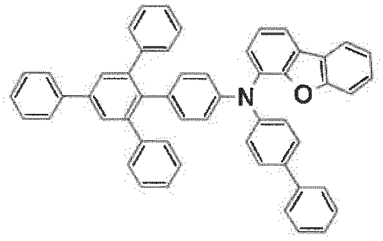

(1—9)

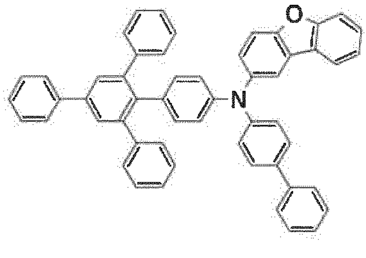

(1—10)

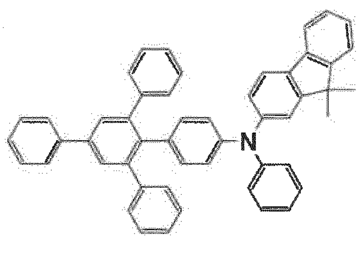

(1—11)

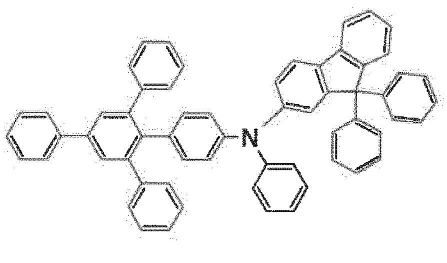

(1—12)

FIG.1

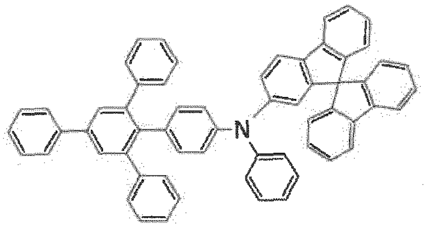

(1−13)

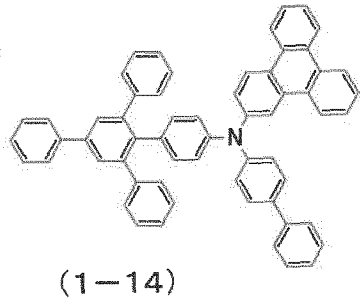

(1−14)

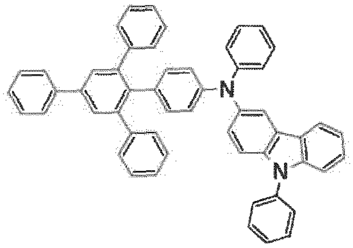

(1−15)

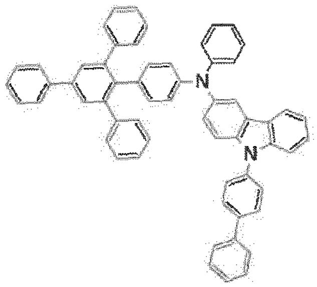

(1−16)

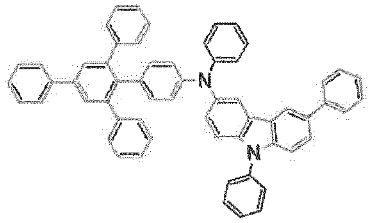

(1−17)

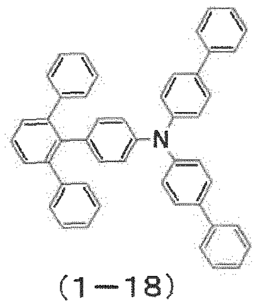

(1−18)

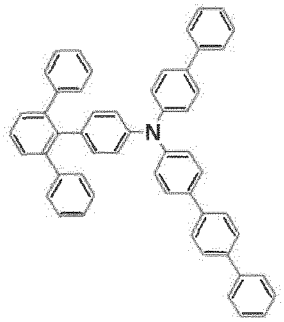

(1−19)

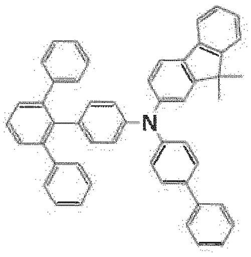

(1−20)

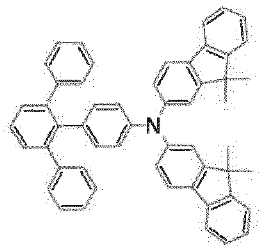

(1−21)

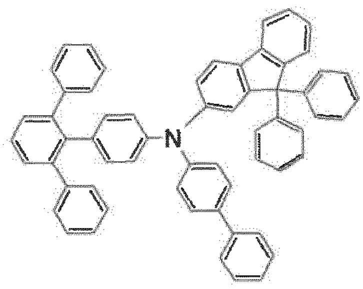

(1−22)

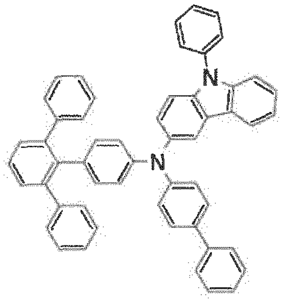

(1−23)

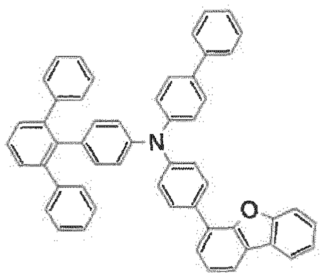

(1−24)

FIG.2

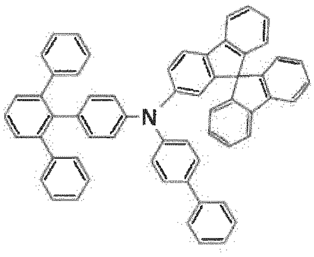
(1−25)

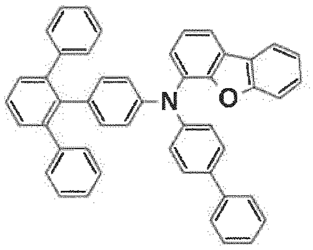
(1−26)

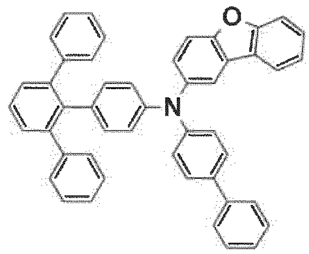
(1−27)

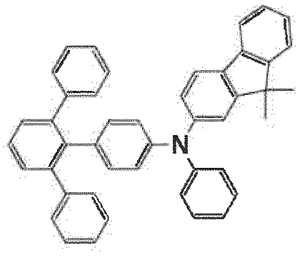
(1−28)

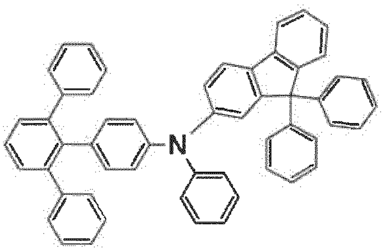
(1−29)

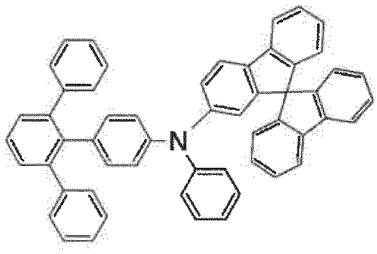
(1−30)

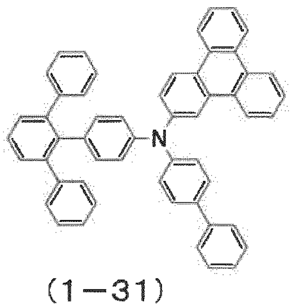
(1−31)

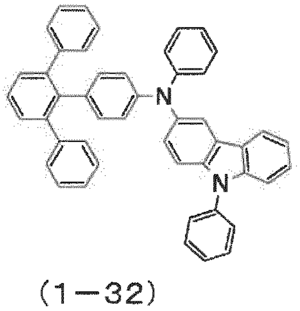
(1−32)

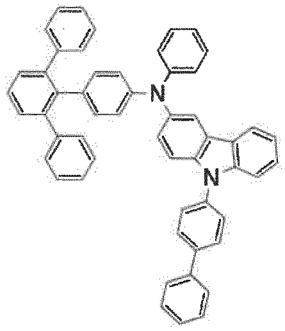
(1−33)

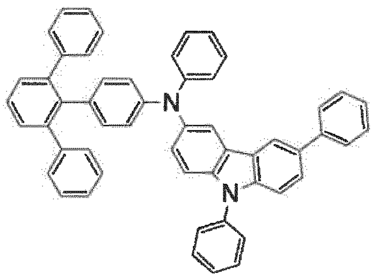
(1−34)

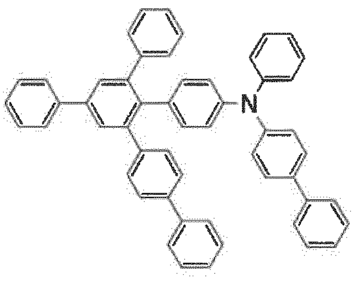
(1−35)

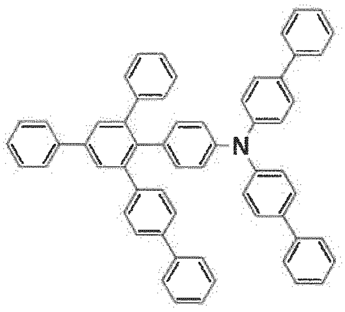
(1−36)

FIG.3

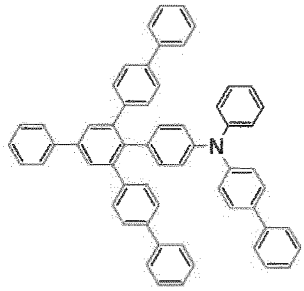
(1−37)

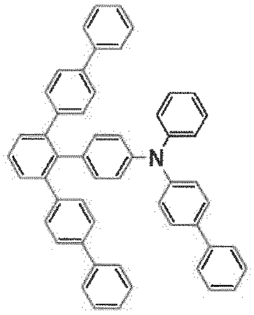
(1−38)

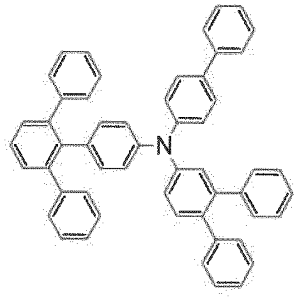
(1−39)

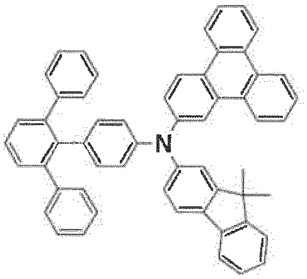
(1−40)

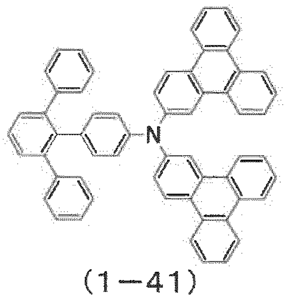
(1−41)

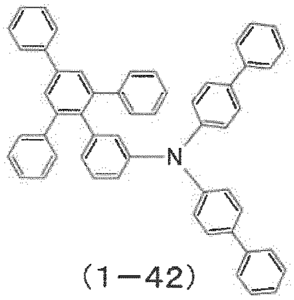
(1−42)

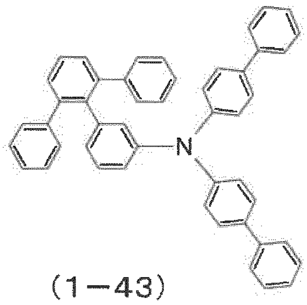
(1−43)

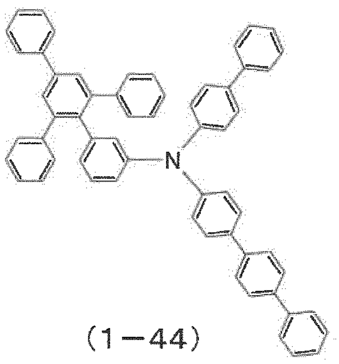
(1−44)

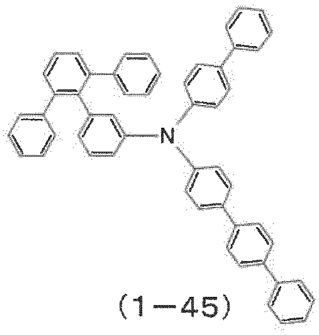
(1−45)

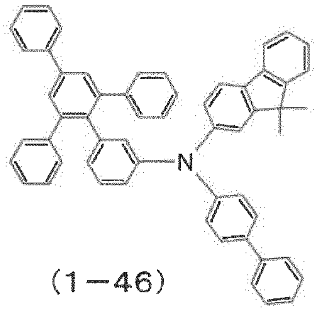
(1−46)

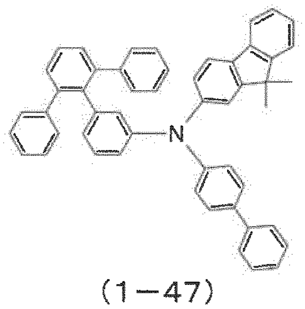
(1−47)

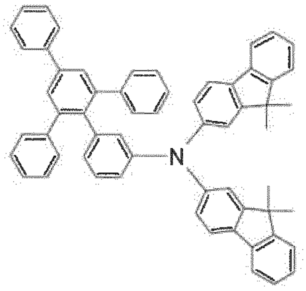
(1−48)

FIG.4

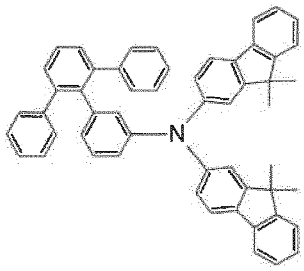

(1−49)

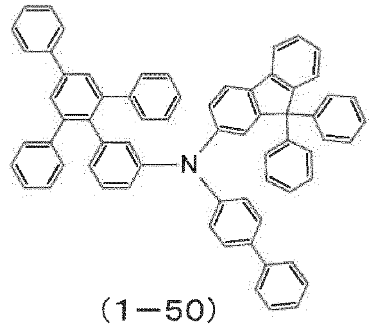

(1−50)

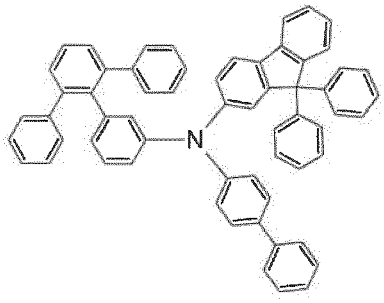

(1−51)

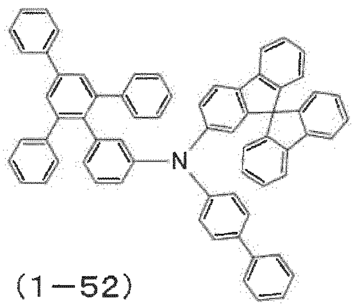

(1−52)

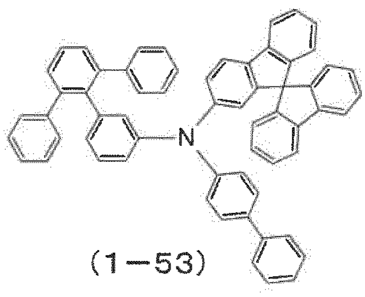

(1−53)

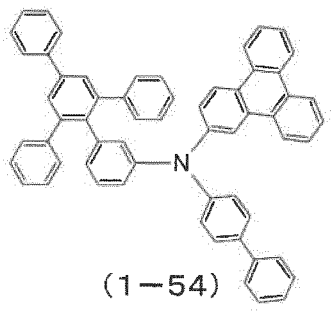

(1−54)

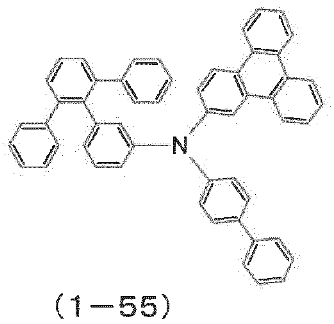

(1−55)

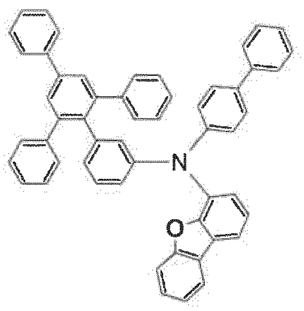

(1−56)

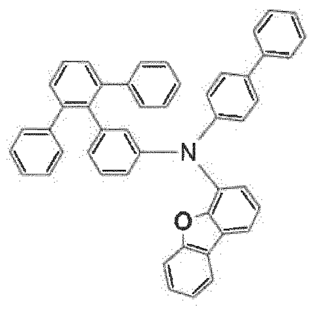

(1−57)

FIG.5

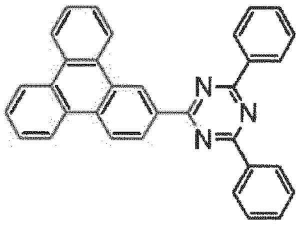

(A-1)

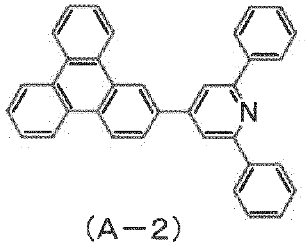

(A-2)

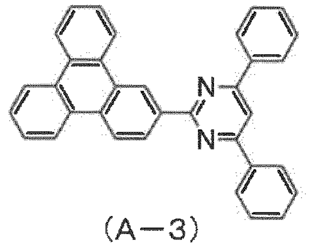

(A-3)

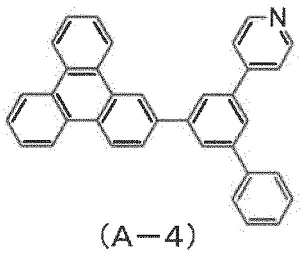

(A-4)

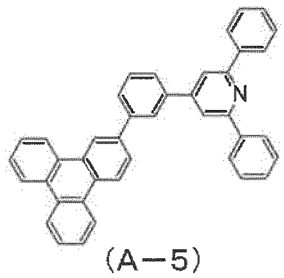

(A-5)

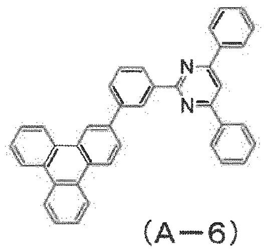

(A-6)

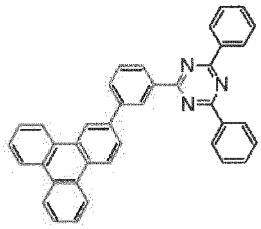

(A-7)

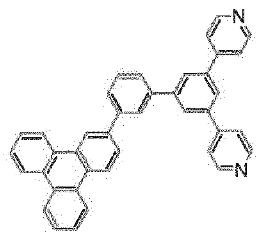

(A-8)

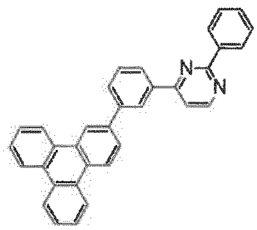

(A-9)

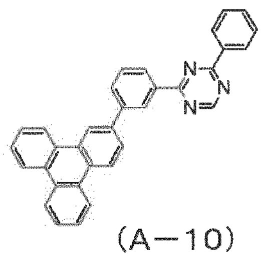

(A-10)

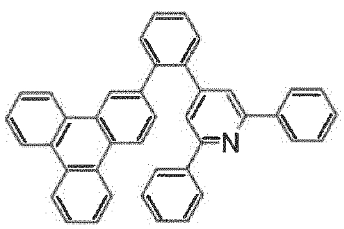

(A-11)

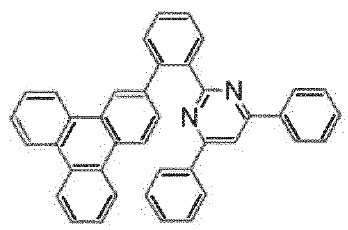

(A-12)

FIG.6

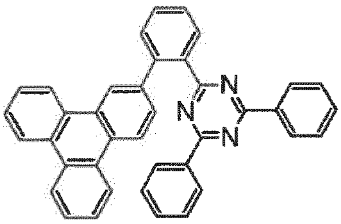
(A−13)

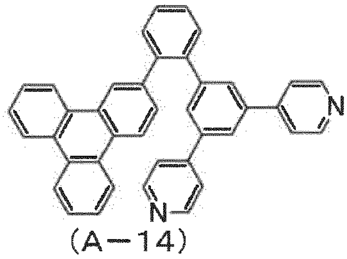
(A−14)

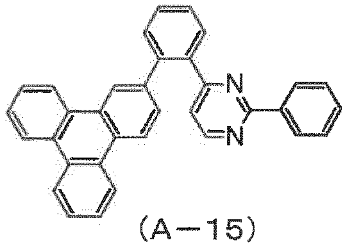
(A−15)

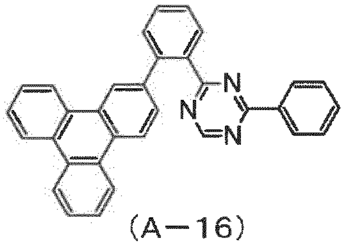
(A−16)

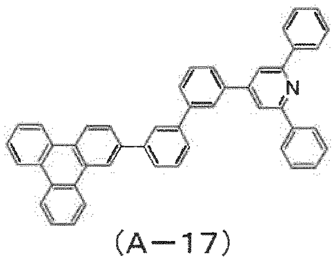
(A−17)

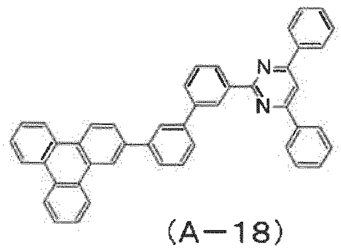
(A−18)

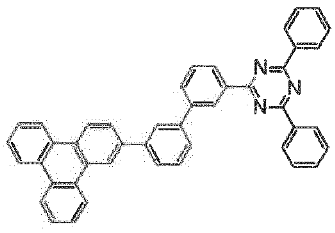
(A−19)

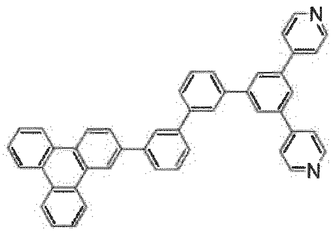
(A−20)

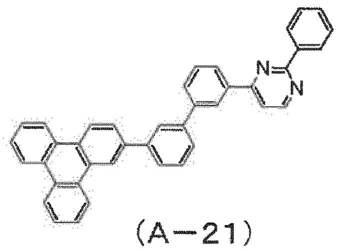
(A−21)

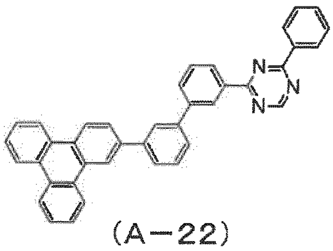
(A−22)

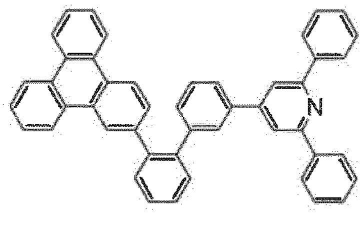
(A−23)

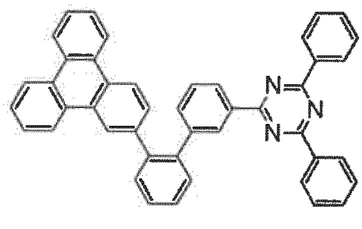
(A−24)

FIG.7

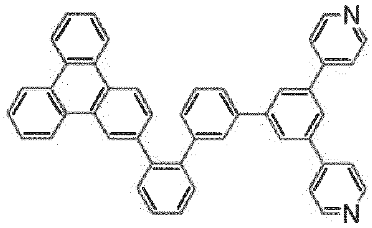

(A−25)

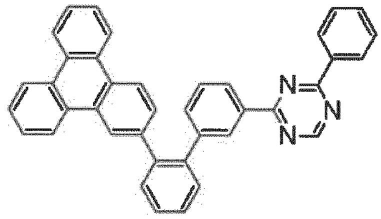

(A−27)

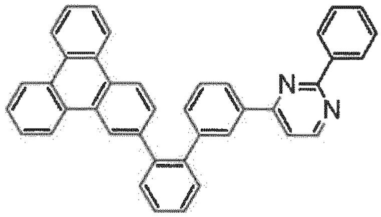

(A−26)

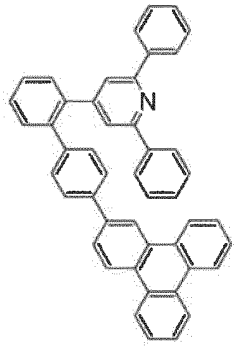

(A−28)

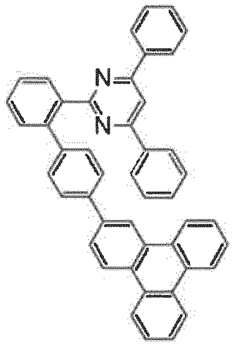

(A−29)

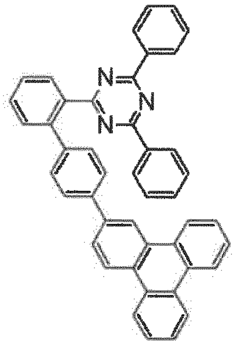

(A−30)

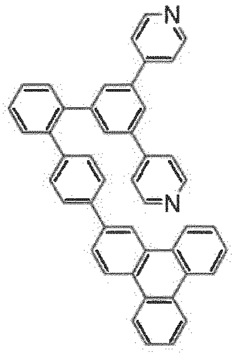

(A−31)

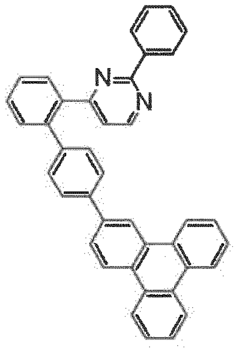

(A−32)

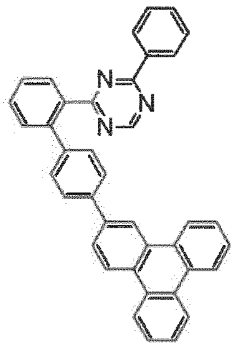

(A−33)

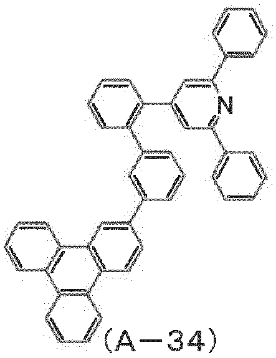

(A−34)

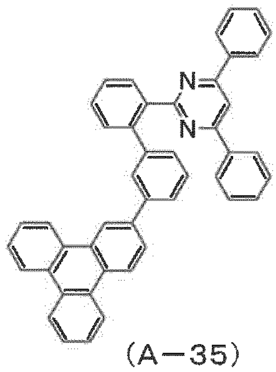

(A−35)

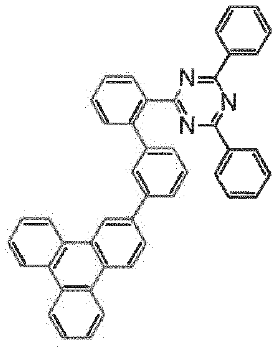

(A−36)

FIG.8

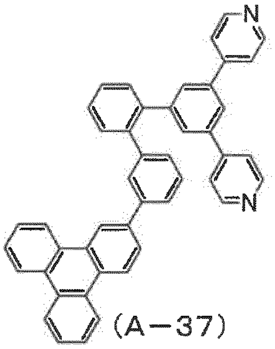

(A-37)

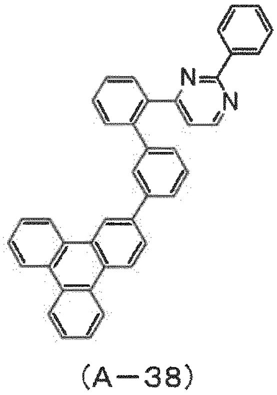

(A-38)

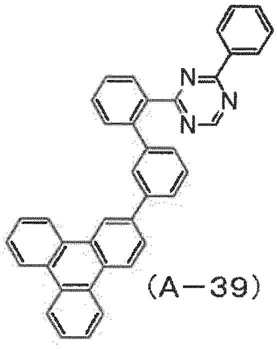

(A-39)

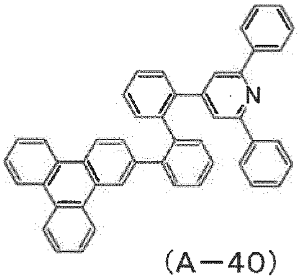

(A-40)

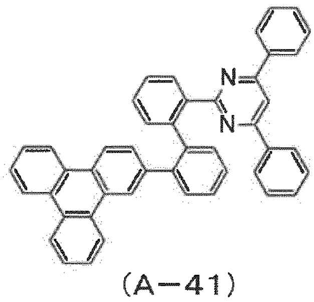

(A-41)

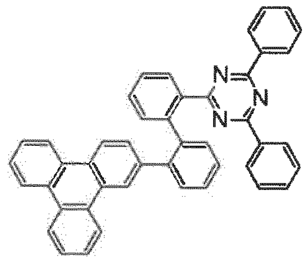

(A-42)

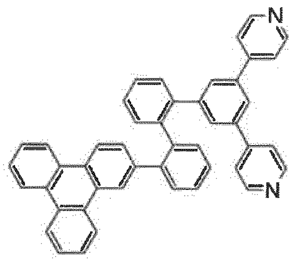

(A-43)

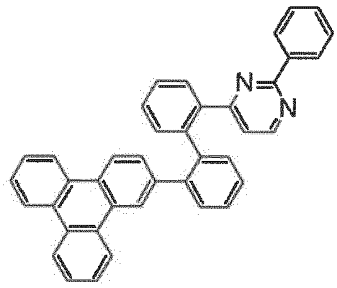

(A-44)

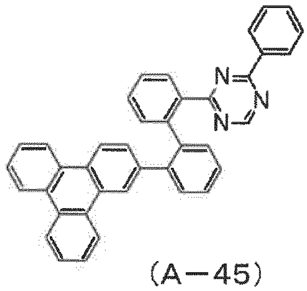

(A-45)

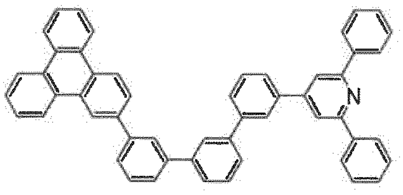

(A-46)

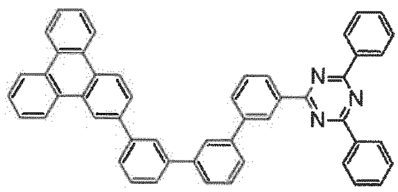

(A-48)

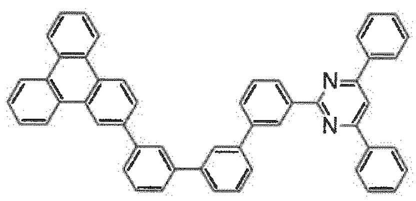

(A-47)

FIG.9

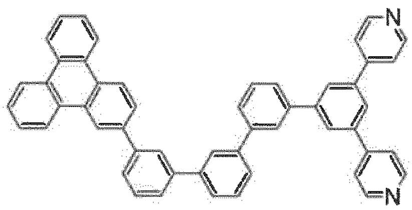

(A−49)

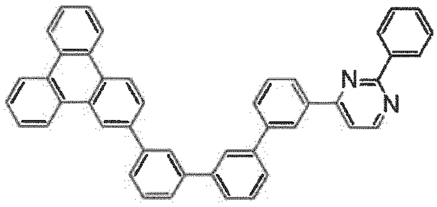

(A−50)

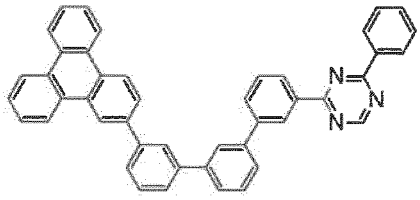

(A−51)

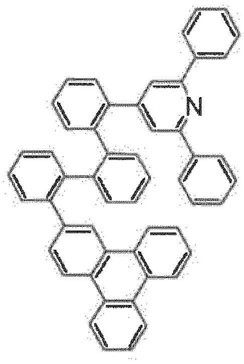

(A−52)

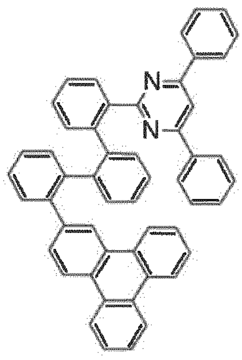

(A−53)

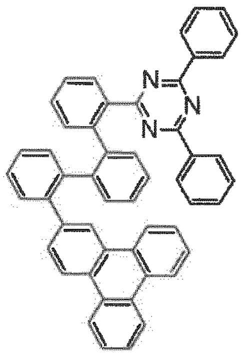

(A−54)

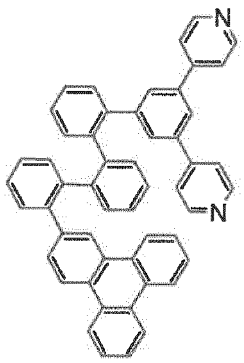

(A−55)

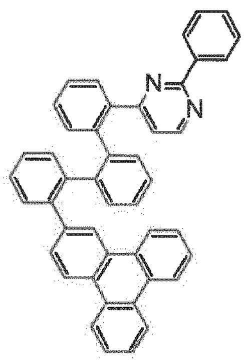

(A−56)

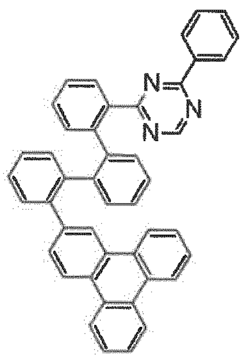

(A−57)

FIG.10

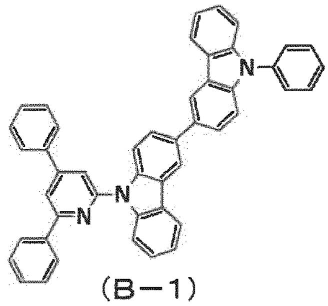

(B−1)

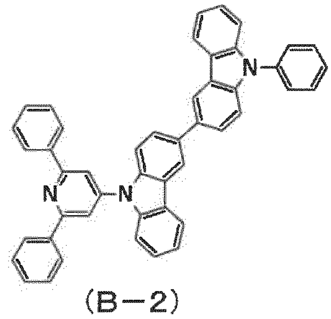

(B−2)

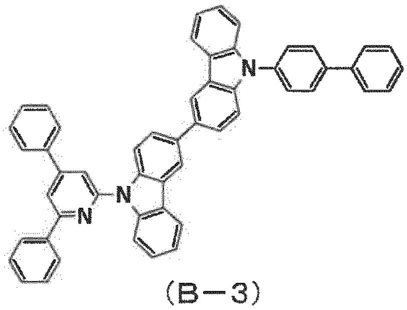

(B−3)

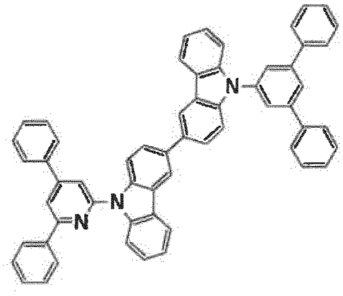

(B−4)

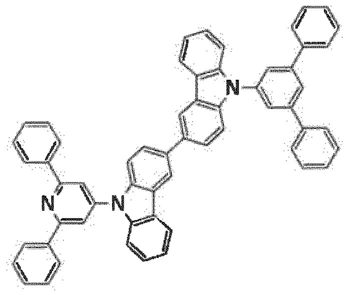

(B−5)

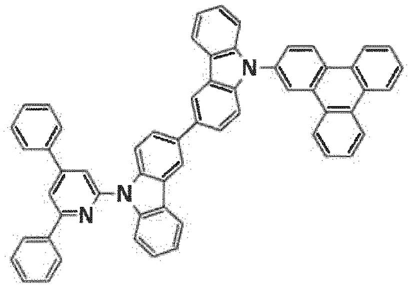

(B−6)

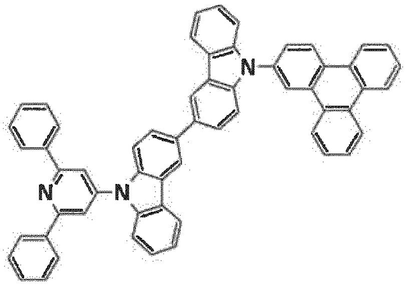

(B−7)

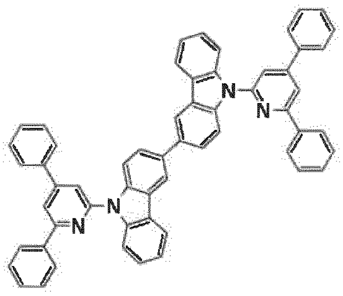

(B−8)

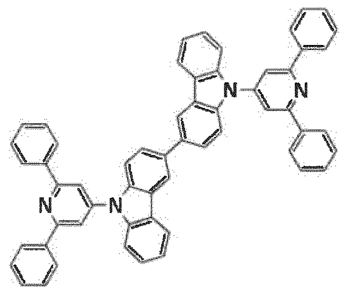

(B−9)

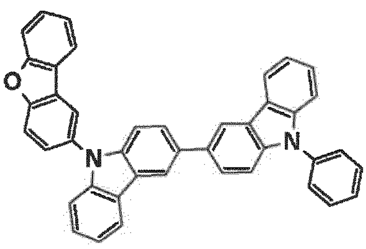

(B−10)

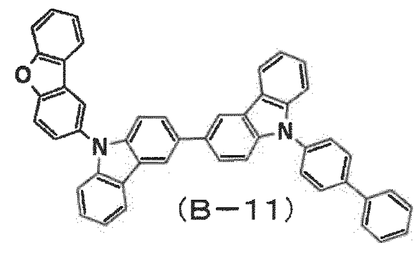

(B−11)

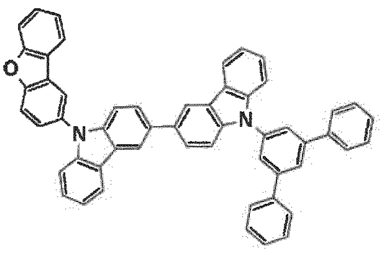

(B−12)

FIG.11

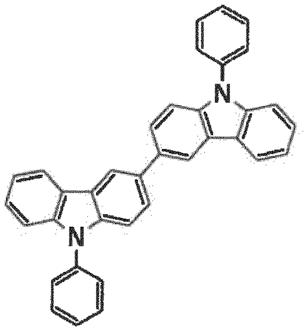

(B−13)

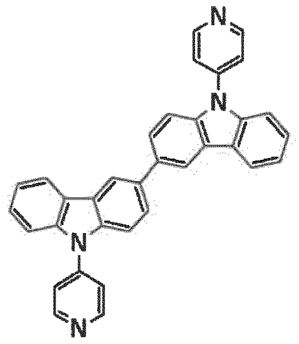

(B−14)

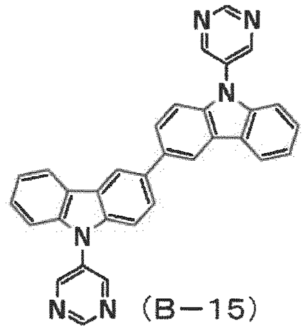

(B−15)

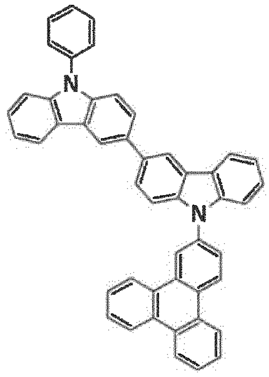

(B−16)

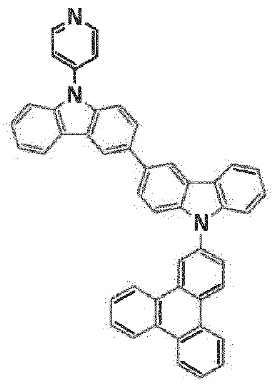

(B−17)

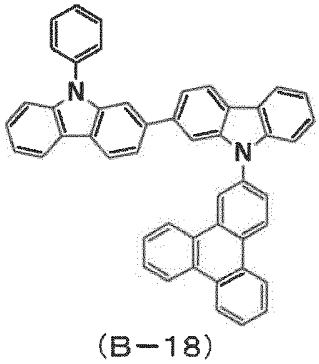

(B−18)

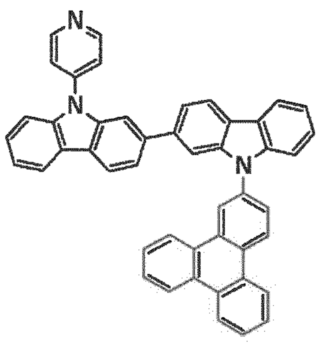

(B−19)

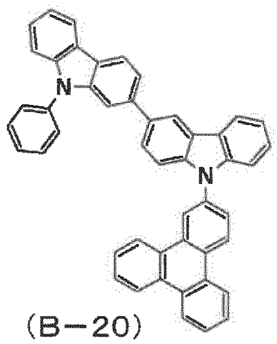

(B−20)

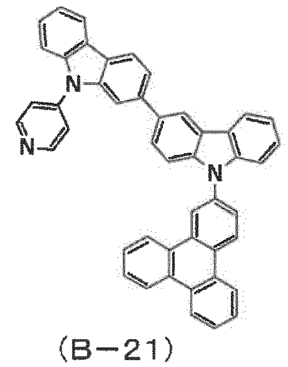

(B−21)

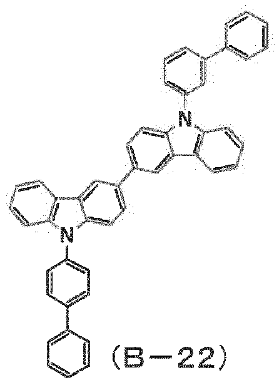

(B−22)

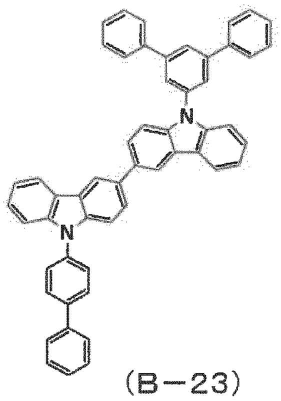

(B−23)

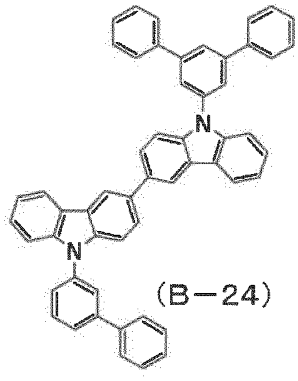

(B−24)

FIG.12

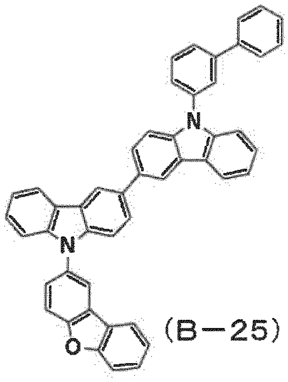

(B−25)

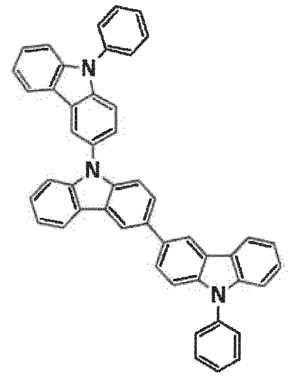

(B−26)

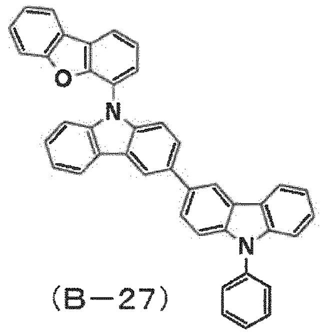

(B−27)

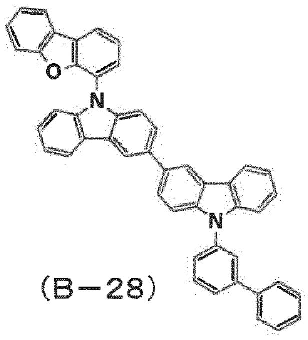

(B−28)

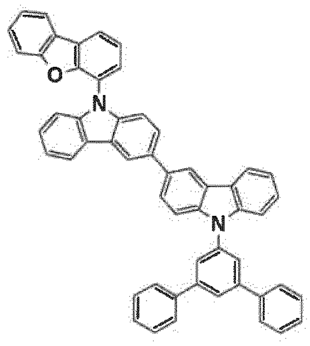

(B−29)

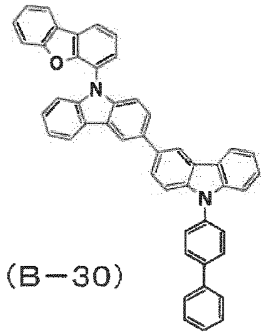

(B−30)

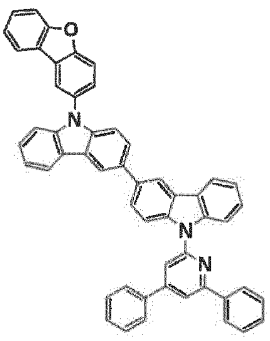

(B−31)

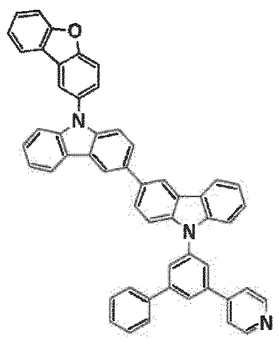

(B−32)

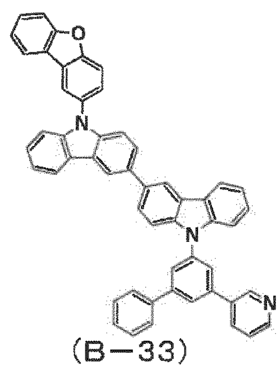

(B−33)

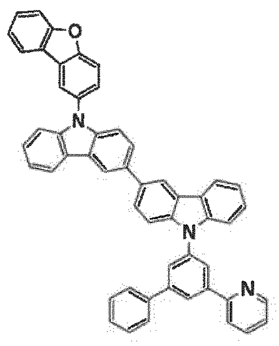

(B−34)

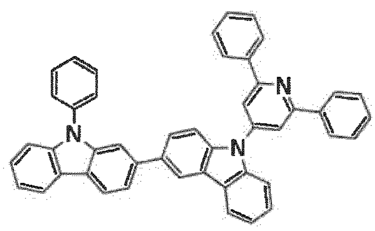

(B−35)

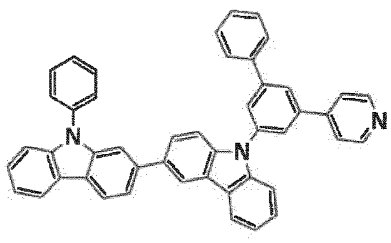

(B−36)

FIG.13

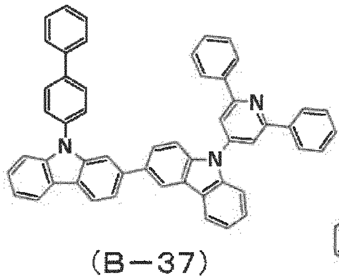

(B−37)

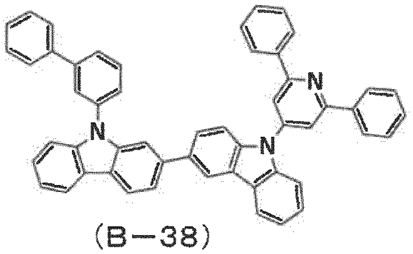

(B−38)

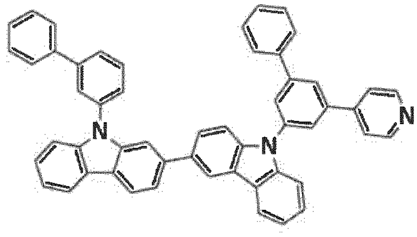

(B−39)

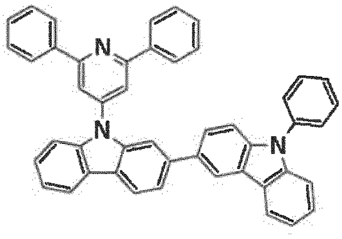

(B−40)

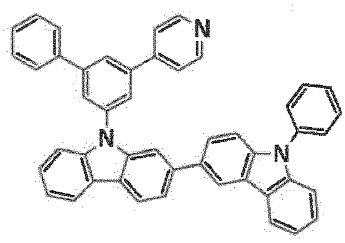

(B−41)

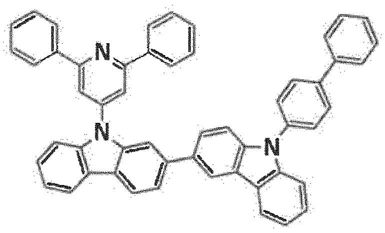

(B−42)

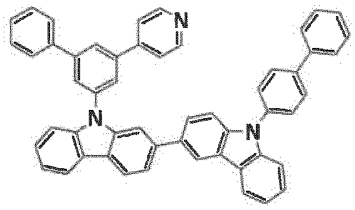

(B−43)

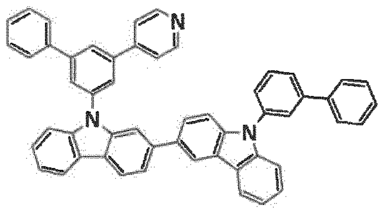

(B−44)

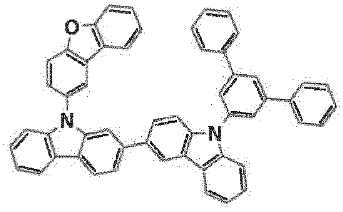

(B−45)

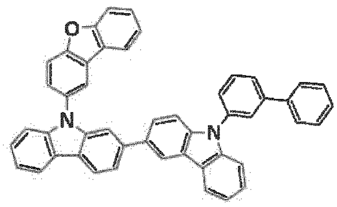

(B−46)

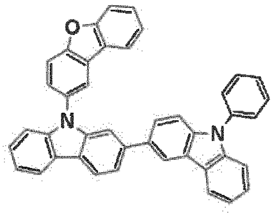

(B−47)

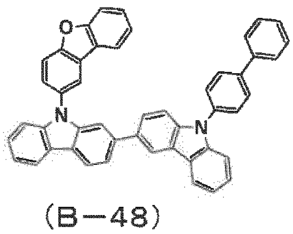

(B−48)

FIG.14

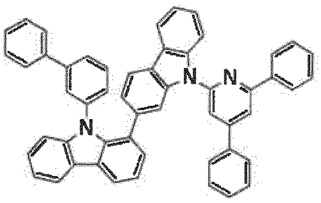

(B−49)

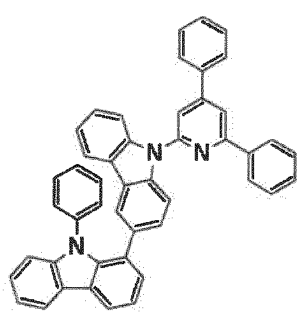

(B−50)

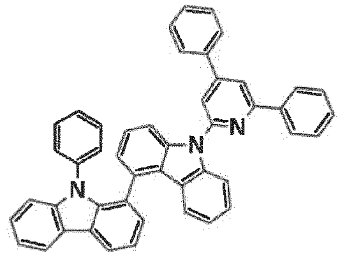

(B−51)

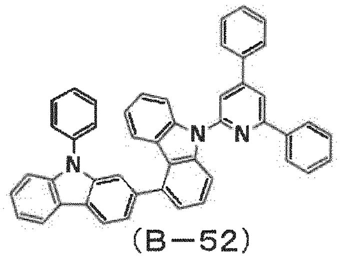

(B−52)

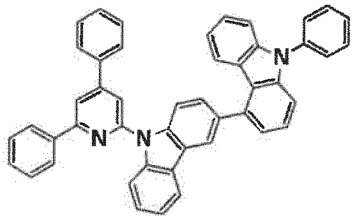

(B−53)

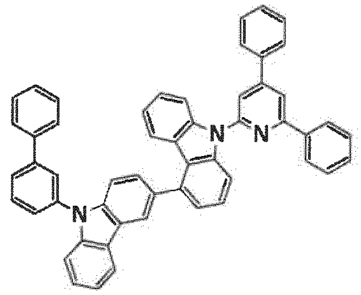

(B−54)

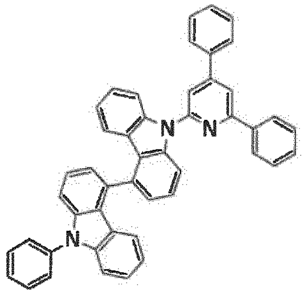

(B−55)

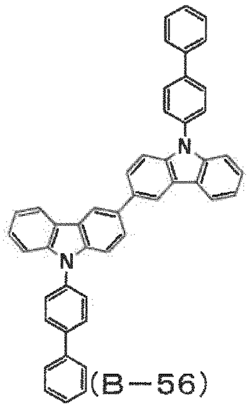

(B−56)

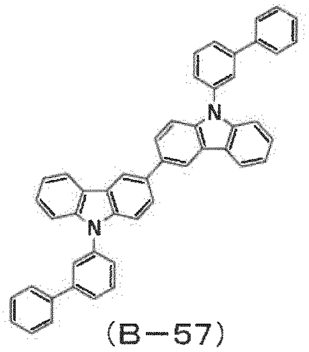

(B−57)

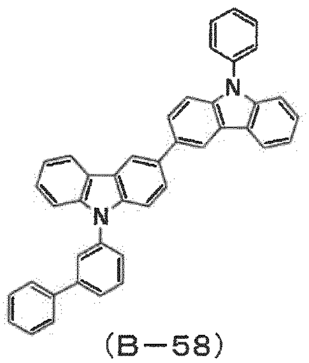

(B−58)

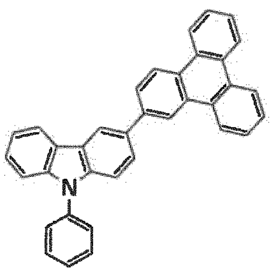

(B−59)

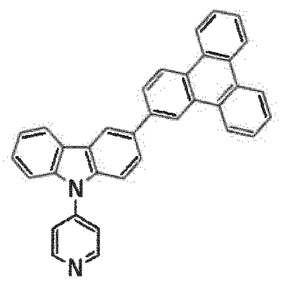

(B −60)

FIG.15

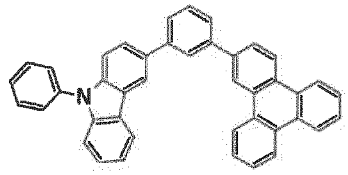

(B−61)

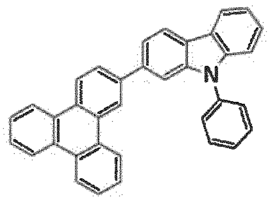

(B−62)

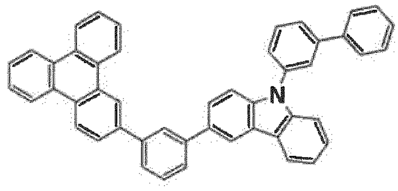

(B−63)

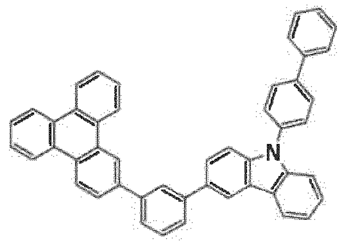

(B−64)

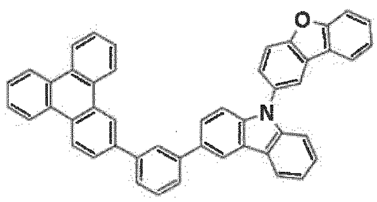

(B−65)

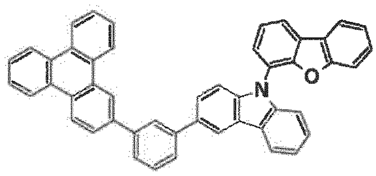

(B−66)

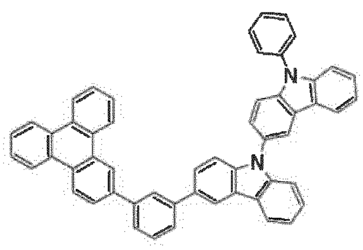

(B−67)

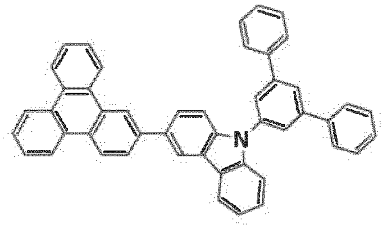

(B−68)

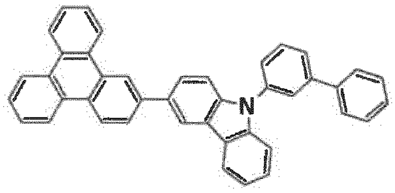

(B−69)

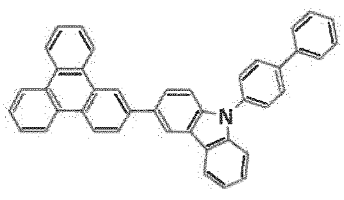

(B−70)

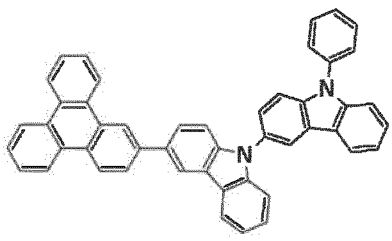

(B−71)

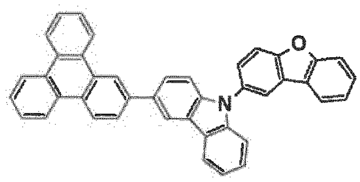

(B−72)

FIG.16

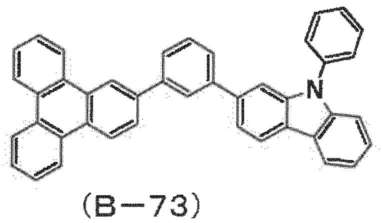

(B−73)

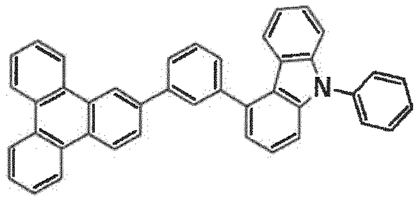

(B−74)

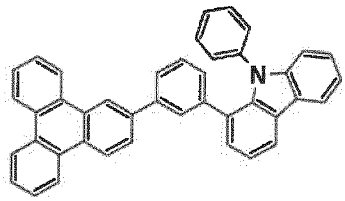

(B−75)

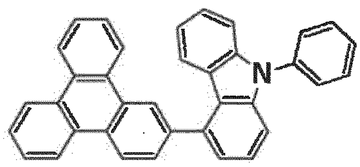

(B−76)

FIG.17

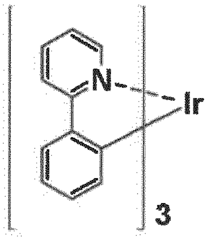

(3−1)

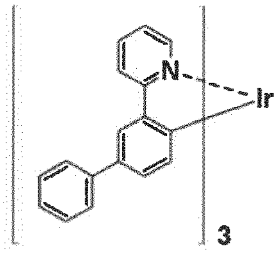

(3−2)

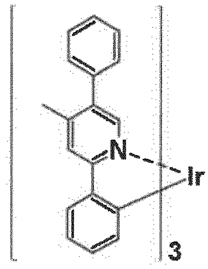

(3−3)

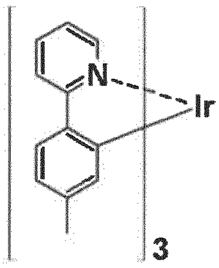

(3−4)

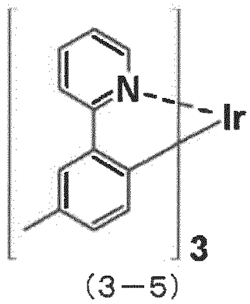

(3−5)

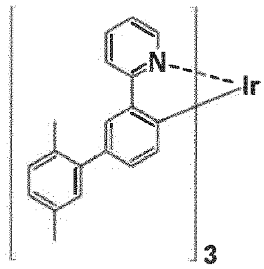

(3−6)

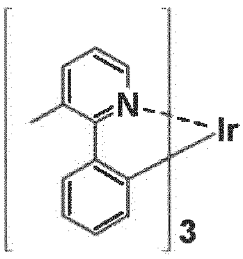

(3−7)

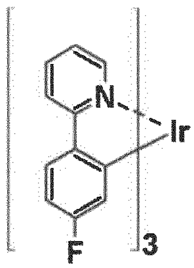

(3−8)

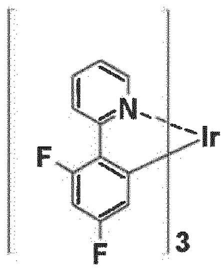

(3−9)

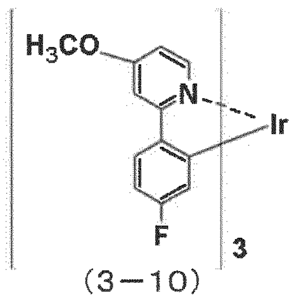

(3−10)

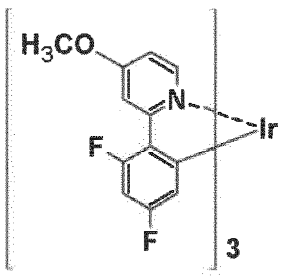

(3−11)

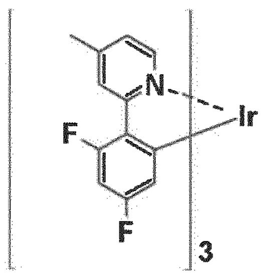

(3−12)

FIG.18

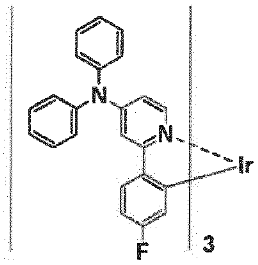

(3−13)

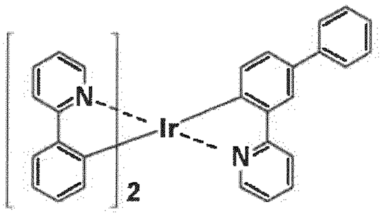

(3−14)

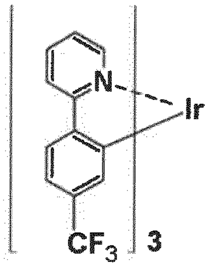

(3−15)

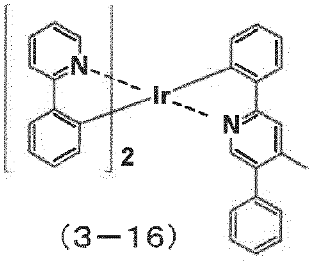

(3−16)

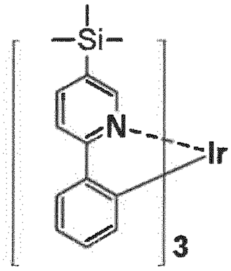

(3−17)

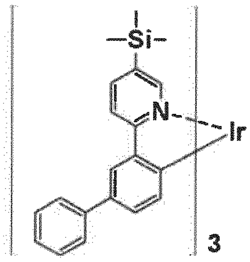

(3−18)

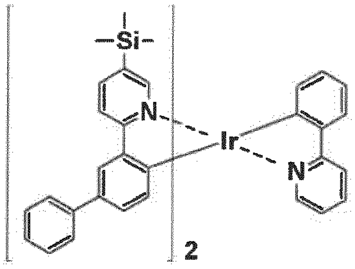

(3−19)

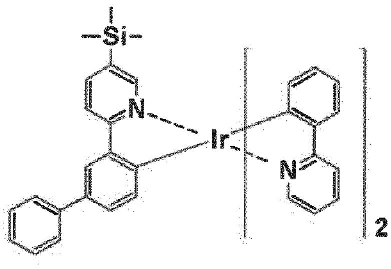

(3−20)

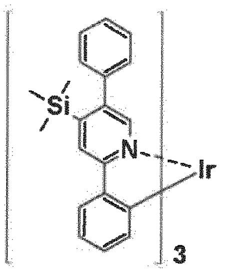

(3−21)

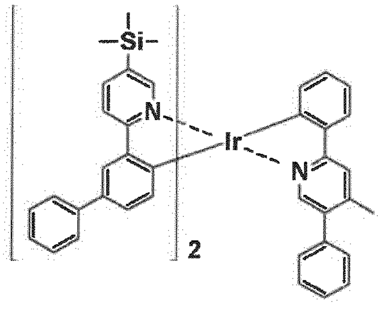

(3−22)

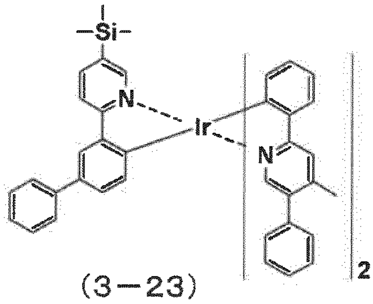

(3−23)

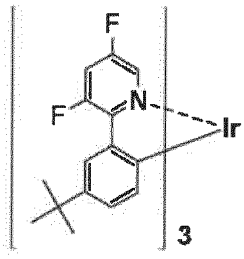

(3−24)

FIG.19

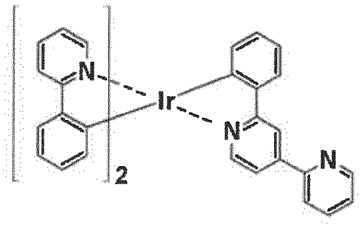

(3−25)

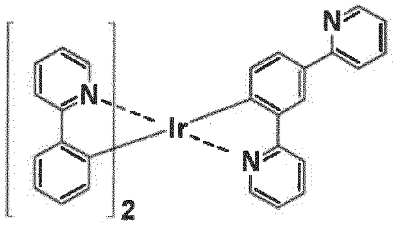

(3−26)

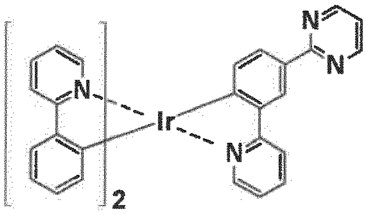

(3−27)

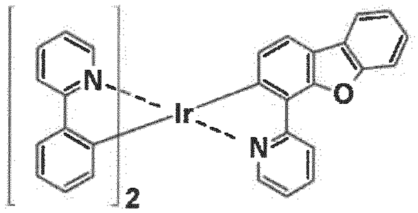

(3−28)

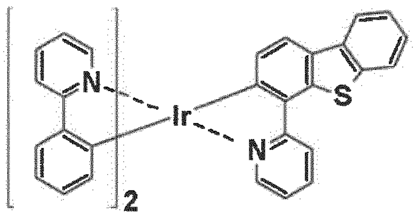

(3−29)

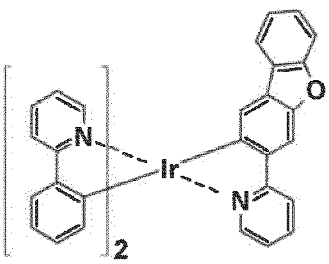

(3−31)

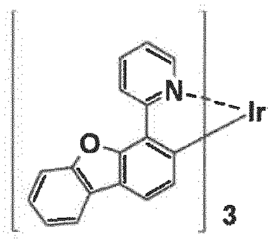

(3−32)

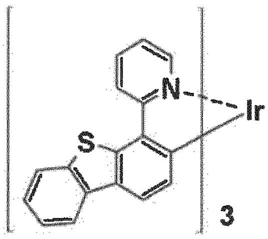

(3−33)

FIG.20

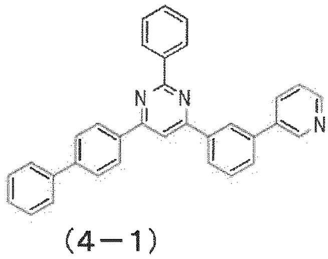

(4−1)

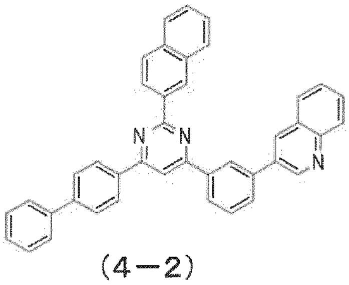

(4−2)

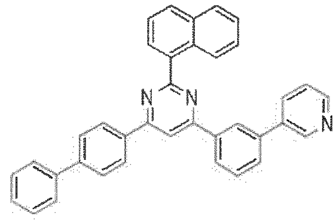

(4−3)

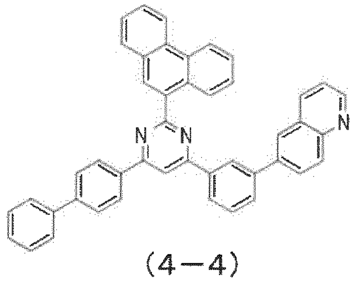

(4−4)

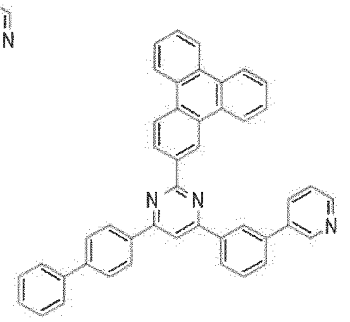

(4−5)

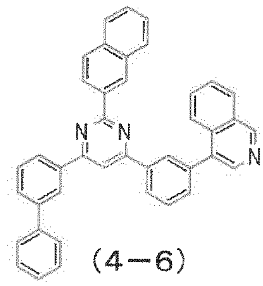

(4−6)

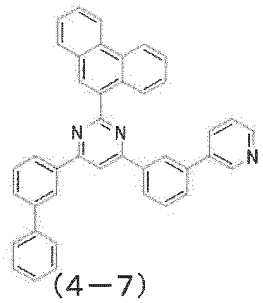

(4−7)

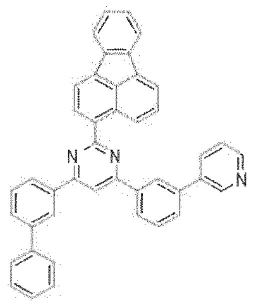

(4−8)

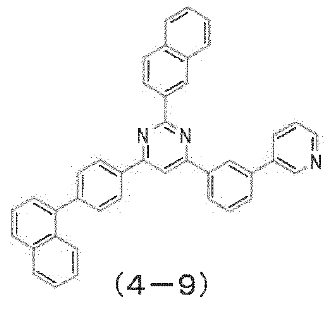

(4−9)

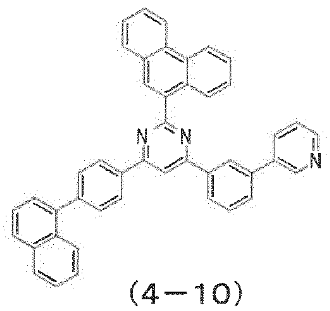

(4−10)

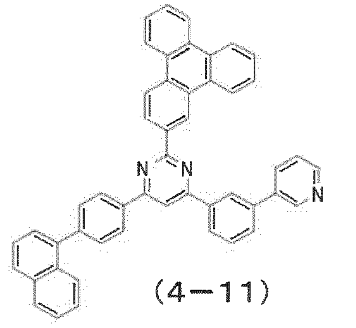

(4−11)

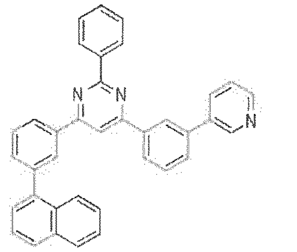

(4−12)

FIG.21

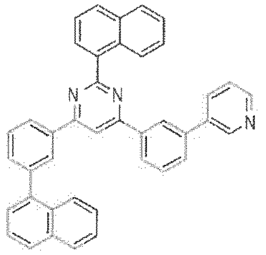

(4−13)

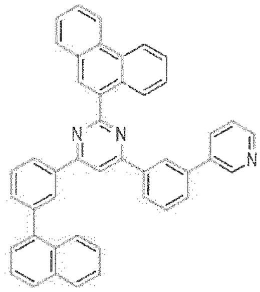

(4−14)

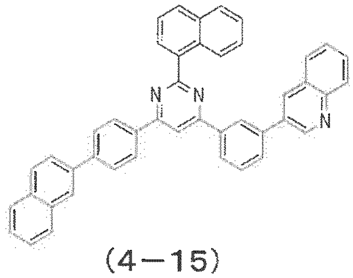

(4−15)

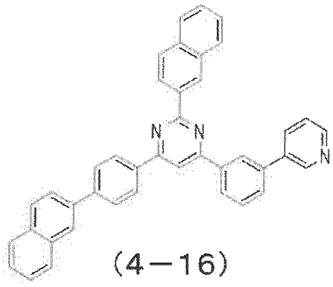

(4−16)

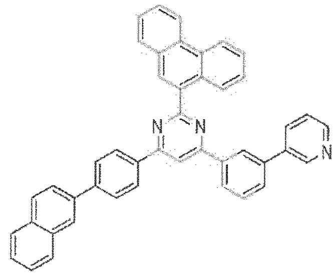

(4−17)

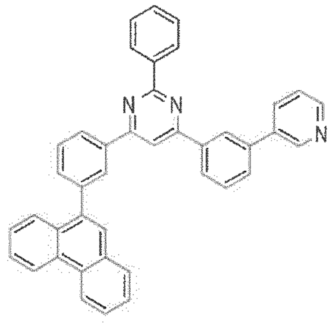

(4−18)

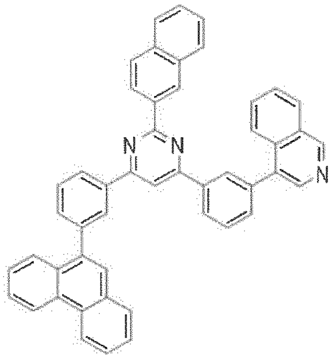

(4−19)

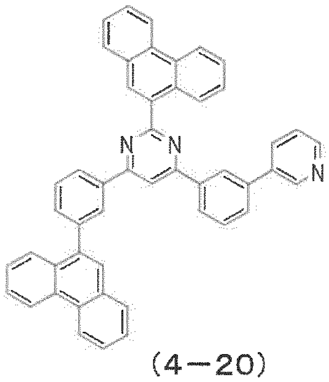

(4−20)

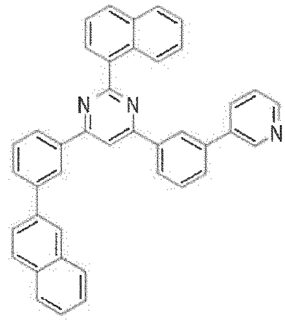

(4−21)

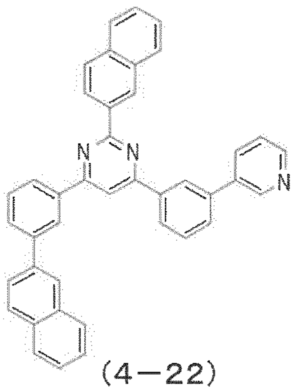

(4−22)

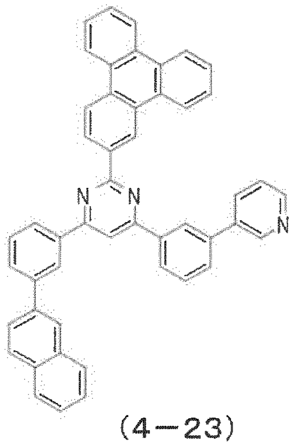

(4−23)

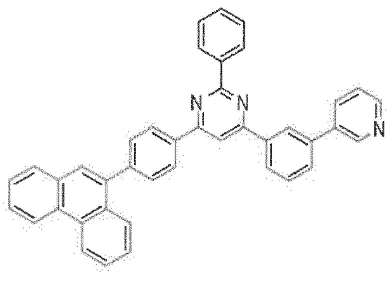

(4−24)

FIG.22

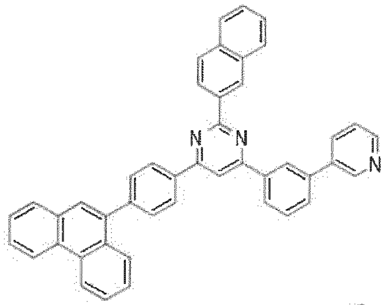

(4-25)

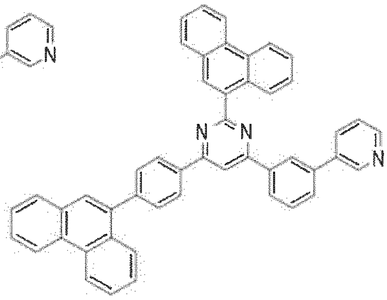

(4-26)

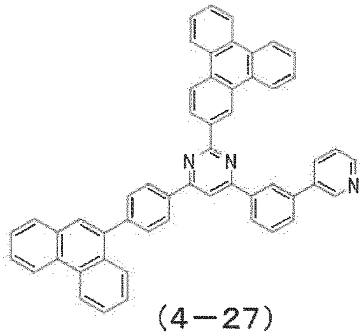

(4-27)

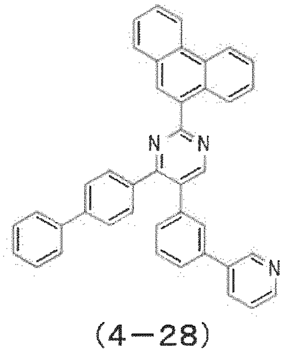

(4-28)

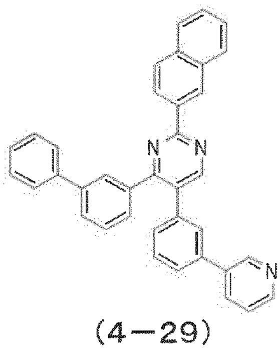

(4-29)

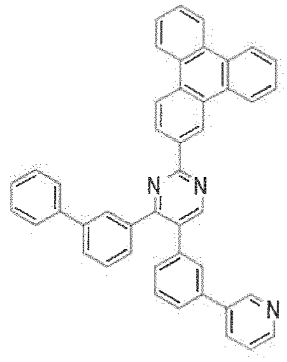

(4-30)

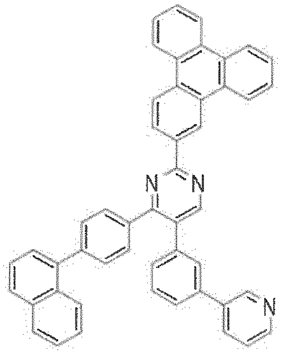

(4-31)

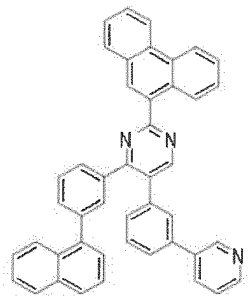

(4-32)

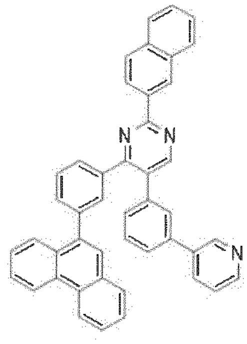

(4-33)

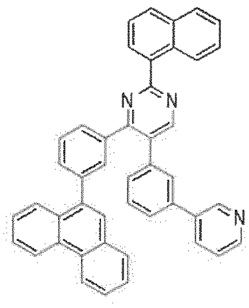

(4-34)

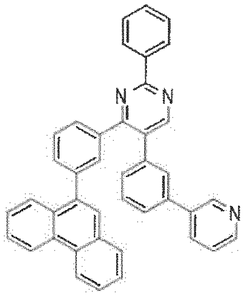

(4-35)

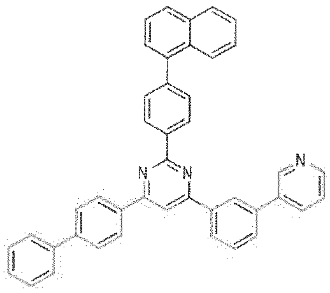

(4-36)

FIG.23

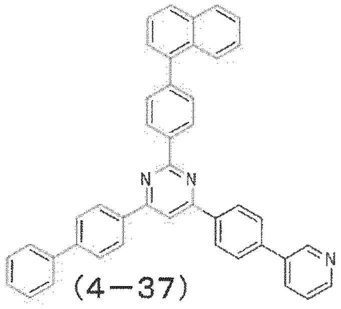

(4−37)

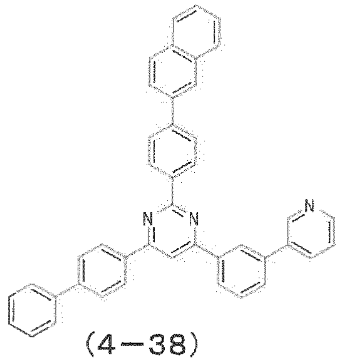

(4−38)

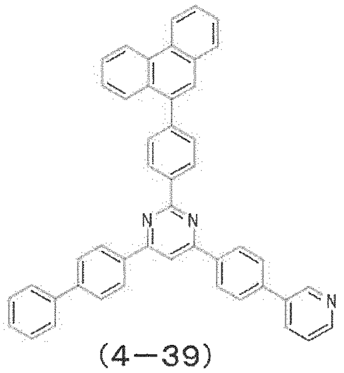

(4−39)

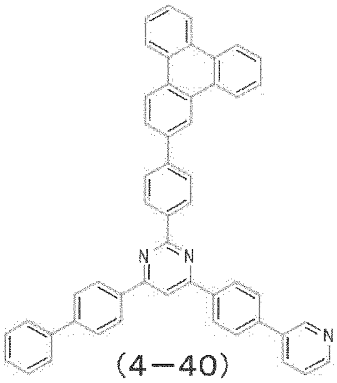

(4−40)

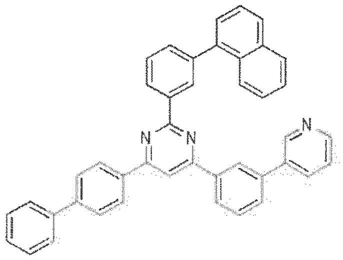

(4−41)

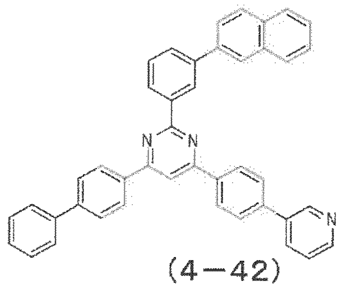

(4−42)

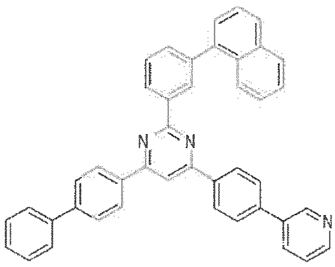

(4−43)

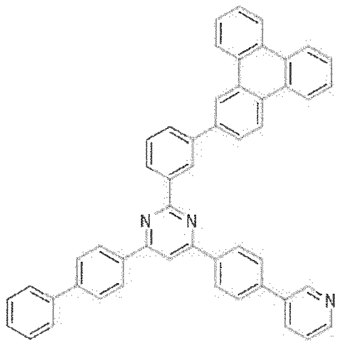

(4−44)

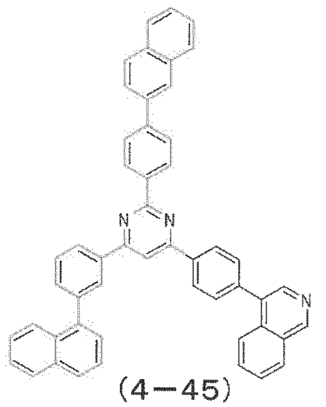

(4−45)

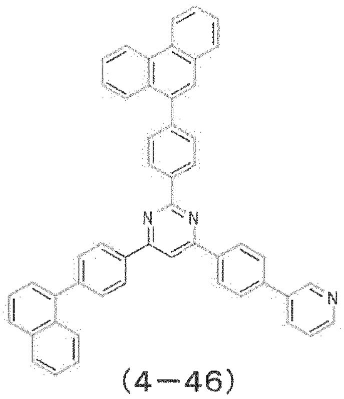

(4−46)

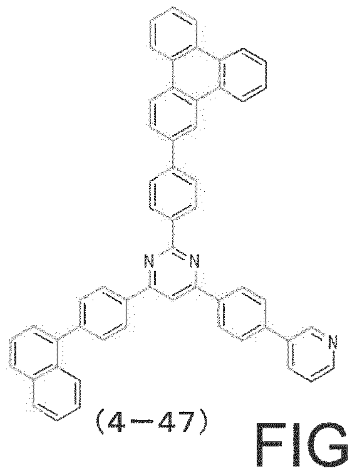

(4−47)

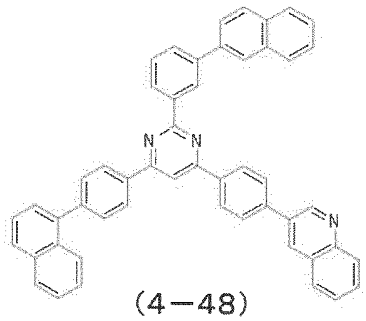

(4−48)

FIG.24

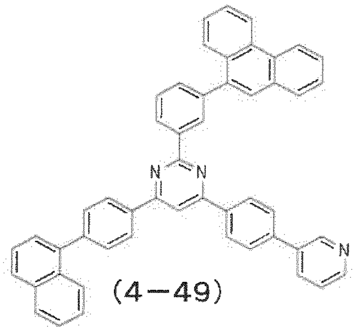

(4−49)

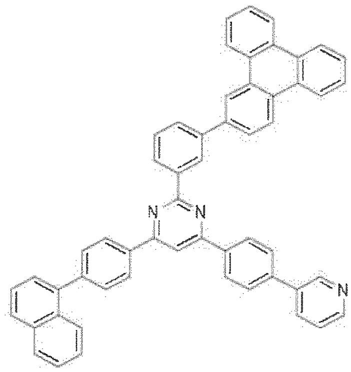

(4−50)

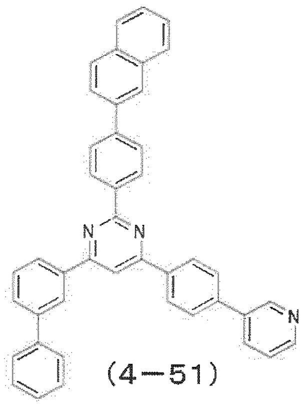

(4−51)

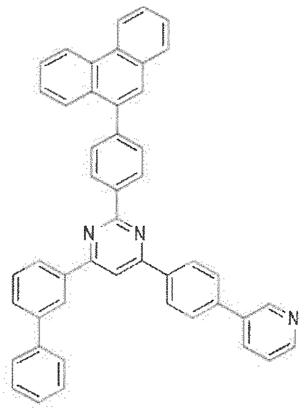

(4−52)

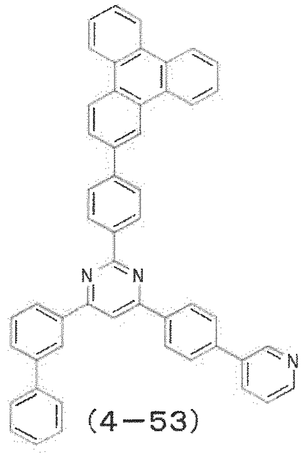

(4−53)

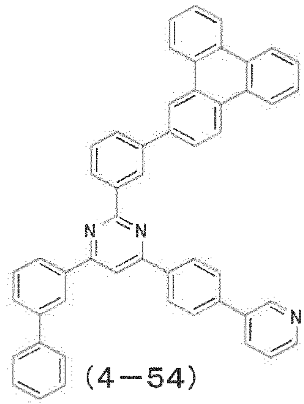

(4−54)

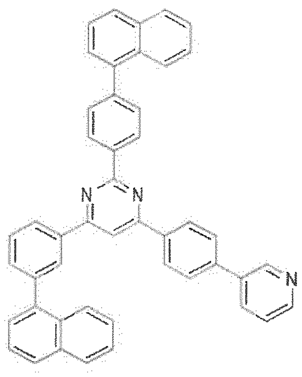

(4−55)

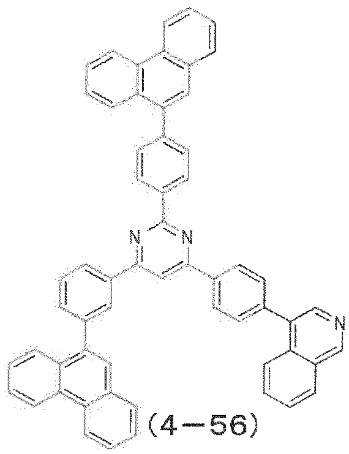

(4−56)

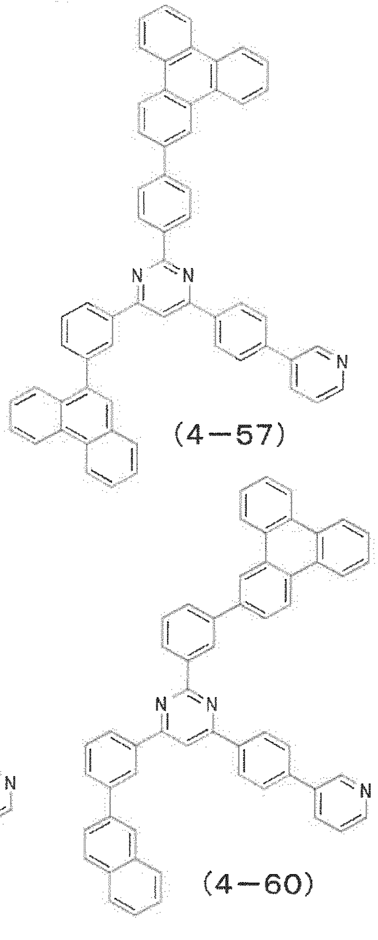

(4−57)

(4−60)

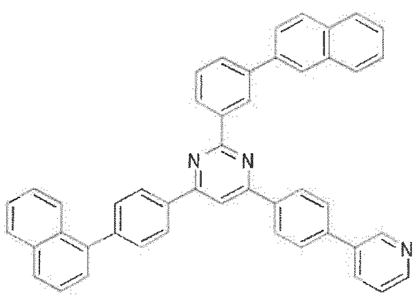

(4−58)

FIG.25

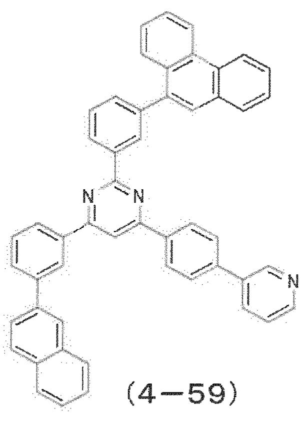

(4−59)

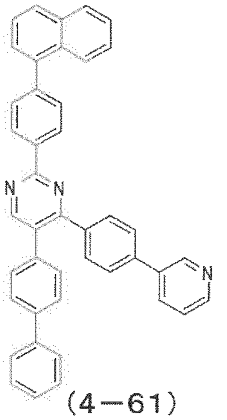

(4−61)

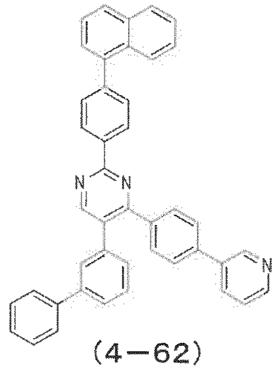

(4−62)

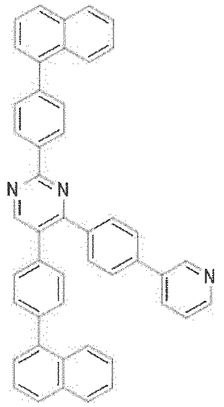

(4−63)

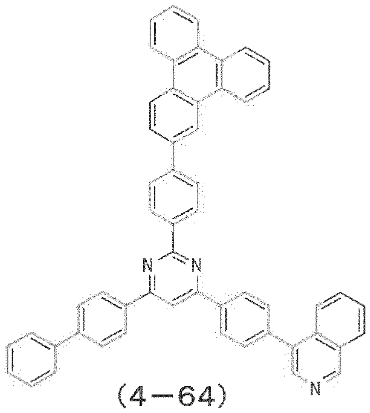

(4−64)

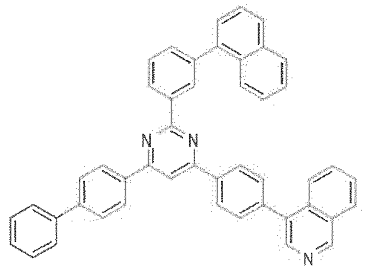

(4−65)

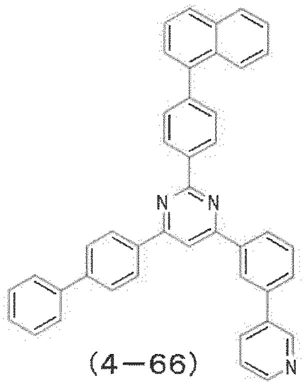

(4−66)

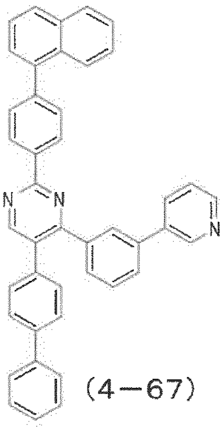

(4−67)

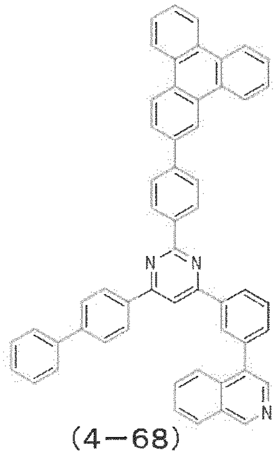

(4−68)

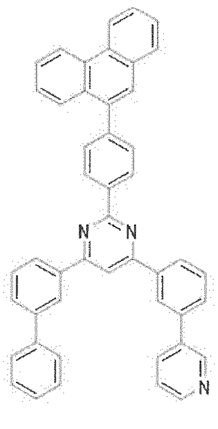

(4−69)

FIG.26

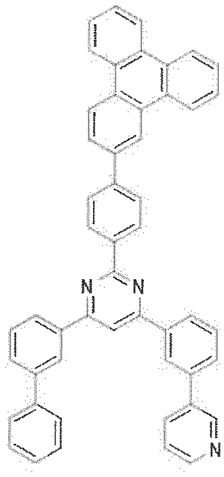

(4-70)

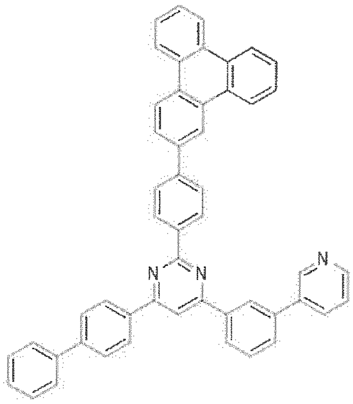

(4-71)

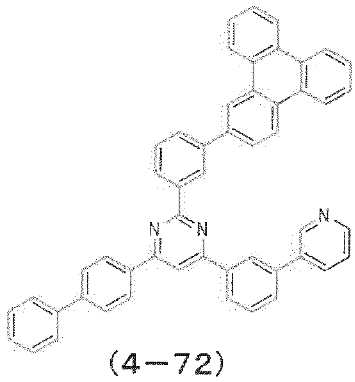

(4-72)

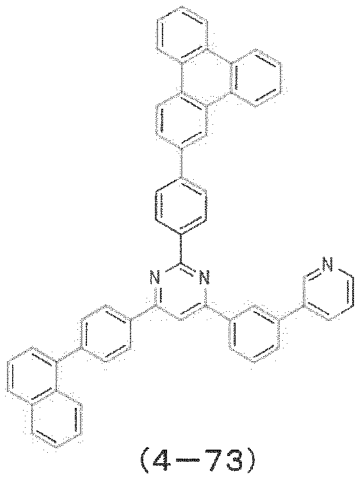

(4-73)

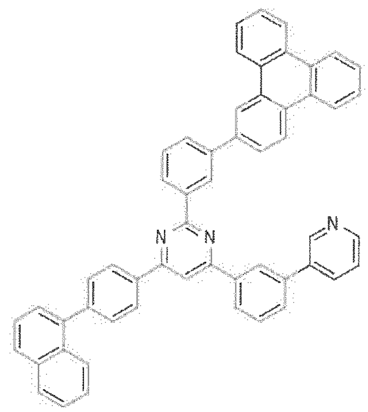

(4-74)

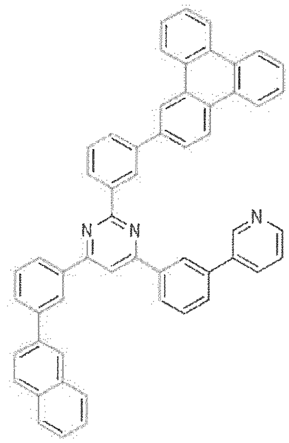

(4-75)

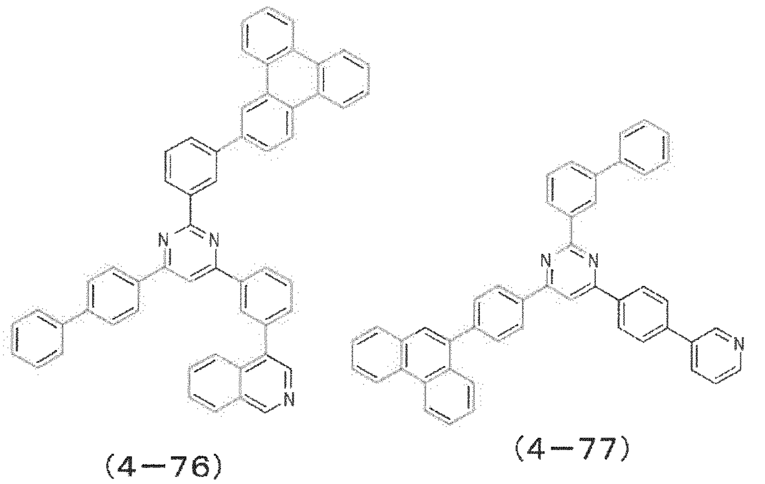

(4-76)

(4-77)

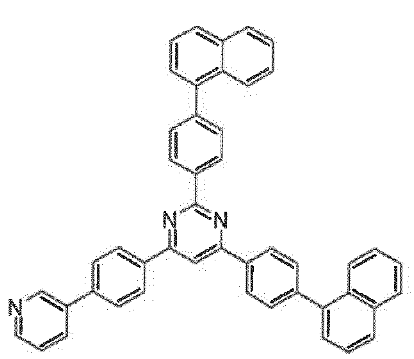

(4-78)

FIG.27

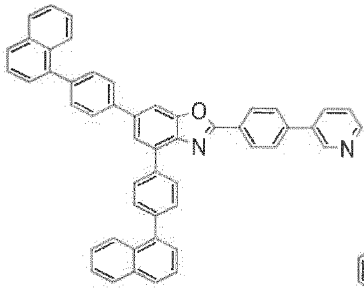

(6−1)

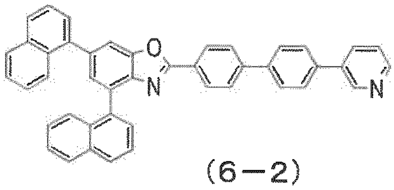

(6−2)

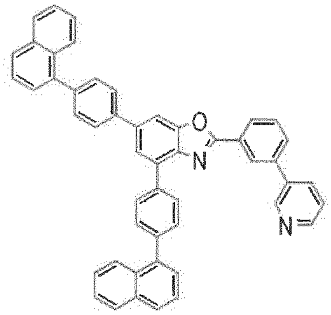

(6−3)

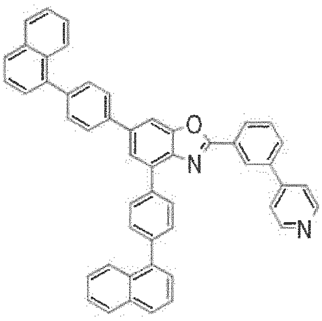

(6−4)

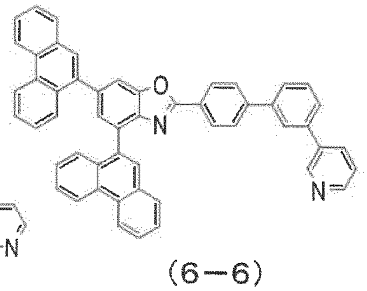

(6−6)

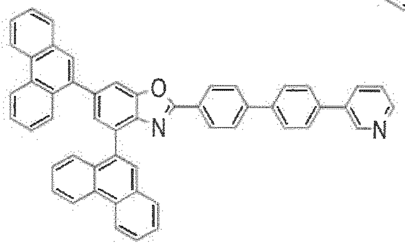

(6−5)

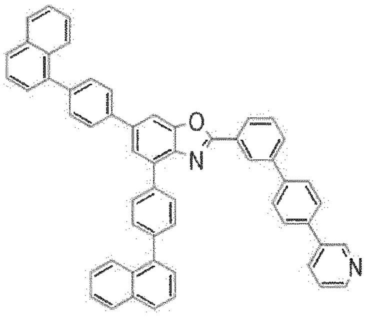

(6−7)

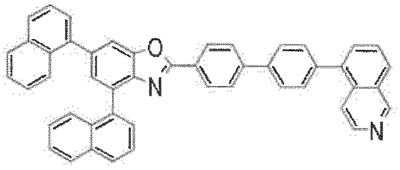

(6−9)

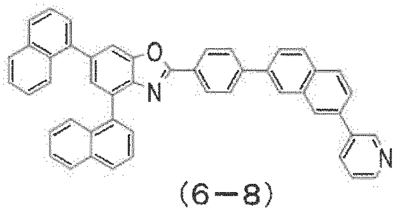

(6−8)

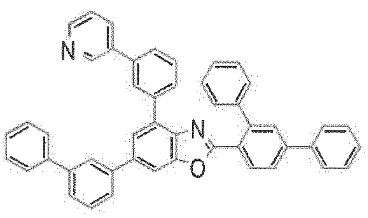

(6−10)

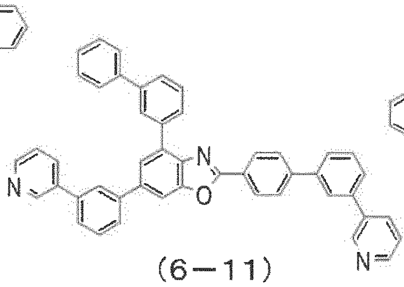

FIG.28

(6−11)

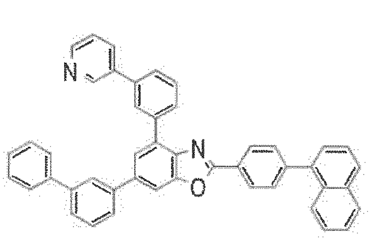

(6−12)

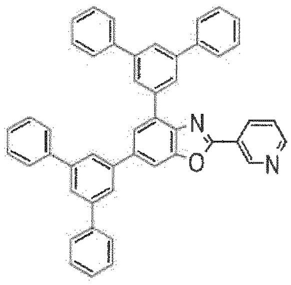

(6−13)

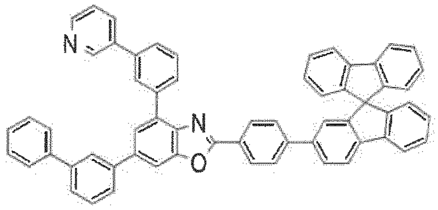

(6−14)

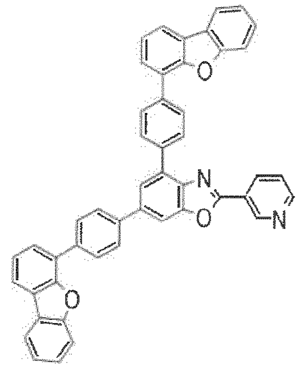

(6−15)

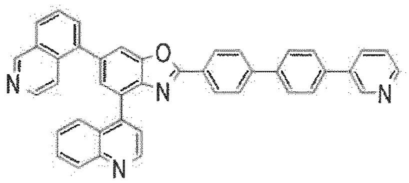

(6−16)

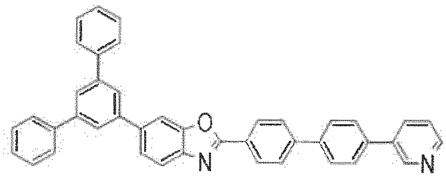

(6−18)

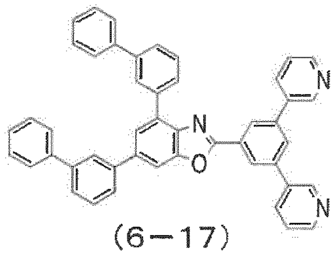

(6−17)

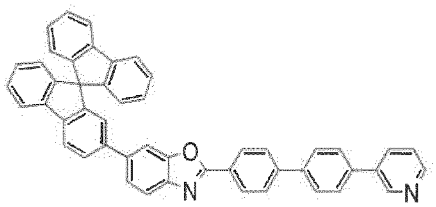

(6−19)

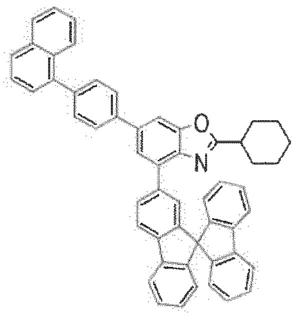

(6−21)

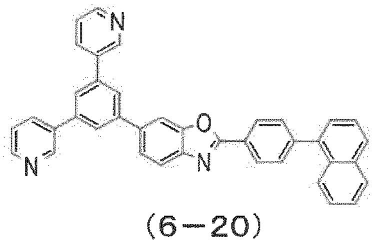

(6−20)

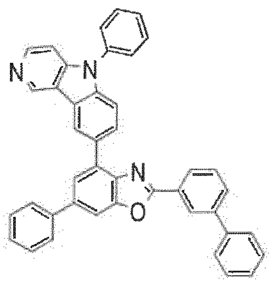

(6−22)

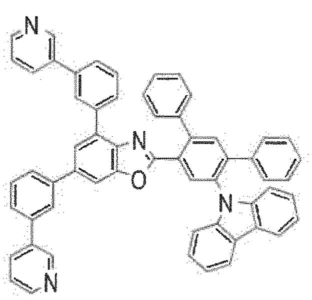

(6−23)

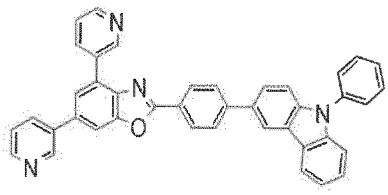

(6−24)

FIG.29

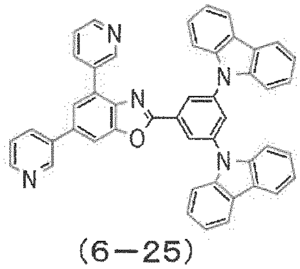

(6−25)

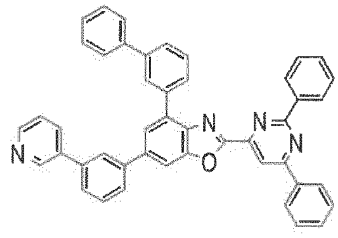

(6−26)

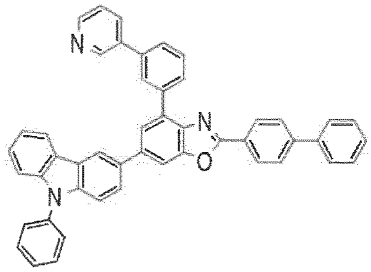

(6−27)

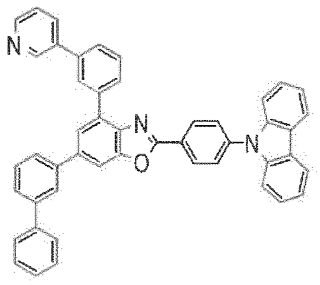

(6−28)

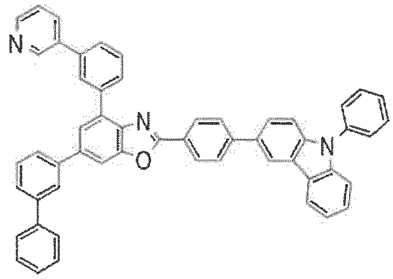

(6−29)

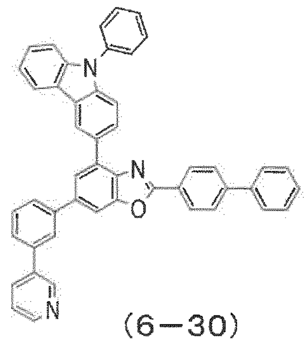

(6−30)

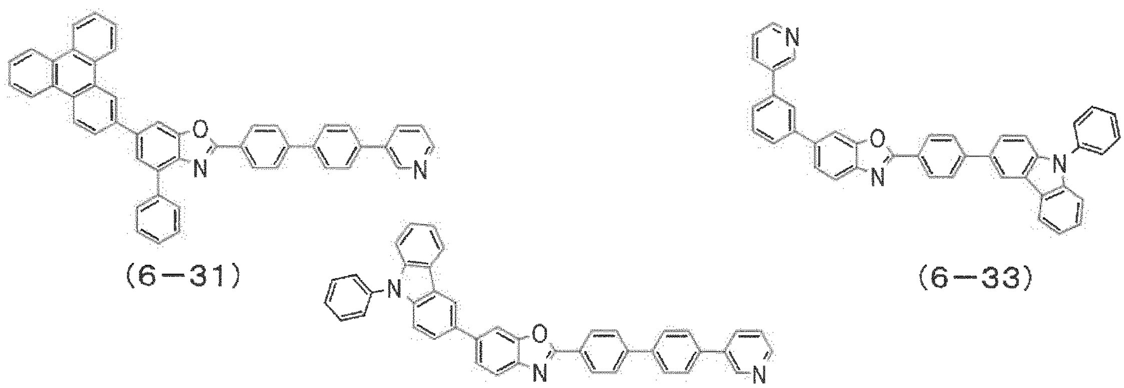

(6−31)

(6−32)

(6−33)

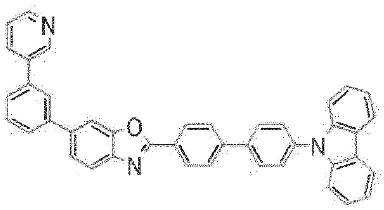

(6−34)

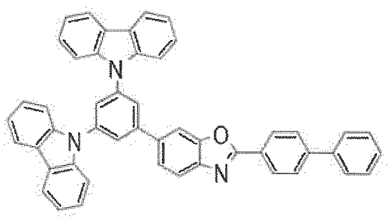

(6−36)

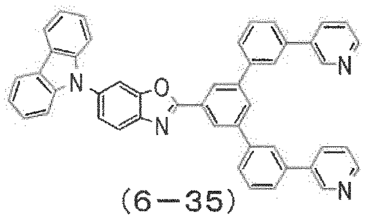

(6−35)

FIG.30

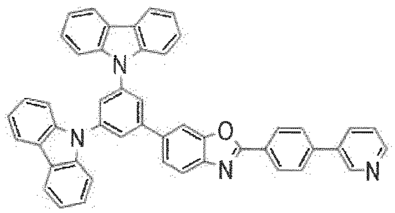

(6-37)

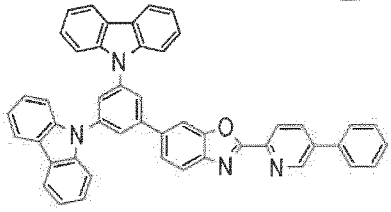

(6-38)

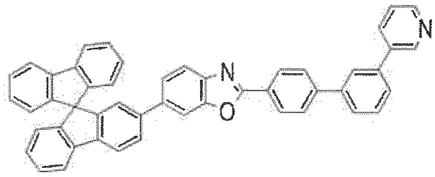

(6-39)

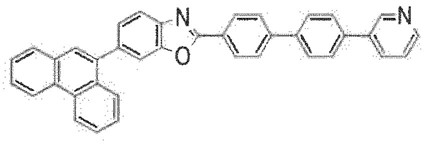

(6-40)

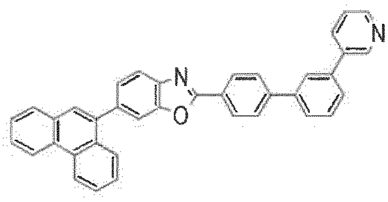

(6-41)

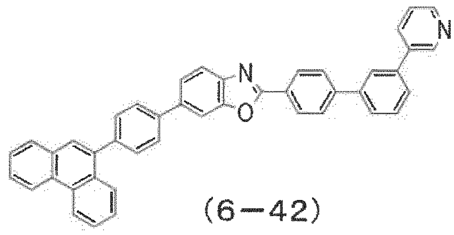

(6-42)

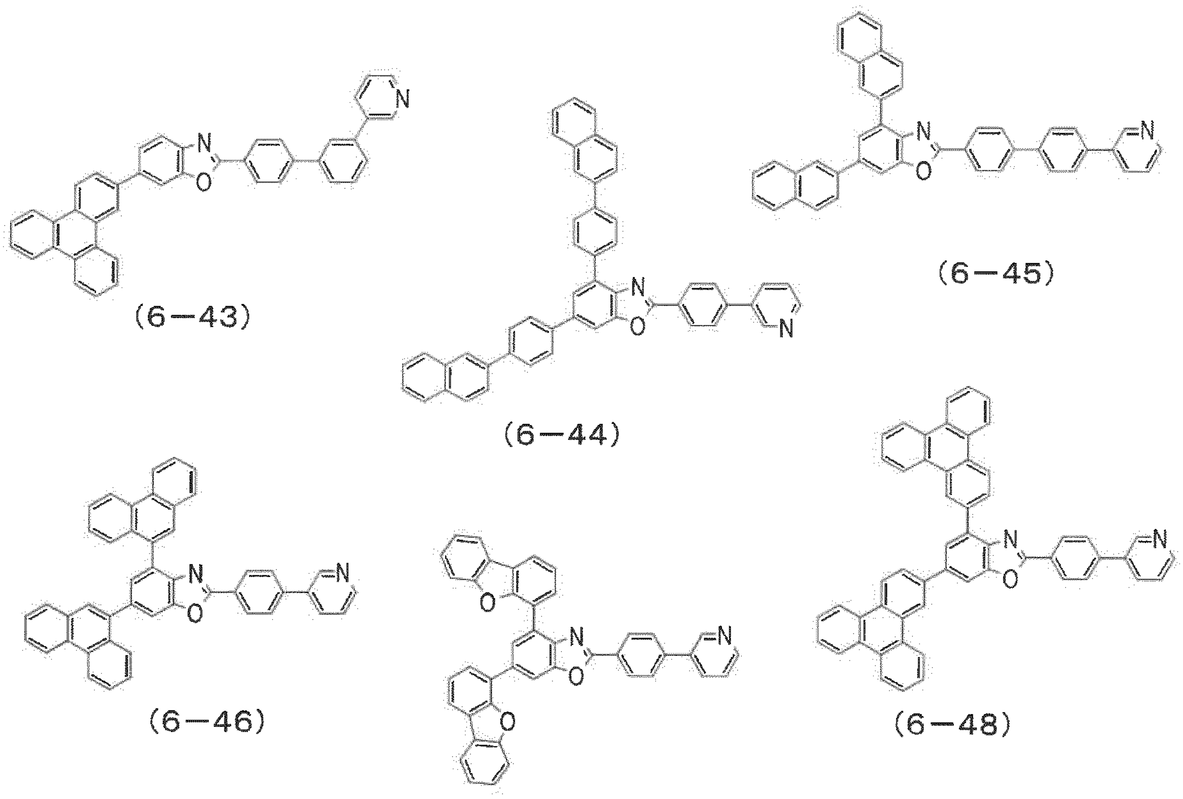

(6-43)

(6-44)

(6-45)

(6-46)

(6-47)

(6-48)

FIG.31

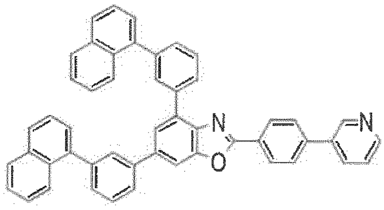

(6−49)

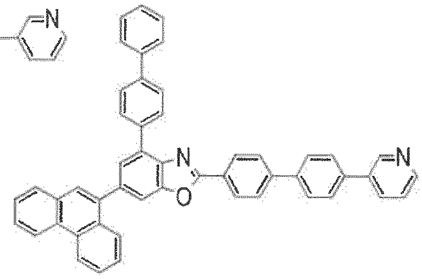

(6−50)

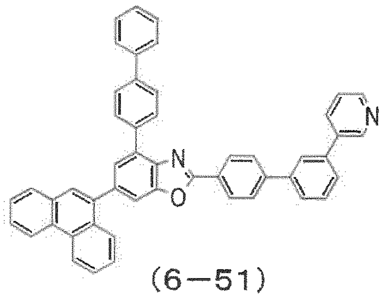

(6−51)

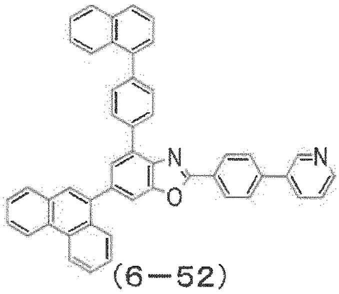

(6−52)

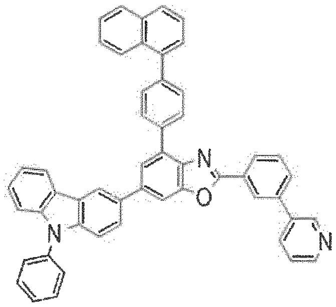

(6−53)

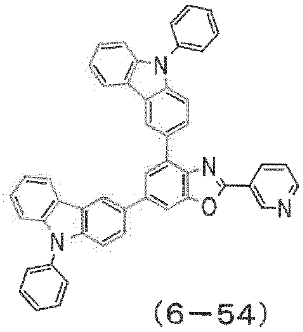

(6−54)

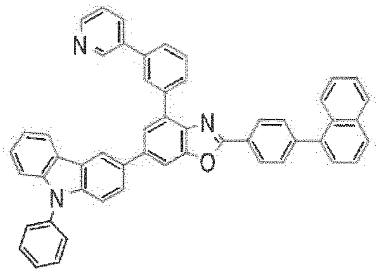

(6−55)

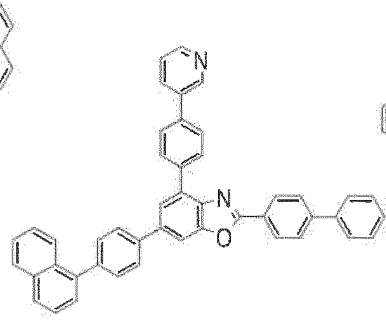

(6−56)

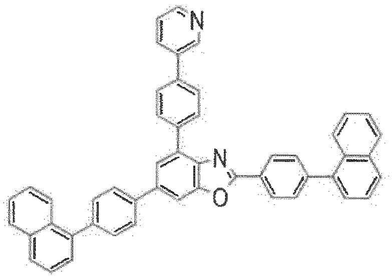

(6−57)

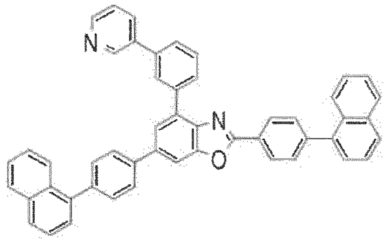

(6−58)

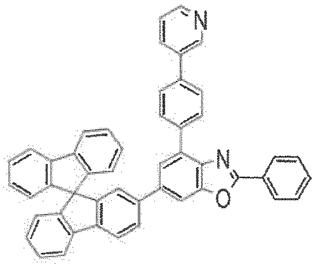

(6−59)

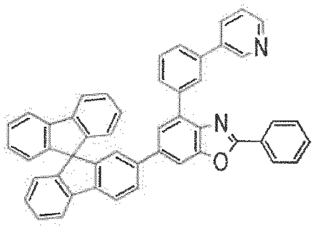

(6−60)

FIG.32

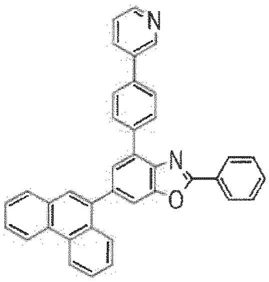

(6−61)

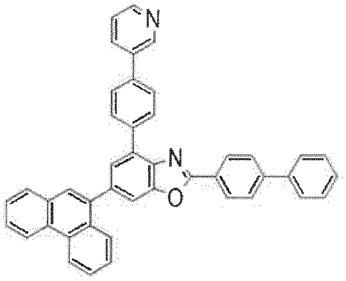

(6−62)

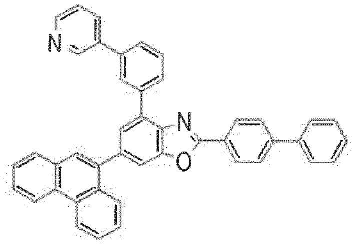

(6−63)

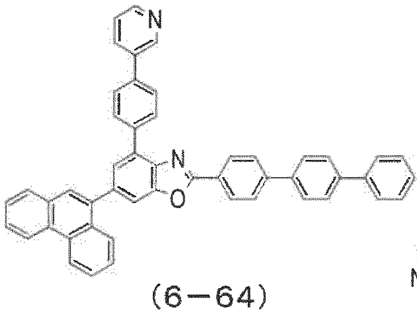

(6−64)

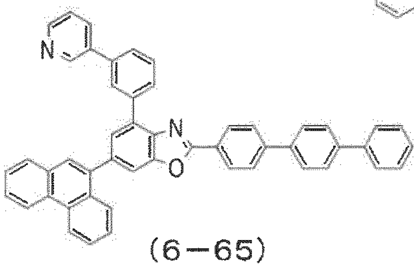

(6−65)

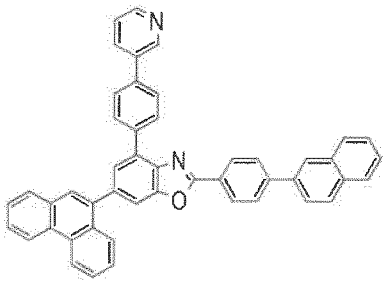

(6−66)

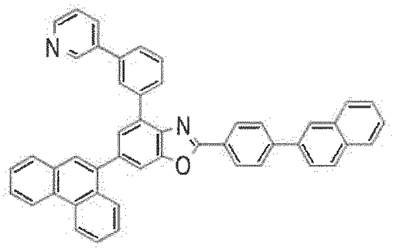

(6−67)

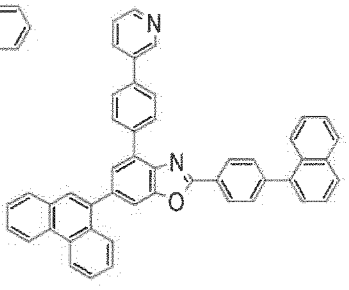

(6−68)

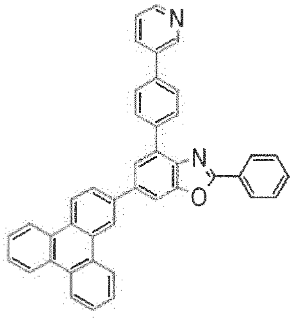

(6−69)

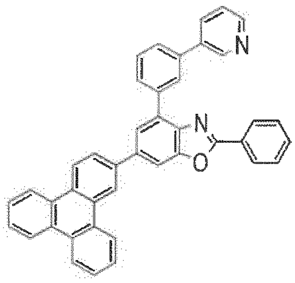

(6−70)

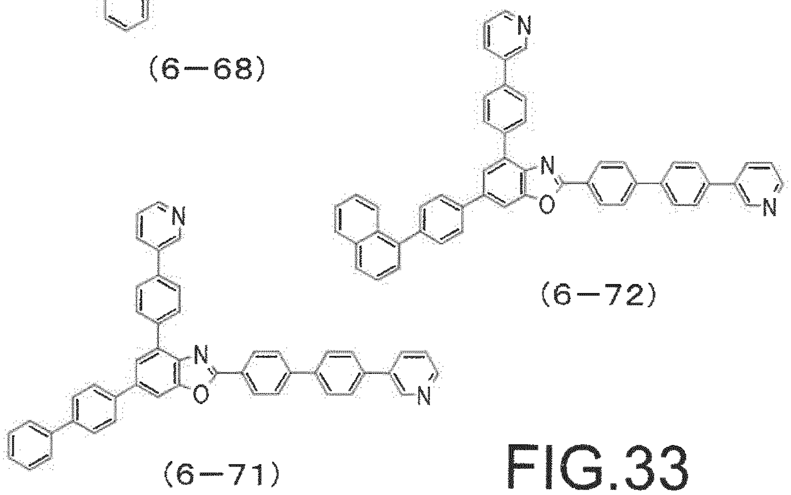

(6−71)

(6−72)

FIG.33

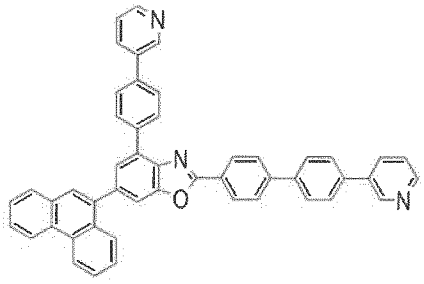

(6−73)

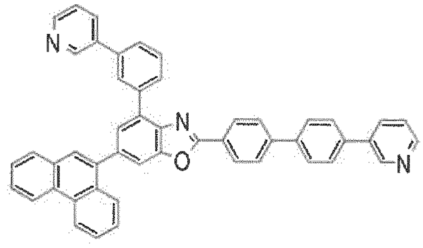

(6−74)

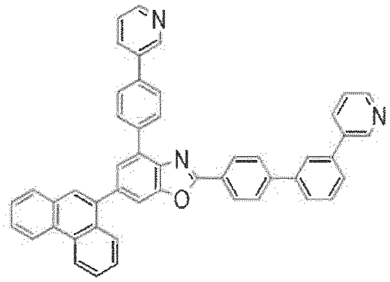

(6−75)

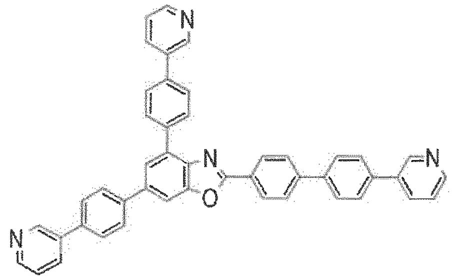

(6−76)

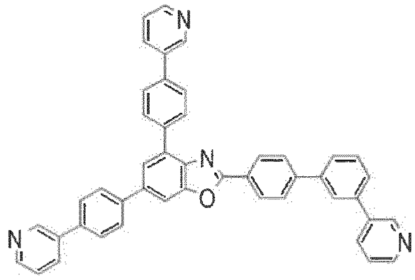

(6−77)

FIG.34

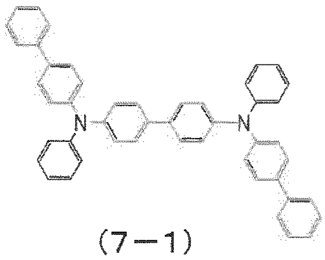

(7−1)

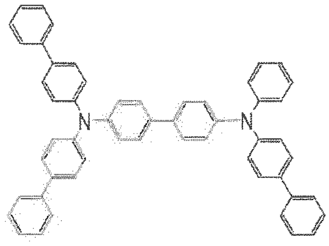

(7−2)

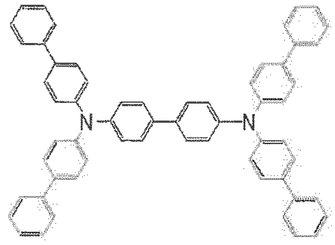

(7−3)

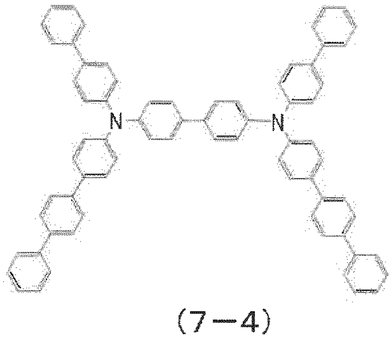

(7−4)

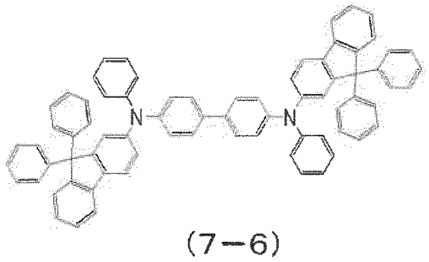

(7−6)

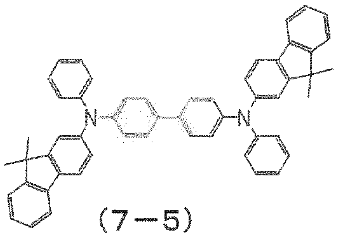

(7−5)

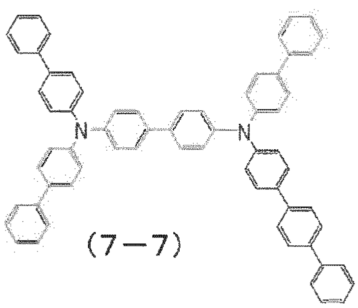

(7−7)

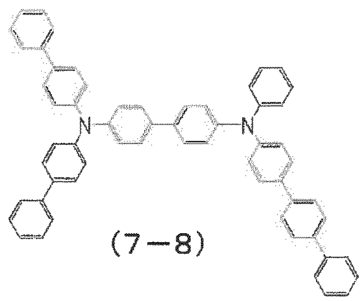

(7−8)

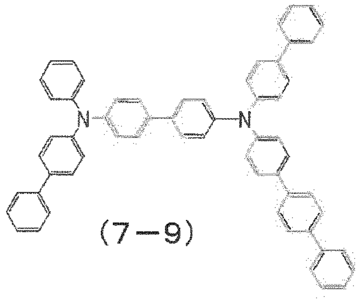

(7−9)

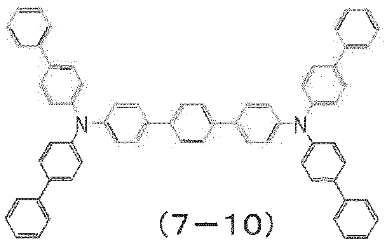

(7−10)

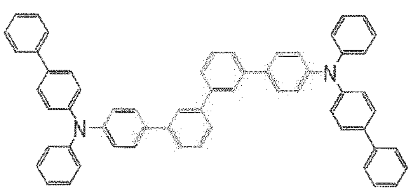

(7−12)

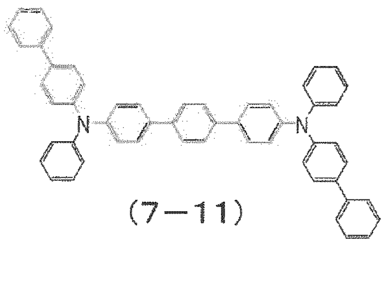

(7−11)

FIG.35

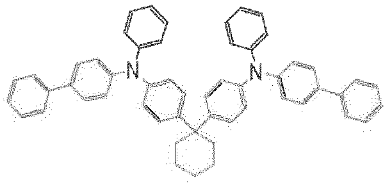

(7—13)

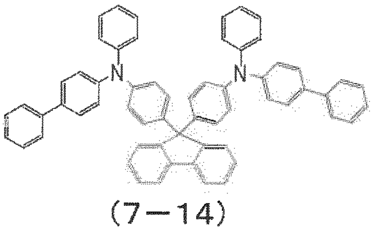

(7—14)

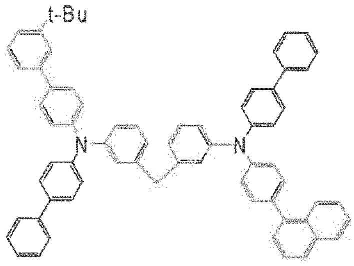

(7—15)

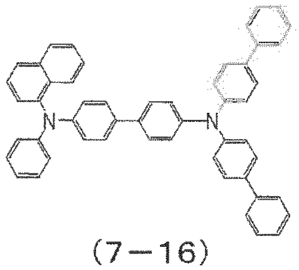

(7—16)

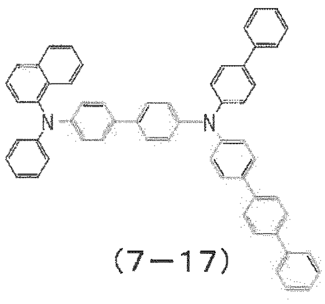

(7—17)

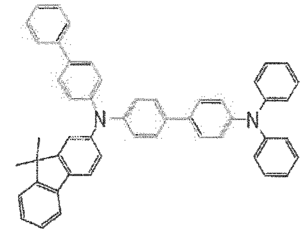

(7—18)

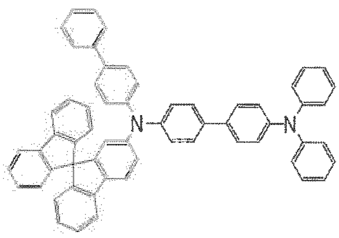

(7—19)

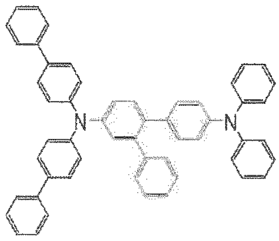

(7—20)

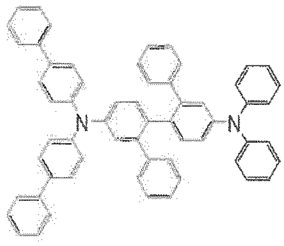

(7—21)

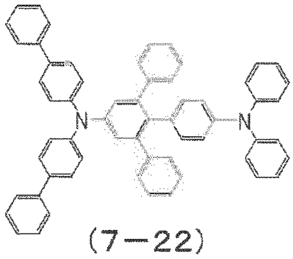

(7—22)

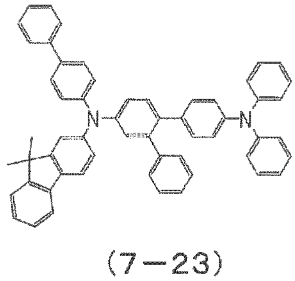

(7—23)

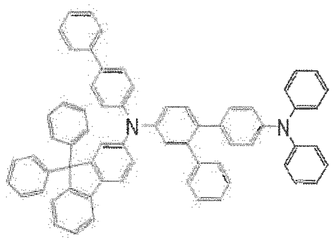

(7—24)

FIG.36

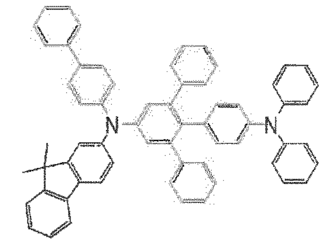

(7−25)

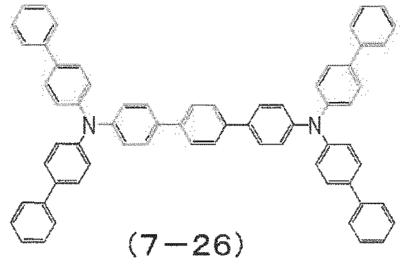

(7−26)

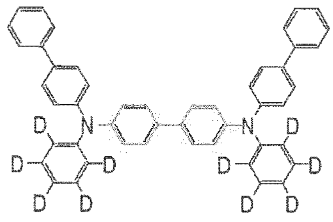

(7−27)

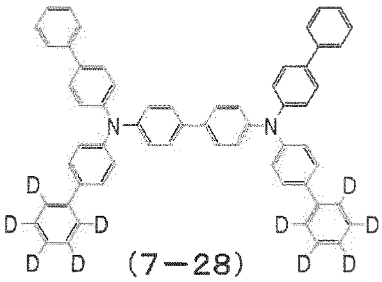

(7−28)

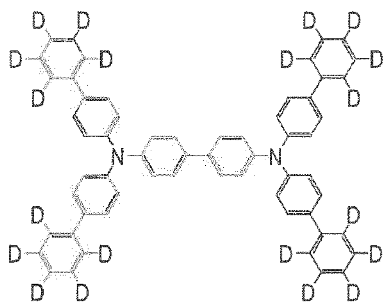

(7−29)

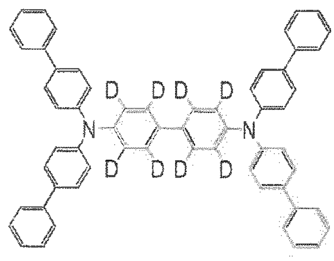

(7−30)

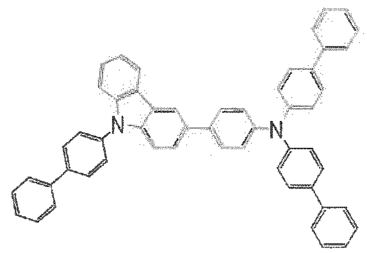

(7−31)

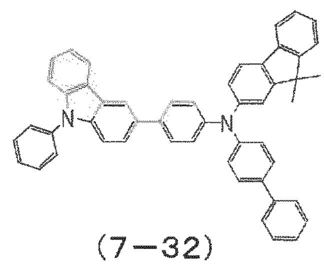

(7−32)

FIG.37

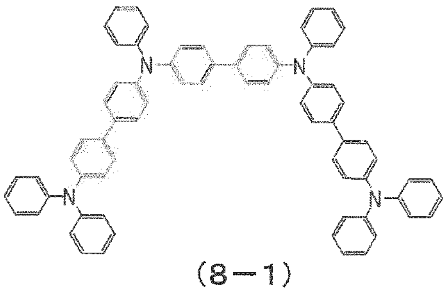

(8−1)

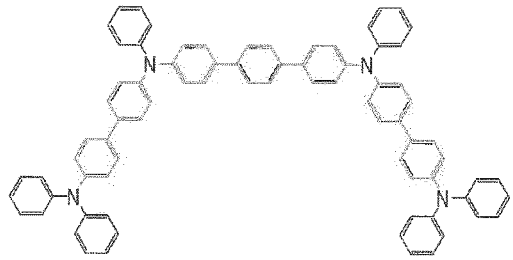

(8−2)

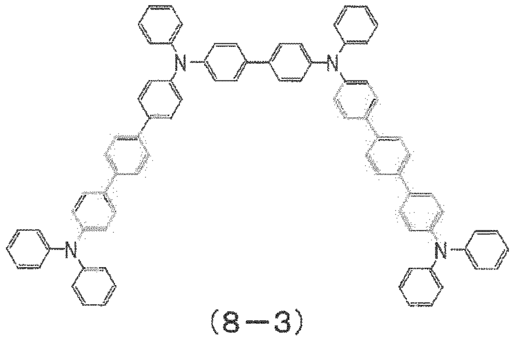

(8−3)

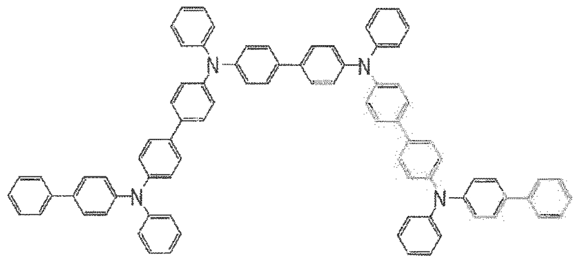

(8−4)

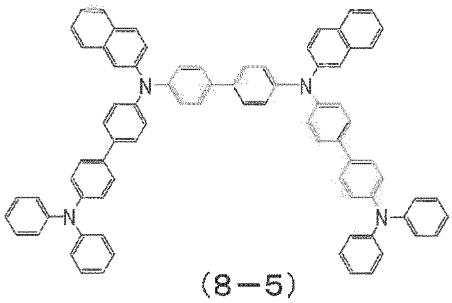

(8−5)

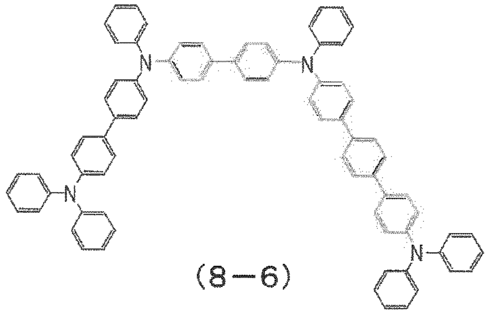

(8−6)

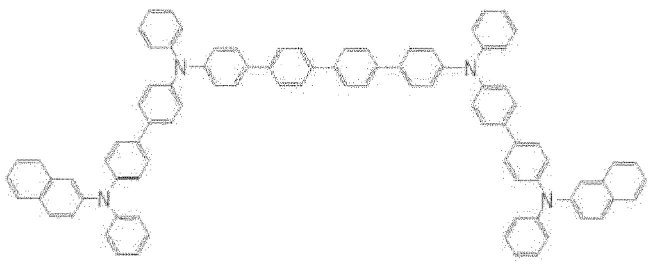

(8−7)

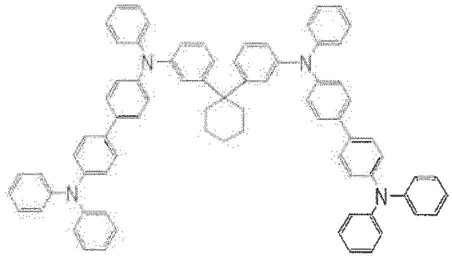

(8−8)

FIG.38

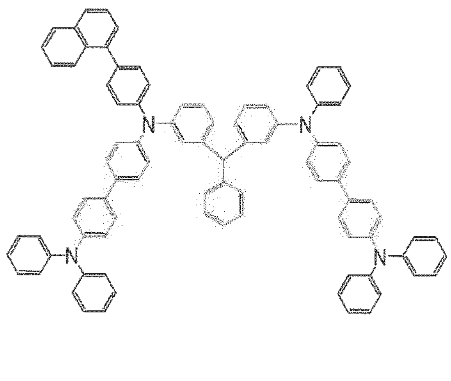

(8−9)

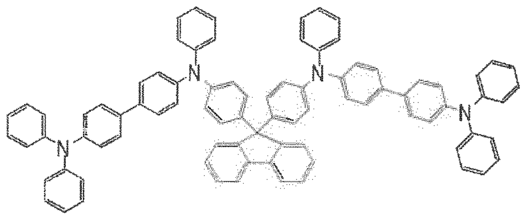

(8−10)

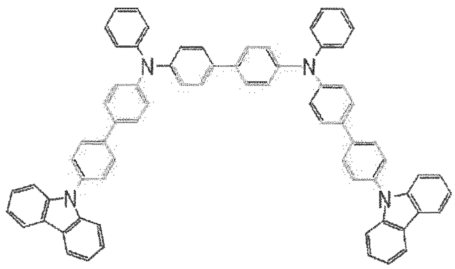

(8−11)

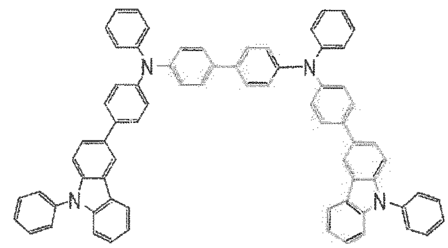

(8−12)

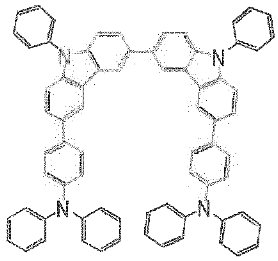

(8−13)

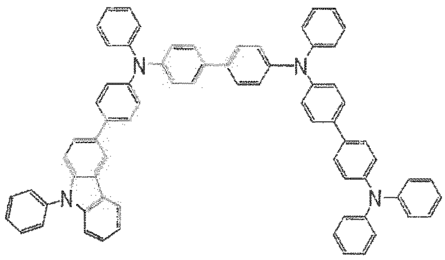

(8−14)

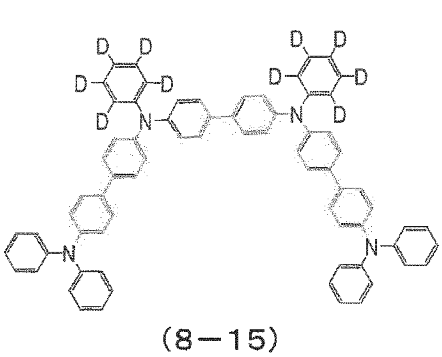

(8−15)

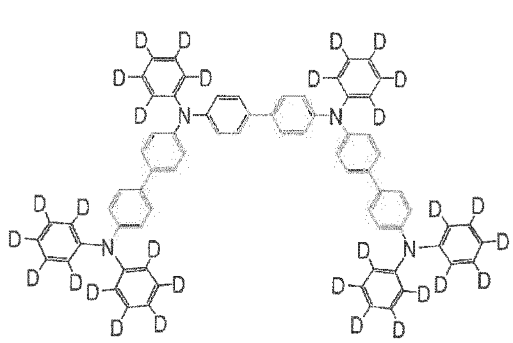

(8−16)

FIG.39

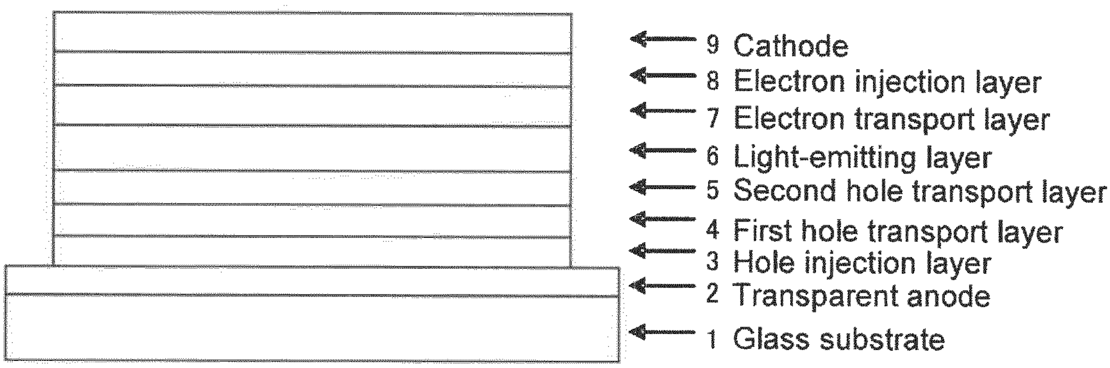
9 Cathode
8 Electron injection layer
7 Electron transport layer
6 Light-emitting layer
5 Second hole transport layer
4 First hole transport layer
3 Hole injection layer
2 Transparent anode
1 Glass substrate

FIG.40

## INTERNATIONAL SEARCH REPORT

International application No. PCT/JP2018/042115

**A. CLASSIFICATION OF SUBJECT MATTER**

Int.Cl. H01L51/50(2006.01)i, C07D209/88(2006.01)i, C07D307/91(2006.01)i, C09K11/06(2006.01)i, C07C211/54(2006.01)n, C07C211/61(2006.01)n, C07F15/00(2006.01)n

According to International Patent Classification (IPC) or to both national classification and IPC

**B. FIELDS SEARCHED**

Minimum documentation searched (classification system followed by classification symbols)

Int.Cl. H01L51/50, C07D209/88, C07D307/91, C09K11/06, C07C211/54, C07C211/61, C07F15/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

CAplus/REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | US 2015/0270492 A1 (SK CHEMICALS CO., LTD.) 24 September 2015, paragraphs [0042], [0235]-[0237], [0245], [0248] & WO 2014/073791 A1 & KR 10-2014-0060220 A & TW 201422769 A & CN 104903422 A | 1-2, 4-5, 8<br>3, 6-7 |

☒ Further documents are listed in the continuation of Box C. ☐ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 31 January 2019 (30.01.2019) | 12 February 2019 (12.02.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2018/042115

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | WO 2016/199743 A1 (HODOGAYA CHEMICAL CO., LTD.) 15 December 2016, paragraphs [0070], [0126]-[0134] & US 2018/0175301 A1 paragraphs [0145], [0352]-[0360] & EP 3309141 A1 & CN 107709285 A & KR 10-2018-0017130 A & TW 201708183 A | 1-2, 4-5, 8<br>3, 6-7 |
| Y | US 2015/0200373 A1 (SAMSUNG DISPLAY CO., LTD.) 16 July 2015, paragraphs [0334], [0337] & KR 10-2015-0085772 A & CN 104795503 A & TW 201533024 A & KR 10-2018-0027457 A | 3 |
| Y | WO 2015/190400 A1 (HODOGAYA CHEMICAL CO., LTD.) 17 December 2015, paragraphs [0061]-[0324], [0340], [0400] & US 2017/0186967 A1 paragraphs [0185], [0243], [0622] & EP 3156402 A1 & KR 10-2017-0016936 A & CN 106573911 A & TW 201602097 A | 6 |
| Y | KR 10-2015-0064442 A (DUK SAN NEOLUX CO., LTD.) 11 June 2015, paragraphs [0083]-[0088], [0192]-[0194] & WO 2015/083948 A1 | 7 |
| Y | KR 10-2014-0094408 A (DUK SAN NEOLUX CO., LTD.) 30 July 2014, paragraphs [0062]-[0075], [0326] (Family: none) | 7 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 8048656 A
- JP 7126615 A
- JP 2006151979 A
- WO 2015034125 A
- WO 2016013732 A
- JP 8003547 A
- JP 2006352088 A
- WO 2016199743 A
- JP 2002105055 A
- WO 2014007565 A
- WO 2014188947 A
- WO 2015190400 A
- JP 2010083862 A
- WO 2015038503 A
- JP 2005108804 A
- WO 200862636 A
- WO 2014009310 A

### Non-patent literature cited in the description

- *The Japan Society of Applied Physics, proceedings of the ninth workshop,* 2001, 55-61
- *The Japan Society of Applied Physics, proceedings of the ninth workshop,* 2001, 23-31
- *Synth.Commun.,* 1981, vol. 11, 513